# EUROPEAN PATENT SPECIFICATION

(11) **EP 4 233 536 B1**
(45) Date of publication and mention of the grant of the patent: **31.12.2025**
(21) Application number: 23160947.0
(22) Date of filing: 12.08.2014
(51) Int. Cl.: A01K 67/0271, A61K 49/00

(54) **METHOD FOR ENHANCING TUMOR GROWTH**
VERFAHREN ZUR VERBESSERUNG VON TUMORWACHSTUM
PROCÉDÉ DE STIMULATION DE CROISSANCE DE TUMEUR

(30) Priority: 12.08.2013 US 201361865092 P; 20.02.2014 WO PCT/US2014/017515
(43) Date of publication of application: 30.08.2023
(62) Divisional of application: 14835875.7
(73) Proprietor: Minerva Biotechnologies Corporation, Woburn, MA 01801 (US)
(72) Inventor: BAMDAD, Cynthia C., Waltham, MA, 02451 (US)
(74) Representative: Mewburn Ellis LLP

(56) References cited:
- WO-A2-2012/126013
- SAMUEL A WILLIAMS ET AL: "Patient-derived xenografts, the cancer stem cell paradigm, and cancer pathobiology in the 21st century", LABORATORY INVESTIGATION, vol. 93, no. 9, 5 August 2013 (2013-08-05), The United States and Canadian Academy of Pathology, Inc., pages 970 - 982, XP055335273, ISSN: 0023-6837, DOI: 10.1038/labinvest.2013.92
- DALERBA PIERO ET AL: "Phenotypic characterization of human colorectal cancer stem cells", vol. 104, no. 24, 12 June 2007 (2007-06-12), pages 10158 - 10163, XP093080818, ISSN: 0027-8424, Retrieved from the Internet <URL:https://www.ncbi.nlm.nih.gov/pmc/articles/PMC1891215/pdf/zpq10158.pdf> DOI: 10.1073/pnas.0703478104

## Description

### BACKGROUND OF THE INVENTION

### 1. Field of the Invention:

The present application relates to methods for enhancing engraftment of tumor in experimental animals. The present application also relates to a method of making cancer stem cells.

### 2. General Background and State of the Art:

Mice, rodents and other animals traditionally, used in drug discovery, do not accurately mimic humans in cancer drug studies using mouse models or xenograft experiments. First, the response that mice have to drug candidates often does not reflect how those drug candidates do in human studies, as is evidenced by the number of lawsuits against pharmaceutical companies for 'bad' drugs. The FDA and drug companies have raised the bar significantly in terms of the level of pre-clinical testing required for permission to test in humans, as well as increasing the numbers of patients tested in clinical trials. Still, compounds that showed no toxicities in mice, and other test animals, continue to cause significant adverse events, including death, when administered to humans. It appears that there are critical differences between mouse and human biology that confound drug safety testing in mice as well as in other test animals, especially, non-primate test animals. A great improvement to the current practice would be to develop methods that make animals, especially rodents, respond more like humans to cells that are implanted or for the study of basic science, drug efficacy, toxicity or dosing.

Secondly, it is hard to get some cancer cells or cancer cell lines to engraft in mice. Many cancer cell lines that are routinely used in *in vitro* testing simply have engraftment rates that are too low for reliable animal studies. For example, T47D breast cancer cell line is typically used for *in vitro* studies because of its overexpression of the oncogene MUC1. However, T47D cells are infrequently used in mouse xenograft studies because of their poor engraftment rate. Animal models have been selected or are genetically altered such that they mimic certain human diseases. Some animal models spontaneously get certain types of cancer. However, these models are generally only useful for studying disease that arises from a specific mutation or do not exactly mimic human disease. As in traditional mouse studies, the effects of a drug candidate in mice are often not predictive of the effects the drug will have in humans. Thus, a significant improvement over the state of the art would be to develop methods that increase the engraftment rate of human cancer cells into a test animal, especially rodent test animals.

In a related matter, there is currently no practical method for evaluating the efficacy, toxicity or dosing of compounds, biologicals or drugs on tumor initiating cells or cancer stem cells. Oncomed, for example has a protracted method for doing so in which a surviving tumor from a first animal is then transplanted into a second animal, then surviving tumor cells from the second animal, which may have survived treatment with an anti-cancer agent, are then implanted into a third animal which is then tested with an agent to test its ability to inhibit the growth of the surviving cancer cells, proposed to be cancer stem cells or tumor initiating cells. These methods were based on research showing that only a small percentage of cancer cells have the ability to metastasize and the identification of markers of these 'cancer stem cells', such as high expression of CD44 accompanied by low expression of CD24 (Clarke MF et al 2006, Chen K et al 2013). The receptor CXCR4 has also been identified as a metastatic receptor whose expression is elevated in cancer stem cells (Darash-Yahana M, Pikarsky E, Abramovitch R, Zeira E et al 2004). However, to date, there has been no evidence that it is possible to expose cancer cells to an agent or agents which causes them to be transformed to cancer stem cells. Thus, a significant improvement over the state of the art would be to develop or identify agents or methods that when added to cancer cells transform some of the cells to cancer stem cells. Drugs and drug candidates for the treatment or progression of metastatic cancers could then be tested on these cancer stem cells. Additionally, the agents and methods would provide a practical method for enriching a population of cancer cells for cancer stem cells which could also be used for the identification of additional yet still unknown markers of a metastatic cancer cells, which would then be new targets for anti-cancer drugs.

It is also important to develop *in vitro* methods to accelerate the transformation of local cancer cells into the highly aggressive and metastatic cancer stem cells so that metastatic drug targets can be identified and so that drugs can be developed that specifically are able to kill those cancer stem cells that can evade chemotherapy. However, it is also important Animals xenografted with NME-7 induced cancer stem cell characteristics not only generated tumors from very low numbers of implanted cancer cells, but also developed metastatic cancer with multiple tumors developing, including ones at locations remote from the implantation site.

Essentially, there are but a handful of cancer cell lines that are repeatedly used to study the basic science of cancer biology and to test for drug efficacy as well as drug toxicities. This is because human cells, unlike mouse cells, can only divide in culture a very limited number of times before they senesce. The cancer cell lines that researchers use today are either naturally immortalized cancer cells isolated from pleural effusions of a single metastatic cancer patient or induced to become immortalized by fusing to an immortalized cell line, often of mouse origin, or more recently by transfecting the cancer cells with an immortalizing gene. The main point is that the methods used to get these cells to continue to self-replicate significantly alter the molecular characteristics of the original or primary cancer cell. The reality is that these cell lines are cancer cells from patients that lived and died years ago and may in no way resemble a particular cancer that a particular patient is stricken with. Thus a significant improvement over the state of the art would be to develop methods to study cancer cells from a patient in a way that does not alter the molecular characteristics of the patient's cancer cells or if the molecular characteristics are altered, those alterations would ideally mimic the progression of that cancer in the patient's body. For example, an acceptable alteration would be if the method for increasing the number of divisions that a patient's cancer cell could undergo transformed the cancer cell or cells into a more aggressive form of that cancer or a metastatic form of that cancer. Then, the efficacy, toxicity or dosing of a compound, biological or drug could be evaluated on the patient's own cancer cells.

Similarly, the efficacy, safety testing and dosing schedule of candidate drugs is established to a first order approximation in rodents using just a handful of immortalized cancer cell lines, which as stated above, bear little or no resemblance to the particular cancer of a particular patient. Thus, a significant improvement over the state of the art would be to develop methods that enable engraftment of a patient's cancer cells into an animal, preferably a rodent, and optionally evaluating the efficacy, toxicity or dosing of a compound, biological or drug on the patient's cancer in the test animal.

In addition to the problems stated above that essentially prevent the engraftment of patient cancer cells into a test animal using current methods, approximately 4 to 6 million cancer cells must be implanted into a test animal in order to establish a tumor in a host animal. The reason for this is that scientists recently discovered that not every cell within a tumor has the ability to give rise to a tumor; the vast majority cannot. The small population of cancer cells that can give rise to a tumor if implanted are called 'tumor initiating cells' or 'cancer stem cells'. It is estimated that as few as 1 in 100,000 cancer cells can give rise to a tumor and established protocols usually call for implantation of millions of cancer cells in order to generate a tumor in a test animal. The problem of studying patient cancer cells in test animals then becomes a problem numbers in addition to the previously described problem of immortalization. A typical tumor biopsy simply would not yield enough cells to implant hundreds of mice with 4-6 million cancer cells each in order to do a proper evaluation of the efficacy, toxicity or dosing of a proposed candidate drug. Recall also that primary patient cells would not be immortalized so would not proliferate as in a patient for the duration of the evaluation experiment. Thus a significant improvement over the state of the art would be to develop methods that enable engraftment of a very few patient cancer cells into an animal.
Williams et al., Laboratory Investigation (2013) 93, 970-982 is a review paper considering the cancer stem cell paradigm, cancer pathobiology and patient-derived xenograft models.
Dalerba et al., PNAS (2007) 104(24), 10158-10163 describe a cancer stem cell model for the study of human colorectal cancer, providing a surface marker profile for colorectal cancer stem cell isolation.
WO 2012/126013 describes methods for inducing cells to gain characteristics of naïve stem cell state comprising culturing the cells in the presence of a MUC1* activator.

### SUMMARY OF THE INVENTION

The invention is defined by the appended claims.

Disclosed herein, not according to the claimed invention, is a method of testing for efficacy of a potential drug agent against cancerous cells in a non-human mammal, comprising: (i) generating the cancer cells in the non-human mammal; (ii) contacting the cancer cells with a potential drug agent by administering the potential drug agent to the mammal; and (iii) measuring effect of the potential drug agent on the cancer cells, wherein reduction of number of cancer cells in the mammal may be indicative of efficaciousness of the potential drug agent against cancerous cells, wherein the method comprises contacting the cancer cells with an agent that maintains stem cells in the naive state or reverts primed stem cells to the naive state before carrying out step (i), after carrying out step (i), or both before and after carrying out step (i).

In the method above, the agent that maintains stem cells in the naive state or reverts primed stem cells to the naive state may be an NME protein, 2i, 5i, chemical, or nucleic acid. The NME protein may be NME1 dimer, NME7 monomer, NME7-AB, NME6 dimer, or bacterial NME.

The mammal may be a rodent, such as a mouse or rat. The cancer may be spontaneously generated or implanted from a human being.

In the method above, the non-human mammal may be transgenic, wherein the mammal expresses human MUC1 or MUC1* or NME protein in the germ cells or somatic cells, wherein the germ cells and somatic cells contain a recombinant human MUC1 or MUC1* or NME gene sequence introduced into said mammal. The gene expressing the human MUC1 or MUC1* or NME protein may be under control of an inducible promoter. The promoter may be inducibly responsive to a naturally occurring protein in the non-human mammal. The amount of cells implanted into the mammal may be at least about 30, about 30 to about 1,000,000, about 50 to about 500,000, about 50 to 100,000, or from about 1,000 to about 1,000,000. The NME protein may be present in serum-free media as the single growth factor.

The invention is directed to a method for engrafting human tumor in a mouse, comprising injecting or implanting human tumor cells into the mouse, the method comprising contacting the tumor cells with an agent that maintains stem cells in the naive state or reverts primed stem cells to the naive state before carrying out the injecting or implanting step, after carrying out the injecting or implanting step, or both before and after carrying out the injecting or implanting step.

In the method above, the agent that maintains stem cells in the naive state or reverts primed stem cells to the naive state is an NME protein. The NME protein may be NME1 dimer, NME7 monomer, NME7-AB, NME6 dimer, or bacterial NME.

In the method above, the mouse may be transgenic, wherein the mouse expresses human MUC1 or MUC1* or NME protein in the germ cells or somatic cells, wherein the germ cells and somatic cells contain a recombinant human MUC1 or MUC1* or NME gene sequence introduced into said mouse. The gene expressing the human MUC1 or MUC1* or NME protein may be under control of an inducible promoter. The promoter may be inducibly responsive to a naturally occurring protein in the mouse. The amount of cells implanted into the mouse may be at least about 30, about 30 to about 1,000,000, about 50 to about 500,000, about 50 to 100,000, or from about 1,000 to about 1,000,000. The NME protein may be present in serum-free media as the single growth factor.

Further disclosed herein, not according to the claimed invention, is a method of generating cancer stem cells, comprising contacting cancer cells with an agent that maintains stem cells in the naive state or reverts primed stem cells to the naïve state.

In the method above, the agent that maintains stem cells in the naive state or reverts primed stem cells to the naive state may be an NME protein, 2i, 5i, chemical, or nucleic acid. The NME protein may be NME1 dimer, NME7 monomer, NME7-AB, NME6 dimer, or bacterial NME.

In this method, the agent may suppress expression of MBD3, CHD4, BRD4 or JMJD6. The agent may be siRNA made against MBD3, CHD4, BRD4 or JMJD6, or siRNA made against any gene that encodes a protein that upregulates expression of MBD3, CHD4, BRD4 or JMJD6. The cancer stem cell may be characterized by increased expression of CXCR4 or E-cadherin (CDH1) compared with cancer cells or normal cells.

Further disclosed herein, not according to the claimed invention, is a method of generating metastatic tumors in a non-human mammal, comprising transferring cancer cells into a mammal, wherein the method comprises contacting the cancer cells with an agent that maintains stem cells in the naive state or reverts primed stem cells to the naive state, before carrying out the transferring step, after carrying out the transferring step, or both before and after carrying out the transferring step.

In the method above, the agent that maintains stem cells in the naive state or reverts primed stem cells to the naive state may be an NME protein, 2i, 5i, chemical, or nucleic acid. The NME protein may be NME1 dimer, NME7 monomer, NME7-AB, NME6 dimer, or bacterial NME.

In this method, the agent may suppress expression of MBD3, CHD4, BRD4 or JMJD6. The agent may be siRNA made against MBD3, CHD4, BRD4 or JMJD6, or siRNA made against any gene that encodes a protein that upregulates expression of MBD3, CHD4, BRD4 or JMJD6. The cancer stem cell may be characterized by increased expression of CXCR4 or E-cadherin (CDH1) compared with cancer cells or normal cells.

In the method above, the mammal may be a rodent, such as a mouse or rat. The non-human mammal may be transgenic, wherein the mammal expresses human MUC1 or MUC1* or NME protein in the germ cells or somatic cells, wherein the germ cells and somatic cells contain a recombinant human MUC1 or MUC1* or NME gene sequence introduced into said mammal. The gene expressing the human MUC1 or MUC1* or NME protein may be under control of an inducible promoter. The promoter may be inducibly responsive to a naturally occurring protein in the non-human mammal. The amount of cells implanted into the mammal may be at least about 30, about 30 to about 1,000,000, about 50 to about 500,000, about 50 to 100,000, or from about 1,000 to about 1,000,000. The NME protein may be present in serum-free media as the single growth factor.

Further disclosed herein, not according to the claimed invention, is a method of testing for efficacy of a potential drug agent against a patient's cancerous cells in a non-human mammal, comprising: (i) transferring the patient's cancer cells into the non-human mammal; (ii) contacting the cancer cells with a potential drug agent by administering the potential drug agent to the mammal; and (iii) measuring effect of the potential drug agent on the cancer cells, wherein reduction of number of the patient's cancer cells in the mammal may be indicative of efficaciousness of the potential drug agent against cancerous cells, wherein the method comprises contacting the patient's cancer cells with an agent that maintains stem cells in the naive state or reverts primed stem cells to the naive state before carrying out step (i), after carrying out step (i), or both before and after carrying out step (i).

In the method above, the agent that maintains stem cells in the naive state or reverts primed stem cells to the naive state may be an NME protein, 2i, 5i, chemical, or nucleic acid. The NME protein may be NME1 dimer, NME7 monomer, NME7-AB, NME6 dimer, or bacterial NME.

In this method, the agent may suppress expression of MBD3, CHD4, BRD4 or JMJD6. The agent may be siRNA made against MBD3, CHD4, BRD4 or JMJD6, or siRNA made against any gene that encodes a protein that upregulates expression of MBD3, CHD4, BRD4 or JMJD6. The cancer stem cell may be characterized by increased expression of CXCR4 or E-cadherin (CDH1) compared with cancer cells or normal cells.

In the method above, the mammal may be a rodent, such as a mouse or rat. The non-human mammal may be transgenic, wherein the mammal expresses human MUC1 or MUC1* or NME protein in the germ cells or somatic cells, wherein the germ cells and somatic cells contain a recombinant human MUC1 or MUC1* or NME gene sequence introduced into said mammal. The gene expressing the human MUC1 or MUC1* or NME protein may be under control of an inducible promoter. The promoter may be inducibly responsive to a naturally occurring protein in the non-human mammal. The amount of cells implanted into the mammal may be at least about 30, about 30 to about 1,000,000, about 50 to about 500,000, about 50 to 100,000, or from about 1,000 to about 1,000,000. The NME protein may be present in serum-free media as the single growth factor.

Further disclosed herein, not according to the claimed invention, is a method for generating tissue from xenograft in a non-human mammal, comprising: (i) generating a transgenic non-human mammal, wherein the mammal expresses human MUC1 or MUC1* or NME protein in the germ cells and somatic cells, wherein the germ cells and somatic cells contain a recombinant human MUC1 or MUC1* or NME gene sequence introduced into said mammal, wherein the expression of the gene sequence may be under control of an inducible and repressible regulatory sequence; (ii) transferring stem cells or progenitor cells that are xenogeneic in origin to the non-human mammal such that the gene may be induced to be expressed so as to multiply the number of stem or progenitor cells; and (iii) repressing the gene expression so as to generate tissue from the xenografted stem cells.

In this method, in step (iii), the gene expression repression may be carried out by contacting the stem cells with a tissue differentiation factor, or in step (iii) the gene expression repression may be carried out naturally in the mammal in response to naturally produced host tissue differentiation factor. The transferred cells may be human. The tissue may be an organ. The NME protein may be NME1 dimer, NME7 monomer, NME7-AB, NME6 dimer, or bacterial NME. The mammal may be a rodent, such as a mouse or rat.

Further disclosed herein, not according to the claimed invention, is a method of generating cancer preventative peptide comprising: (i) generating a non-human transgenic host mammal, wherein the mammal expresses human MUC1 or MUC1* or NME protein in the germ cells or somatic cells, wherein the germ cells and somatic cells contain a recombinant human MUC1 or MUC1* or NME gene sequence introduced into said mammal; (ii) immunizing the mammal with a fragment of NME protein; (iii) implanting human tumor cells into the mammal; and (iv) comparing tumor engraftment, tumor growth rate or tumor initiating potential of cells in the mammal with a control transgenic non-human mammal such that peptide causing significantly reduced tumor engraftment, tumor growth rate, or tumor initiating potential are selected as an immunizing peptide.

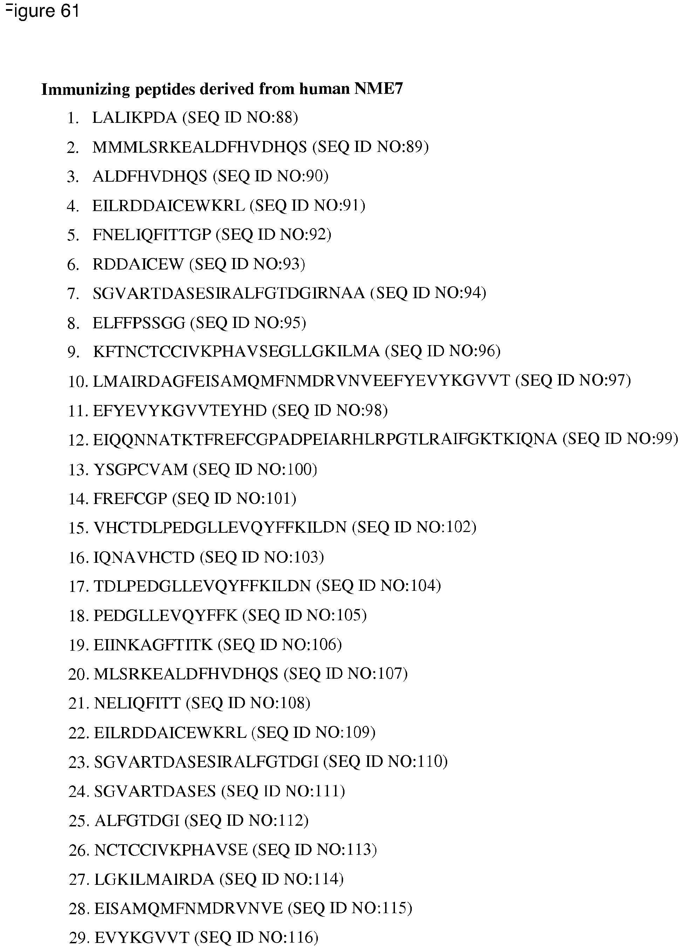

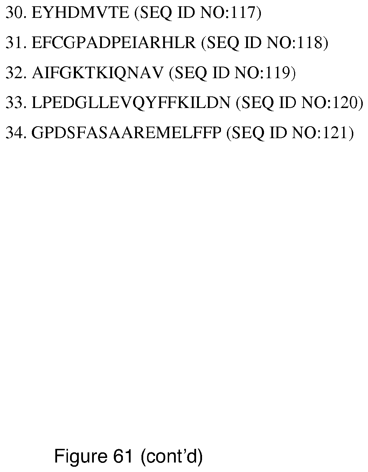

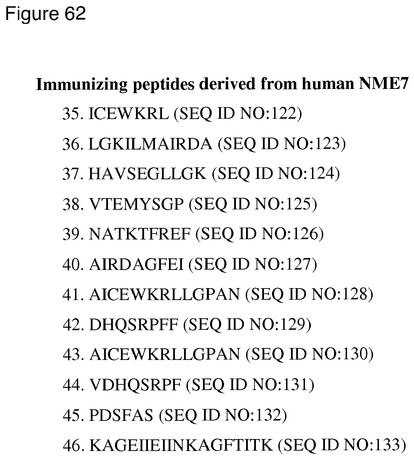

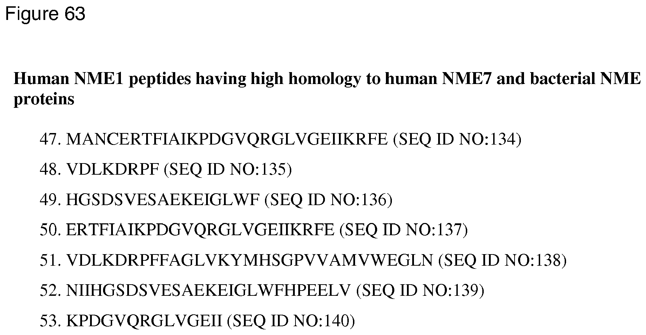

In this method, the fragment of NME protein may be a peptide selected from peptide number 1 to 53 in Figures 61-63. The NME protein may be NME1 dimer, NME7 monomer, NME7-AB, NME6 dimer, or bacterial NME. The mammal may be a rodent, such as a mouse or rat.

### BRIEF DESCRIPTION OF THE DRAWINGS

The present invention will become more fully understood from the detailed description given herein below, and the accompanying drawings which are given by way of illustration only, and thus are not limitative of the present invention, and wherein;
**Figure 1** shows graphs of tumor cell growth in mice. Panel (A) shows a graph of the growth of T47D breast tumor cells mixed with either the standard Matrigel or Matrigel plus NME7 and xenografted into immune compromised (nu/nu) mice. After Day 14, the mice whose tumor cells were mixed with NME7 were also injected once daily with human recombinant NME7. Panel (B) shows a graph of the growth of T47D breast tumor cells mixed with Matrigel plus NME7 and xenografted into immune compromised mice. After Day 14, half of the mice were also injected once daily with human recombinant NME7.
**Figure 2** shows graphs of the growth of human tumor cells in individual mice. Panel (A) shows a graph of the growth of T47D breast cancer cells that were mixed with the standard Matrigel. Only two (2) of the six (6) implanted mice showed tumor growth characteristic of engraftment. Panel (B) shows a graph of the growth of T47D breast cancer cells that were mixed with Matrigel and NME7. Four (4) of the six (6) implanted mice showed tumor growth characteristic of engraftment. Dashed lines indicate mice that were also injected with NME7 after Day 14.
**Figure 3** shows a graph of T47D tumor cells mixed with the standard Matrigel and xenografted into immune compromised (nu/nu) mice. The graph shows the average of two identical groups of twenty mice each, with an average increase of 22% in tumor volume but a downward trend.
**Figure 4** shows a graph of the growth of the T47D human breast tumor cells in the forty (40) individual mice, with about 25% showing tumor engraftment.
**Figure 5** shows graphs of the growth of T47D breast cancer cells mixed with Matrigel and xenografted into the flanks of NOD/SCID mice. Panel (A) shows average tumor growth. Panel (B) shows tumor growth in individual mice, revealing that only one (1) of six (6) mice had good tumor engraftment using the standard method.

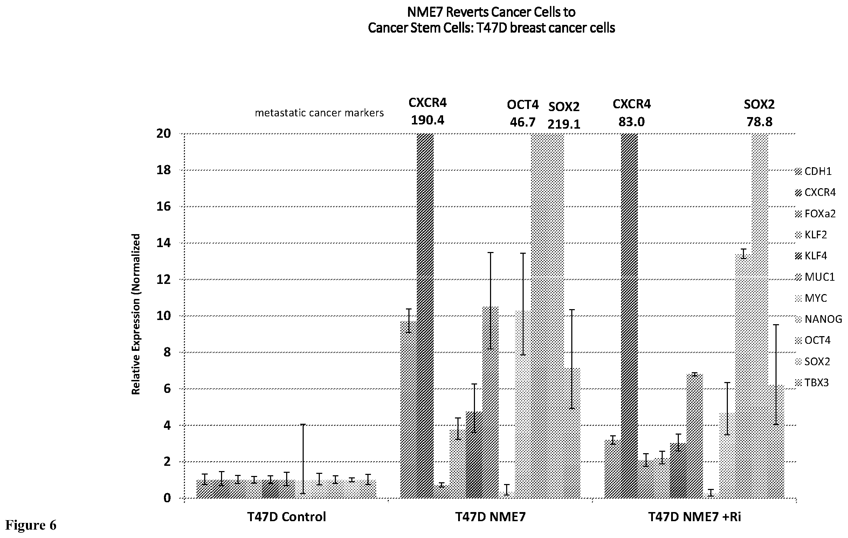

**Figure 6** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added recombinant human NME7-AB as the single growth factor. A portion of those cells became non-adherent and floated off the surface and were collected while '+ Ri' refers to Rho kinase inhibitor that was added to the media so that all the cells would remain attached to the surface, thus giving an average reading of the adherent and the non-adherent cells.
**Figure 7** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added a human recombinant NM23, also called NME1, dimers or NME7-AB as the single growth factor. 'Floaters' refers to those cells that became non-adherent and were collected, while '+ Ri' refers to Rho kinase inhibitor that was added to some cells to make them adhere to the surface.
**Figure 8** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added recombinant bacterial NME1 dimers from species HSP593. 'Floaters' refers to those cells that became non-adherent and were collected, then analyzed.

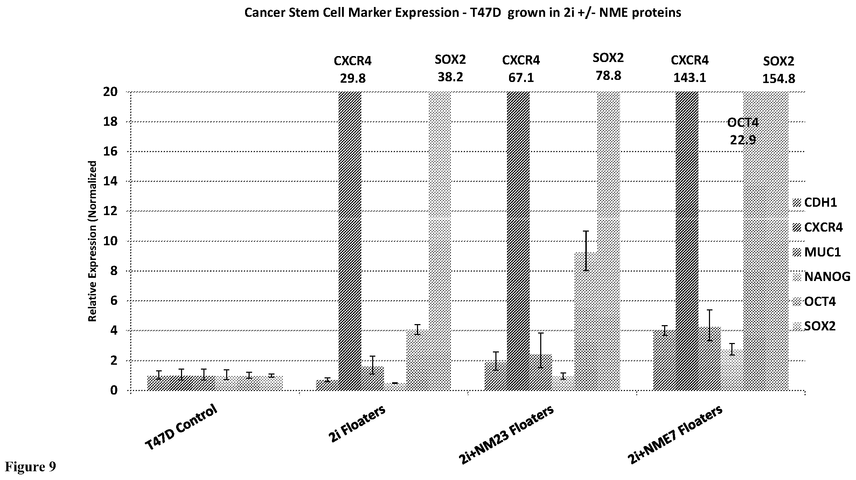

**Figure 9** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added '2i', which are biochemical inhibitors of MAP kinase and GSK3, previously shown to revert stem cells in the primed state to the earlier naive state, 2i plus recombinant human NM23 dimers, or 2i plus recombinant human NME7-AB. 'Floaters' refers to those cells that became non-adherent and were collected, then analyzed.

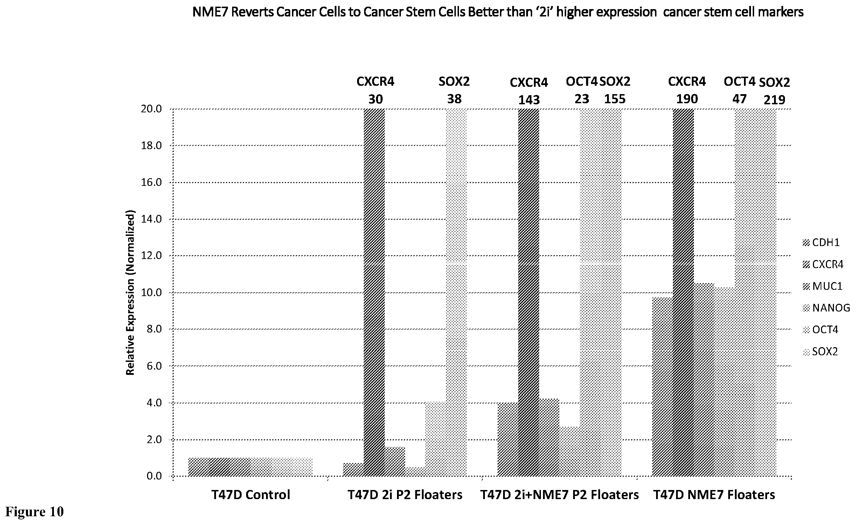

**Figure 10** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added '2i', which are biochemical inhibitors of MAP kinase and GSK3, previously shown to revert stem cells in the primed state to the earlier naive state, 2i plus recombinant human NME7-AB, or NME7-AB alone. 'Floaters' refers to those cells that became non-adherent and were collected, then analyzed.
**Figure 11a** shows a graph of RT-PCR measurements of the expression of a panel of genes, reportedly overexpressed in some cancer stem cells or metastatic cancer cells, in MUC1-positive DU145 prostate cancer cells that were cultured in either normal RPMI growth media, or a serum free minimal media to which was added a human recombinant NM23, also called NME1, dimers or NME7-AB as the single growth factor. These cells did not float off the surface although many were only loosely attached, making the measurements the average of what would be 'Floaters' and adherent cells.
**Figure 11b** shows a graph of RT-PCR measurements of the expression of a panel of genes after DU145 prostate cancer cells were cultured for 9 or 10 passages in recombinant human NME7-AB, bacterial HSP593 NME1 or human NME1/NM23 dimers. The graph shows an increase in the expression of prostate cancer marker CDH1/E-cadherin and stem cell markers after prolonged culture in the agents.
**Figure 12a** shows a graph of RT-PCR measurements of the expression of a panel of genes, reportedly overexpressed in some cancer stem cells or metastatic cancer cells, in MUC1-negative PC3 prostate cancer cells that were cultured in either normal RPMI growth media, or a serum free minimal media to which was added recombinant human NME1/ NM23, dimers or NME7-AB as the single growth factor.
**Figure 12b** shows a graph of RT-PCR measurements of the expression of a panel of genes, in MUC1-negative PC3 prostate cancer cells after serial passaging in serum-free minimal media to which was added recombinant human NME1/NM23, bacterial HSP593 NME1 or NME7-AB as the single growth factor.
**Figure 13** shows a graph of RT-PCR measurements of the expression of genes that code for chromatin rearrangement factors BRD4, JMJD6, MBD3 and CHD4, in MUC1-positive T47D breast cancer cells were cultured in either normal RPMI growth media, or a serum free minimal media to which was added either '2i' or recombinant human NME7-AB wherein the 'floater' cells were the cells analyzed.
**Figure 14** shows a graph of RT-PCR measurements of the expression of genes that code for chromatin rearrangement factors BRD4, JMJD6, MBD3 and CHD4, in MUC1-positive T47D breast cancer cells were cultured in either normal RPMI growth media, or a serum free minimal media to which was added either 2i, 2i plus recombinant human NM23 dimers, or 2i plus recombinant human NME7-AB. 'Floaters' refers to those cells that became non-adherent and were collected, then analyzed.

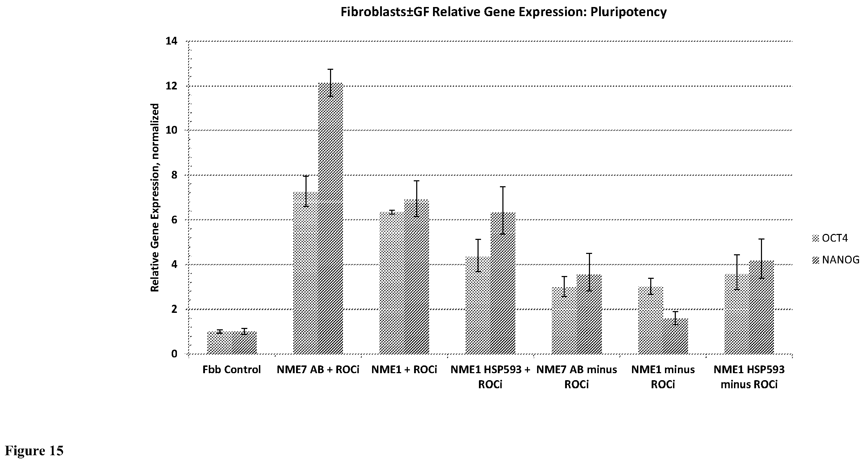

**Figure 15** shows a graph of RT-PCR measurements of the expression of genes, reportedly overexpressed in some cancer stem cells or metastatic cancer cells, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface. 'Minus ROCi' does not refer to floaters but rather refers to cells that remained adherent in the absence of a rho kinase inhibitor.
**Figure 16** shows a graph of RT-PCR measurements of the expression of genes that code for chromatin rearrangement factors BRD4, JMJD6, MBD3 and CHD4, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface.

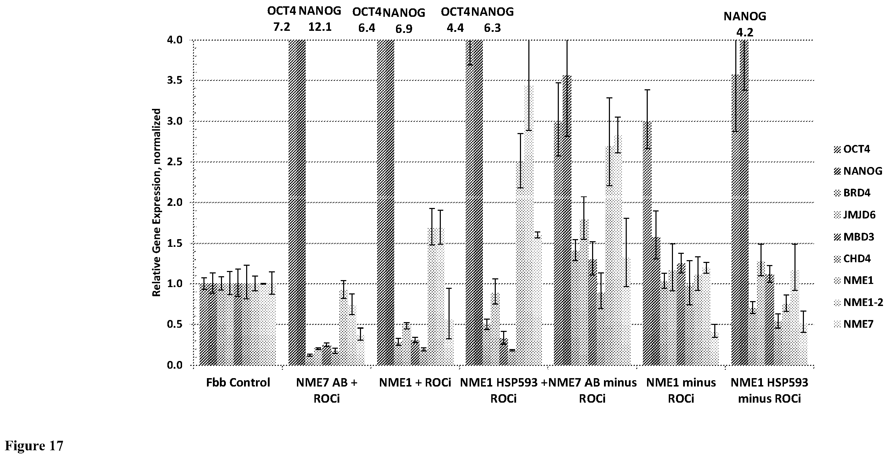

**Figure 17** shows a graph of RT-PCR measurements of the expression of genes, reportedly overexpressed in some cancer stem cells or metastatic cancer cells and genes that code for chromatin rearrangement factors BRD4, JMJD6, MBD3 and CHD4, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface. 'Minus ROCi' here refers to cells that became non-adherent and floated off the surface.
**Figure 18** shows a photograph of human embryonic stem cells with pluripotent morphology wherein the stem cells have been cultured in a serum-free minimal media with recombinant human NME1 dimers as the only growth factor added.
**Figure 19** shows a photograph of human embryonic stem cells with pluripotent morphology wherein the stem cells have been cultured in a serum-free minimal media with recombinant human NME7-AB as the only growth factor added.
**Figure 20** shows a photograph of human embryonic stem cells with pluripotent morphology wherein the stem cells have been cultured in a serum-free minimal media with recombinant HSP593 bacterial NME1 dimers as the only growth factor added.

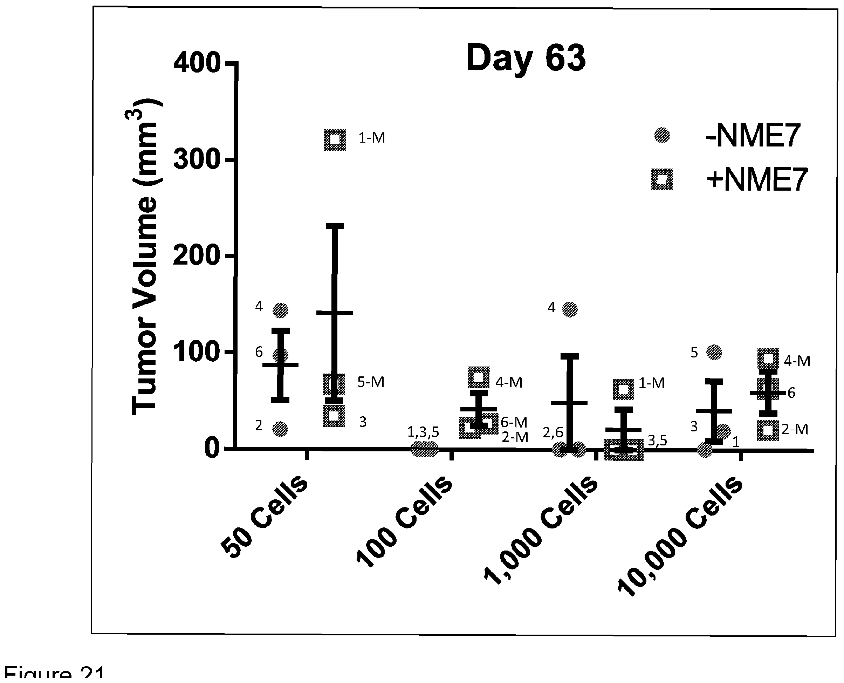

**Figure 21** shows a graph of tumor volume measurements for four (4) groups of immune-compromised nu/nu female mice implanted with either 50, 100, 1,000 or 10,000 cells sub-cutaneously in the flank wherein the cells that were implanted were human MUC1-positive breast cancer cells that were cultured for seven (7) days in recombinant human NME7-AB wherein the 'floaters' were collected and verified to overexpress metastasis receptor CXCR4 by more than 100-fold. Half the mice in each group were injected daily with human recombinant NME7-AB. Numbers within the graph refer to the mouse tracking number. 'M' denotes a mouse with multiple tumors.

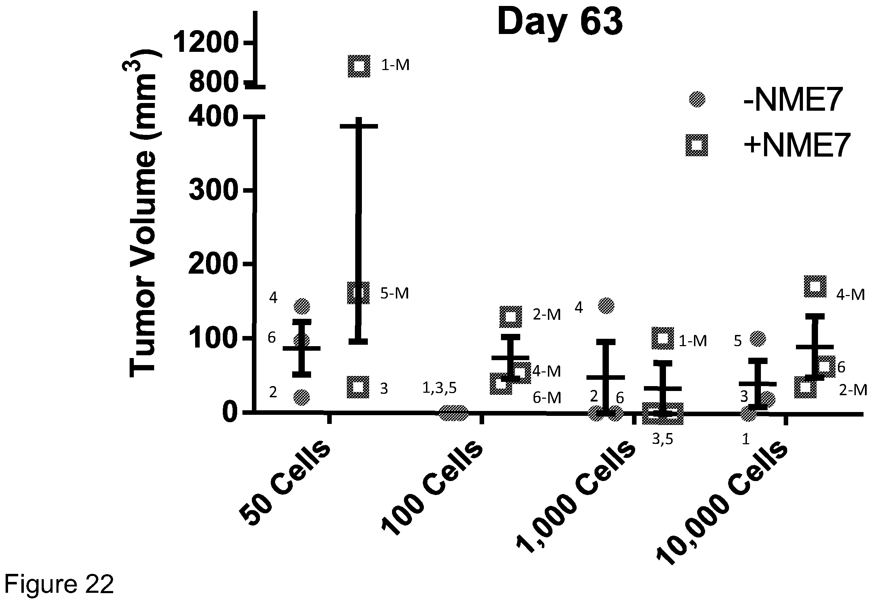

**Figure 22** shows a graph of tumor volume measurements for four (4) groups of immune-compromised nu/nu female mice implanted with either 50, 100, 1,000 or 10,000 cells sub-cutaneously in the flank wherein the cells that were implanted were human MUC1-positive breast cancer cells that were cultured for seven (7) days in recombinant human NME7-AB wherein the 'floaters' were collected and verified to overexpress metastasis receptor CXCR4 by more than 100-fold. Half the mice in each group were injected daily with human recombinant NME7-AB. Of the mice that received daily injections of NME7-AB, 80% developed multiple tumors. This graph shows the combined volumes of multiple tumors in the same mouse. Numbers within the graph refer to the mouse tracking number. 'M' denotes a mouse with multiple tumors.
**Figure 23** shows photographs of mouse #1 implanted with 50 cancer cells and injected daily with rhNME7-AB, at Day 28 and Day 58. Dark arrows point to tumors at the site of injection and light arrows point to metastatic tumors that developed between Day 28 and Day 63.
**Figure 24** shows photographs of a mouse #2 implanted with 50 cancer cells and not injected daily with rhNME7-AB, at Day 28 and Day 58. Dark arrow points to a tumor at the site of injection.
**Figure 25** shows photographs of a mouse #3 implanted with 50 cancer cells and injected daily with rhNME7-AB, at Day 28 and Day 58. Dark arrow points to a tumor at the site of injection.
**Figure 26** shows photographs of a mouse #4 implanted with 50 cancer cells and not injected daily with rhNME7-AB, at Day 28 and Day 58. Dark arrow points to a tumor at the site of injection.
**Figure 27** shows photographs of a mouse #5 implanted with 50 cancer cells and injected daily with rhNME7-AB, at Day 28 and Day 58. Dark arrows point to tumors at the site of injection and light arrows point to metastatic tumors that developed between Day 28 and Day 63.
**Figure 28** shows photographs of a mouse #2 implanted with 50 cancer cells and not injected daily with rhNME7-AB, at Day 28 and Day 58. Dark arrow points to a tumor at the site of injection.
**Figure 29** shows photographs of a mouse #1 implanted with 100 cancer cells and not injected daily with rhNME7-AB, at Day 28. Dark arrow points to a tumor at the site of injection.
**Figure 30** shows photographs of a mouse #2 implanted with 100 cancer cells and injected daily with rhNME7-AB, at Day 28 and Day 58. Dark arrows point to tumors at the site of injection and light arrows point to metastatic tumors that developed between Day 28 and Day 63.
**Figure 31** shows photographs of a mouse #3 implanted with 100 cancer cells and not injected daily with rhNME7-AB, at Day 28. Dark arrow points to a tumor at the site of injection.
**Figure 32** shows photographs of a mouse #4 implanted with 100 cancer cells and injected daily with rhNME7-AB, at Day 28 and Day 58. Dark arrows point to tumors at the site of injection and light arrows point to metastatic tumors that developed between Day 28 and Day 63.
**Figure 33** shows photographs of a mouse #5 implanted with 100 cancer cells and not injected daily with rhNME7-AB, at Day 28 and Day 58. No tumor developed between Day 28 and Day 63.
**Figure 34** shows photographs of a mouse #6 implanted with 100 cancer cells and injected daily with rhNME7-AB, at Day 28 and Day 58. Dark arrows point to tumors at the site of injection and light arrows point to metastatic tumors that developed between Day 28 and Day 63.
**Figure 35** shows photographs of a mouse #1 implanted with 1,000 cancer cells and injected daily with rhNME7-AB, at Day 28 and Day 58. Dark arrows point to tumors at the site of injection and light arrows point to metastatic tumors that developed between Day 28 and Day 63.
**Figure 36** shows photographs of a mouse #2 implanted with 1,000 cancer cells and not injected with rhNME7-AB, at Day 28 and Day 58. No tumor developed between Day 28 and Day 63.
**Figure 37** shows photographs of a mouse #3 implanted with 1,000 cancer cells and injected daily with rhNME7-AB, at Day 28 and Day 58. No tumor developed between Day 28 and Day 63.
**Figure 38** shows photographs of a mouse #4 implanted with 1,000 cancer cells and not injected daily with rhNME7-AB, at Day 28 and Day 58. Dark arrow points to a tumor at the site of injection.
**Figure 39** shows photographs of a mouse #5 implanted with 1,000 cancer cells and injected daily with rhNME7-AB, at Day 28 and Day 58. Dark arrow points to a tumor at the site of injection.
**Figure 40** shows photographs of a mouse #6 implanted with 1,000 cancer cells and not injected daily with rhNME7-AB, at Day 28 and Day 58. No tumor developed between Day 28 and Day 63.
**Figure 41** shows photographs of a mouse #1 implanted with 10,000 cells and not injected daily with rhNME7-AB, at Day 28, was sacrificed due to sickness. Dark arrow points to tumor at the site of injection.
**Figure 42** shows photographs of a mouse #2 implanted with 10,000 cancer cells and injected daily with rhNME7-AB, at Day 28 and Day 58. Dark arrows point to tumors at the site of injection and light arrows point to metastatic tumors that developed between Day 28 and Day 63.
**Figure 43** shows photographs of a mouse #3 implanted with 10,000 cancer cells and not injected daily with rhNME7-AB, at Day 28 and Day 58. Dark arrow points to a tumor at the site of injection.
**Figure 44** shows photographs of a mouse #4 implanted with 10,000 cancer cells and injected daily with rhNME7-AB, at Day 28 and Day 58. Dark arrows point to tumors at the site of injection and light arrows point to metastatic tumors that developed between Day 28 and Day 63.
**Figure 45** shows photographs of a mouse #5 implanted with 10,000 cancer cells and not injected daily with rhNME7-AB, at Day 28 and Day 58. Dark arrow points to tumor at the site of injection.
**Figure 46** shows photographs of a mouse #6 implanted with 10,000 cancer cells and injected daily with rhNME7-AB, at Day 28 and Day 58. Dark arrow points to tumor at the site of injection.
**Figure 47** shows graph of HRP signal from ELISA sandwich assay showing NME7-AB dimerizes MUC1* extra cellular domain peptide.
**Figures 48A-48B****.** (A) shows a polyacrylamide gel of NME from the bacterium *Halomonas* Sp. 593, which was expressed in *E. coli* and expressed as a soluble protein and natural dimer. (B) shows that in an ELISA assay NME from *Halomonas* Sp. 593 bound to the PSMGFR peptide of the MUC1* extra cellular domain.
**Figure 49** shows a polyacrylamide gel of NME from the bacterium *Porphyromonas gingivalis* W83.
**Figures 50A-50C****.** (A) shows sequence alignment of *Halomonas* Sp 593 bacterial NME to human NME-H1. (B) shows sequence alignment of *Halomonas* Sp 593 bacterial NME to human NME7-A domain. (C) shows sequence alignment of *Halomonas* Sp 953 bacterial NME to human NME7-B domain.
**Figure 51** is a graph of RT-PCR measurement of the expression levels of transcription factors BRD4 and co-factor JMJD6 in the earliest stage naive human stem cells compared to the later stage primed stem cells.
**Figure 52** shows photographs of human fibroblast cells after 18 days in culture in a serum-free media containing human NME1 in dimer form at 4X magnification.
**Figure 53** shows photographs of human fibroblast cells after 18 days in culture in a serum-free media containing human NME1 in dimer form at 20X magnification.
**Figure 54** shows photographs of human fibroblast cells after 18 days in culture in a serum-free media containing bacterial NME from *Halomonas* Sp 593 at 4X magnification.
**Figure 55** shows photographs of human fibroblast cells after 18 days in culture in a serum-free media containing bacterial NME from *Halomonas* Sp 593 at 20X magnification.
**Figure 56** shows photographs of human fibroblast cells after 18 days in culture in a serum-free media containing human NME7-AB at 4X magnification.
**Figure 57** shows photographs of human fibroblast cells after 18 days in culture in a serum-free media containing human NME7-AB at 20X magnification. **Figure 58** shows photographs of human fibroblast cells after 18 days in standard media without NME protein at 4X magnification.
**Figure 59** shows photographs of human fibroblast cells after 18 days in standard media without NME protein at 20X magnification.
**Figure 60** is a cartoon of the interaction map of NME7 and associated factors resulting from analysis of the experiments described herein.
**Figure 61** lists immunogenic peptides from human NME7 with low sequence identity to NME1. The listed peptide sequences are identified as being immunogenic peptides giving rise to antibodies that target human NME7 but not human NME1. The sequences were chosen for their lack of sequence homology to human NME1, and are useful as NME7 specific peptides for generating antibodies to inhibit NME7 for the treatment or prevention of cancers.
**Figure 62** lists immunogenic peptides from human NME7 that may be important for structural integrity or for binding to MUC1*. Bivalent and bi-specific antibodies wherein each variable region binds to a different peptide portion of NME7 are preferred. Such peptides may be generated by using more than one peptide to generate the antibody specific to both. The peptides are useful as NME7 specific peptides for generating antibodies to inhibit NME7 for the treatment or prevention of cancers.
**Figure 63** lists immunogenic peptides from human NME1 that may be important for structural integrity or for binding to MUC1*. The listed peptide sequences are from human NME1 and were selected for their high homology to human NME7 as well as for their homology to other bacterial NME proteins that are able to mimic its function. In particular, peptides 50 to 53 have high homology to human NME7-A or -B and also to HSP 593.

### DETAILED DESCRIPTION OF THE PREFERRED EMBODIMENTS

### Definitions

As used herein, the "MUC1*" extra cellular domain is defined primarily by the PSMGFR sequence (GTINVHDVETQFNQYKTEAASRYNLTISDVSVSDVPFPFSAQSGA (SEQ ID NO:6)). Because the exact site of MUC1 cleavage depends on the enzyme that clips it, and that the cleavage enzyme varies depending on cell type, tissue type or the time in the evolution of the cell, the exact sequence of the MUC1* extra cellular domain may vary at the N-terminus.

As used herein, the term "PSMGFR" is an acronym for Primary Sequence of MUC1 Growth Factor Receptor as set forth as GTINVHDVETQFNQYKTEAASRYNLTISDVSVSDVPFPFSAQSGA (SEQ ID NO:6). In this regard, the "N-number" as in "N-10 PSMGFR", "N-15 PSMGFR", or "N-20 PSMGFR" refers to the number of amino acid residues that have been deleted at the N-terminal end of PSMGFR. Likewise "C-number" as in "C-10 PSMGFR", "C-15 PSMGFR", or "C-20 PSMGFR" refers to the number of amino acid residues that have been deleted at the C-terminal end of PSMGFR.

As used herein, the "extracellular domain of MUC1*" refers to the extracellular portion of a MUC1 protein that is devoid of the tandem repeat domain. In most cases, MUC1* is a cleavage product wherein the MUC1* portion consists of a short extracellular domain devoid of tandem repeats, a transmembrane domain and a cytoplasmic tail. The precise location of cleavage of MUC1 is not known perhaps because it appears that it can be cleaved by more than one enzyme. The extracellular domain of MUC1* will include most of the PSMGFR sequence but may have an additional 10-20 N-terminal amino acids.

As used herein, "NME family proteins" or "NME family member proteins", numbered 1-10, are proteins grouped together because they all have at least one NDPK (nucleotide diphosphate kinase) domain. In some cases, the NDPK domain is not functional in terms of being able to catalyze the conversion of ATP to ADP. NME proteins were formally known as NM23 proteins, numbered H1, H2 and so on. Herein, the terms NM23 and NME are interchangeable. Herein, terms NME1, NME2, NME6 and NME7 are used to refer to the native protein as well as NME variants. In some cases these variants are more soluble, express better in *E. coli* or are more soluble than the native sequence protein. For example, NME7 as used in the specification can mean the native protein or a variant, such as NME7-AB that has superior commercial applicability because variations allow high yield expression of the soluble, properly folded protein in *E. coli.* "NME1" as referred to herein is interchangeable with "NM23-H1". It is also intended that the invention not be limited by the exact sequence of the NME proteins. The mutant NME1-S120G, also called NM23-S120G, are used interchangeably throughout the application. The S120G mutants and the P96S mutant are preferred because of their preference for dimer formation, but may be referred to herein as NM23 dimers or NME1 dimers.

NME7 as referred to herein is intended to mean native NME7 having a molecular weight of about 42kDa, a cleaved form having a molecular weight between 25 and 33kDa, a variant devoid of the DM10 leader sequence, NME7-AB or a recombinant NME7 protein, or variants thereof whose sequence may be altered to allow for efficient expression or that increase yield, solubility or other characteristics that make the NME7 more effective or commercially more viable.

As used herein, an "an agent that maintains stem cells in the naive state or reverts primed stem cells to the naive state" refers to a protein, small molecule or nucleic acid that alone or in combination maintains stem cells in the naive state, resembling cells of the inner cell mass of an embryo. Examples include but are not limited to NME1 dimers, human or bacterial, NME7, NME7-AB, 2i, 5i, nucleic acids such as siRNA that suppress expression of MBD3, CHD4, BRD4, or JMJD6.

As used herein, in reference to an agent being referred to as a "small molecule", it may be a synthetic chemical or chemically based molecule having a molecular weight between 50Da and 2000Da, more preferably between 150 Da and 1000 Da, still more preferably between 200Da and 750Da.

As used herein, in reference to an agent being referred to as a "natural product", it may be chemical molecule or a biological molecule, so long as the molecule exists in nature.

As used herein, "2i inhibitor" refers to small molecule inhibitors of GSK3-beta and MEK of the MAP kinase signaling pathway. The name 2i was coined in a research article (Silva J et al 2008), however herein "2i" refers to any inhibitor of either GSK3-beta or MEK, as there are many small molecules or biological agents that if they inhibit these targets, have the same effect on pluripotency or tumorigenesis.

As used herein, FGF, FGF-2 or bFGF refer to fibroblast growth factor.

As used herein, "Rho associated kinase inhibitors" may be small molecules, peptides or proteins (Rath N, et al, 2012). Rho kinase inhibitors are abbreviated here and elsewhere as ROCi or ROCKi, or Ri. The use of specific rho kinase inhibitors are meant to be exemplary and can be substituted for any other rho kinase inhibitor.

As used herein, the term "cancer stem cells" or "tumor initiating cells" refers to cancer cells that express levels of genes that have been linked to a more metastatic state or more aggressive cancers. The terms "cancer stem cells" or "tumor initiating cells" can also refer to cancer cells for which far fewer cells are required to give rise to a tumor when transplanted into an animal. Cancer stem cells and tumor initiating cells are often resistant to chemotherapy drugs.

As used herein, the terms "stem/cancer", "cancer-like", "stem-like" refers to a state in which cells acquire characteristics of stem cells or cancer cells, share important elements of the gene expression profile of stem cells, cancer cells or cancer stem cells. Stem-like cells may be somatic cells undergoing induction to a less mature state, such as increasing expression of pluripotency genes. Stem-like cells also refers to cells that have undergone some de-differentiation or are in a meta-stable state from which they can alter their terminal differentiation. Cancer like cells may be cancer cells that have not yet been fully characterized but display morphology and characteristics of cancer cells, such as being able to grow anchorage-independently or being able to give rise to a tumor in an animal.

### Sequence Listing Free Text

As regards the use of nucleotide symbols other than a, g, c, t, they follow the convention set forth in WIPO Standard ST.25, Appendix 2, Table 1, wherein k represents t or g; n represents a, c, t or g; m represents a or c; r represents a or g; s represents c or g; w represents a or t and y represents c or t.
MTPGTQSPFF LLLLLTVLTV VTGSGHASST PGGEKETSAT QRSSVPSSTE KNAVSMTSSV LSSHSPGSGS STTQGQDVTL APATEPASGS AATWGQDVTS VPVTRPALGS TTPPAHDVTS APDNKPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDTRPAPGS TAPPAHGVTS APDNRPALGS TAPPVHNVTS ASGSASGSAS TLVHNGTSAR ATTTPASKST PFSIPSHHSD TPTTLASHST KTDASSTHHS SVPPLTSSNH STSPQLSTGV SFFFLSFHIS NLQFNSSLED PSTDYYQELQ RDISEMFLQI YKQGGFLGLS NIKFRPGSVV VQLTLAFREG TINVHDVETQ FNQYKTEAAS RYNLTISDVS VSDVPFPFSA QSGAGVPGWG IALLVLVCVL VALAIVYLIA LAVCQCRRKN YGQLDIFPAR DTYHPMSEYP TYHTHGRYVP PSSTDRSPYE KVSAGNGGSS LSYTNPAVAA ASANL (SEQ ID NO:1) describes full-length MUC1 Receptor (Mucin 1 precursor, Genbank Accession number: P15941).
MTPGTQSPFFLLLLLTVLT (SEQ ID NO:2)
MTPGTQSPFFLLLLLTVLT VVTA (SEQ ID NO:3)
MTPGTQSPFFLLLLLTVLT VVTG (SEQ ID NO:4)

SEQ ID NOS:2, 3 and 4 describe N-terminal MUC-1 signaling sequence for directing MUC1 receptor and truncated isoforms to cell membrane surface. Up to 3 amino acid residues may be absent at C-terminal end as indicated by variants in SEQ ID NOS:2, 3 and 4.

GTINVHDVETQFNQYKTEAASRYNLTISDVSVSDVPFPFSAQSGAGVPGW GIALLVLVCVLVALAIVYLIALAVCQCRRKNYGQLDIFPARDTYHPMSEYPTYHTHG RYVPPSSTDRSPYEKVSAGNGGSSLSYTNPAVAAASANL (SEQ ID NO:5) describes a truncated MUC1 receptor isoform having nat-PSMGFR at its N-terminus and including the transmembrane and cytoplasmic sequences of a full-length MUC1 receptor.

GTINVHDVETQFNQYKTEAASRYNLTISDVSVSDVPFPFSAQSGA (SEQ ID NO:6) describes the extracellular domain of Native Primary Sequence of the MUC1 Growth Factor Receptor (nat-PSMGFR - an example of "PSMGFR"):
TINVHDVETQFNQYKTEAASRYNLTISDVSVSDVPFPFSAQSGA (SEQ ID NO:7) describes the extracellular domain of Native Primary Sequence of the MUC1 Growth Factor Receptor (nat-PSMGFR - An example of "PSMGFR"), having a single amino acid deletion at the N-terminus of SEQ ID NO:6).

GTINVHDVETQFNQYKTEAASPYNLTISDVSVSDVPFPFSAQSGA (SEQ ID NO:8) describes the extracellular domain of "SPY" functional variant of the native Primary Sequence of the MUC1 Growth Factor Receptor having enhanced stability (var-PSMGFR - An example of "PSMGFR").

TINVHDVETQFNQYKTEAASPYNLTISDVSVSDVPFPFSAQSGA (SEQ ID NO:9) describes the extracellular domain of "SPY" functional variant of the native Primary Sequence of the MUC1 Growth Factor Receptor having enhanced stability (var-PSMGFR - An example of "PSMGFR"), having a single amino acid deletion at the C-terminus of SEQ ID NO:8).
tgtcagtgccgccgaaagaactacgggcagctggacatctttccagcccgggatacctaccatcctatgagcgagta ccccacctaccacacccatgggcgctatgtgccccctagcagtaccgatcgtagcccctatgagaaggtttctgcaggtaacggtggc agcagcctctcttacacaaacccagcagtggcagccgcttctgccaacttg (SEQ ID NO:10) describes MUC1 cytoplasmic domain nucleotide sequence.
CQCRRKNYGQLDIFPARDTYHPMSEYPTYHTHGRYVPPSSTDRSPYEKVS AGNGGSSLSYTNPAVAAASANL (SEQ ID NO:11) describes MUC1 cytoplasmic domain amino acid sequence.
gagatcctgagacaatgaatcatagtgaaagattcgttttcattgcagagtggtatgatccaaatgcttcacttcttcgac gttatgagcttttattttacccaggggatggatctgttgaaatgcatgatgtaaagaatcatcgcacctttttaaagcggaccaaatatgata acctgcacttggaagatttatttataggcaacaaagtgaatgtcttttctcgacaactggtattaattgactatggggatcaatatacagctc gccagctgggcagtaggaaagaaaaaacgctagccctaattaaaccagatgcaatatcaaaggctggagaaataattgaaataataa acaaagctggatttactataaccaaactcaaaatgatgatgctttcaaggaaagaagcattggattttcatgtagatcaccagtcaagacc ctttttcaatgagctgatccagtttattacaactggtcctattattgccatggagattttaagagatgatgctatatgtgaatggaaaagactg ctgggacctgcaaactctggagtggcacgcacagatgcttctgaaagcattagagccctctttggaacagatggcataagaaatgcag cgcatggccctgattcttttgcttctgcggccagagaaatggagttgttttttccttcaagtggaggttgtgggccggcaaacactgctaa atttactaattgtacctgttgcattgttaaaccccatgctgtcagtgaaggtatgttgaatacactatattcagtacattttgttaataggagag caatgtttattttcttgatgtactttatgtatagaaaataa (SEQ ID NO:12) describes NME7 nucleotide sequence (NME7: GENBANK ACCESSION AB209049).
DPETMNHSERFVFIAEWYDPNASLLRRYELLFYPGDGSVEMHDVKNHRT FLKRTKYDNLHLEDLFIGNKVNVFSRQLVLIDYGDQYTARQLGSRKEKTLALIKPDAI SKAGEIIEIINKAGFTITKLKMMMLSRKEALDFHVDHQSRPFFNELIQFITTGPIIAMEIL RDDAICEWKRLLGPANSGVARTDASESIRALFGTDGIRNAAHGPDSFASAAREMELF FPSSGGCGPANTAKFTNCTCCIVKPHAVSEGMLNTLYSVHFVNRRAMFIFLMYFMY RK (SEQ ID NO:13) describes NME7 amino acid sequence (NME7: GENBANK ACCESSION AB209049).
atggtgctactgtctactttagggatcgtctttcaaggcgaggggcctcctatctcaagctgtgatacaggaaccatggc caactgtgagcgtaccttcattgcgatcaaaccagatggggtccagcggggtcttgtgggagagattatcaagcgttttgagcagaaag gattccgccttgttggtctgaaattcatgcaagcttccgaagatcttctcaaggaacactacgttgacctgaaggaccgtccattctttgcc ggcctggtgaaatacatgcactcagggccggtagttgccatggtctgggaggggctgaatgtggtgaagacgggccgagtcatgctc ggggagaccaaccctgcagactccaagcctgggaccatccgtggagacttctgcatacaagttggcaggaacattatacatggcagt gattctgtggagagtgcagagaaggagatcggcttgtggtttcaccctgaggaactggtagattacacgagctgtgctcagaactggat ctatgaatga (SEQ ID NO:14) describes NM23-H1 nucleotide sequence (NM23-H1: GENBANK ACCESSION AF487339).
MVLLSTLGIVFQGEGPPISSCDTGTMANCERTFIAIKPDGVQRGLVGEIIKR FEQKGFRLVGLKFMQASEDLLKEHYVDLKDRPFFAGLVKYMHSGPVVAMVWEGL NVVKTGRVMLGETNPADSKPGTIRGDFCIQVGRNIIHGSDSVESAEKEIGLWFHPEEL VDYTSCAQNWIYE (SEQ ID NO:15) NM23-H1 describes amino acid sequence (NM23-H1: GENBANK ACCESSION AF487339).
atggtgctactgtctactttagggatcgtctttcaaggcgaggggcctcctatctcaagctgtgatacaggaaccatggc caactgtgagcgtaccttcattgcgatcaaaccagatggggtccagcggggtcttgtgggagagattatcaagcgttttgagcagaaag gattccgccttgttggtctgaaattcatgcaagcttccgaagatcttctcaaggaacactacgttgacctgaaggaccgtccattctttgcc ggcctggtgaaatacatgcactcagggccggtagttgccatggtctgggaggggctgaatgtggtgaagacgggccgagtcatgctc ggggagaccaaccctgcagactccaagcctgggaccatccgtggagacttctgcatacaagttggcaggaacattatacatggcggt gattctgtggagagtgcagagaaggagatcggcttgtggtttcaccctgaggaactggtagattacacgagctgtgctcagaactggat ctatgaatga (SEQ ID NO:16) describes NM23-H1 S120G mutant nucleotide sequence (NM23-H1: GENBANK ACCESSION AF487339).
MVLLSTLGIVFQGEGPPISSCDTGTMANCERTFIAIKPDGVQRGLVGEIIKR FEQKGFRLVGLKFMQASEDLLKEHYVDLKDRPFFAGLVKYMHSGPVVAMVWEGL NVVKTGRVMLGETNPADSKPGTIRGDFCIQVGRNIIHGGDSVESAEKEIGLWFHPEEL VDYTSCAQNWIYE (SEQ ID NO:17) describes NM23-H1 S120G mutant amino acid sequence (NM23-H1: GENBANK ACCESSION AF487339).
atggccaacctggagcgcaccttcatcgccatcaagccggacggcgtgcagcgcggcctggtgggcgagatcatc aagcgcttcgagcagaagggattccgcctcgtggccatgaagttcctccgggcctctgaagaacacctgaagcagcactacattgac ctgaaagaccgaccattcttccctgggctggtgaagtacatgaactcagggccggttgtggccatggtctgggaggggctgaacgtg gtgaagacaggccgagtgatgcttggggagaccaatccagcagattcaaagccaggcaccattcgtggggacttctgcattcaggtt ggcaggaacatcattcatggcagtgattcagtaaaaagtgctgaaaaagaaatcagcctatggtttaagcctgaagaactggttgacta caagtcttgtgctcatgactgggtctatgaataa (SEQ ID NO:18) describes NM23-H2 nucleotide sequence (NM23-H2: GENBANK ACCESSION AK313448).
MANLERTFIAIKPDGVQRGLVGEIIKRFEQKGFRLVAMKFLRASEEHLKQH YIDLKDRPFFPGLVKYMNSGPVVAMVWEGLNVVKTGRVMLGETNPADSKPGTIRG DFCIQVGRNIIHGSDSVKSAEKEISLWFKPEELVDYKSCAHDWVYE (SEQ ID NO:19) describes NM23-H2 amino acid sequence (NM23-H2: GENBANK ACCESSION AK313448).

Human NM23-H7-2 sequence optimized for *E. coli* expression:
   (DNA)
   (amino acids)
Human NME7-A:
   (DNA)
   (amino acids)
Human NME7-A1:
   (DNA)
   (amino acids)
Human NME7-A2:
   (DNA)
   (amino acids)
Human NME7-A3:
   (DNA)
   (amino acids)
Human NME7-B:
   (DNA)
   (amino acids)
Human NME7-B1:
   (DNA)
   (amino acids)
Human NME7-B2:
   (DNA)
   (amino acids)
Human NME7-B3:
   (DNA)
   (amino acids)
Human NME7-AB:
   (DNA) (amino acids)
Human NME7-AB1:
   (DNA)
   (amino acids)
Human NME7-A sequence optimized for *E. coli* expression:
   (DNA)
   (amino acids)
Human NME7-A1 sequence optimized for *E. coli* expression:
   (DNA)
   (amino acids)
Human NME7-A2 sequence optimized for *E. coli* expression:
   (DNA)
   (amino acids)
Human NME7-A3 sequence optimized for *E. coli* expression:
   (DNA) (amino acids)
Human NME7-B sequence optimized for *E. coli* expression:
   (DNA)
   (amino acids)
Human NME7-B 1 sequence optimized for *E. coli* expression:
   (DNA)
   (amino acids)
Human NME7-B2 sequence optimized for *E. coli* expression:
   (DNA)
   (amino acids)
Human NME7-B3 sequence optimized for *E. coli* expression:
   (DNA)
   (amino acids)
Human NME7-AB sequence optimized for *E. coli* expression:
   (DNA)
   (amino acids)
Human NME7-AB1 sequence optimized for *E. coli* expression:
   (DNA)
   (amino acids)
Mouse NME6
   (DNA)
   (amino acids)
Human NME6:
   (DNA)
   (amino acids)
Human NME6 1:
   (DNA)
   (amino acids)
Human NME6 2:
   (DNA)
   (amino acids)
Human NME6 3:
   (DNA)
   (amino acids)
Human NME6 sequence optimized for *E. coli* expression:
   (DNA)
   (amino acids)
Human NME6 1 sequence optimized for E. *coli* expression:
   (DNA)
   (amino acids)
Human NME6 2 sequence optimized for E. *coli* expression:
   (DNA)
   (amino acids)
Human NME6 3 sequence optimized for *E. coli* expression:
   (DNA)
   (amino acids)
OriGene-NME7-1 full length
   (DNA)
   (amino acids)
   Abnova NME7-1 Full length (amino acids)
Abnova Partial NME7-B
   (amino acids)
Histidine Tag
   (ctcgag)caccaccaccaccaccactga (SEQ ID NO:84)
Strept II Tag
   (accggt)tggagccatcctcagttcgaaaagtaatga (SEQ ID NO:85)
N-10 peptide:
   QFNQYKTEAASRYNLTISDVSVSDVPFPFSAQSGA (SEQ ID NO:86)
C-10 peptide
   GTINVHDVETQFNQYKTEAASRYNLTISDVSVSDV (SEQ ID NO:87)
   LALIKPDA (SEQ ID NO:88)
   MMMLSRKEALDFHVDHQS (SEQ ID NO:89)
   ALDFHVDHQS (SEQ ID NO:90)
   EILRDDAICEWKRL (SEQ ID NO:91)
   FNELIQFITTGP (SEQ ID NO:92)
   RDDAICEW (SEQ ID NO:93)
   SGVARTDASESIRALFGTDGIRNAA (SEQ ID NO:94)
   ELFFPSSGG (SEQ ID NO:95)
   KFTNCTCCIVKPHAVSEGLLGKILMA (SEQ ID NO:96)
   LMAIRDAGFEISAMQMFNMDRVNVEEFYEVYKGVVT (SEQ ID NO:97)
   EFYEVYKGVVTEYHD (SEQ ID NO:98)
   EIQQNNATKTFREFCGPADPEIARHLRPGTLRAIFGKTKIQNA (SEQ ID NO:99)
   YSGPCVAM (SEQ ID NO:100)
   FREFCGP (SEQ ID NO:101)
   VHCTDLPEDGLLEVQYFFKILDN (SEQ ID NO:102)
   IQNAVHCTD (SEQ ID NO:103)
   TDLPEDGLLEVQYFFKILDN (SEQ ID NO:104)
   PEDGLLEVQYFFK (SEQ ID NO:105)
   EIINKAGFTITK (SEQ ID NO:106)
   MLSRKEALDFHVDHQS (SEQ ID NO:107)
   NELIQFITT (SEQ ID NO:108)
   EILRDDAICEWKRL (SEQ ID NO:109)
   SGVARTDASESIRALFGTDGI (SEQ ID NO:110)
   SGVARTDASES (SEQ ID NO:111)
   ALFGTDGI (SEQ ID NO:112)
   NCTCCIVKPHAVSE (SEQ ID NO:113)
   LGKILMAIRDA (SEQ ID NO:114)
   EISAMQMFNMDRVNVE (SEQ ID NO:115)
   EVYKGVVT (SEQ ID NO:116)
   EYHDMVTE (SEQ ID NO:117)
   EFCGPADPEIARHLR (SEQ ID NO:118)
   AIFGKTKIQNAV (SEQ ID NO:119)
   LPEDGLLEVQYFFKILDN (SEQ ID NO:120)
   GPDSFASAAREMELFFP (SEQ ID NO:121)
Immunizing peptides derived from human NME7
   ICEWKRL (SEQ ID NO:122)
   LGKILMAIRDA (SEQ ID NO:123)
   HAVSEGLLGK (SEQ ID NO:124)
   VTEMYSGP (SEQ ID NO:125)
   NATKTFREF (SEQ ID NO:126)
   AIRDAGFEI (SEQ ID NO:127)
   AICEWKRLLGPAN (SEQ ID NO:128)
   DHQSRPFF (SEQ ID NO:129)
   AICEWKRLLGPAN (SEQ ID NO:130)
   VDHQSRPF (SEQ ID NO:131)
   PDSFAS (SEQ ID NO:132)
   KAGEIIEIINKAGFTITK (SEQ ID NO:133)
Immunizing peptides derived from human NME1
   MANCERTFIAIKPDGVQRGLVGEIIKRFE (SEQ ID NO:134)
   VDLKDRPF (SEQ ID NO:135)
   HGSDSVESAEKEIGLWF (SEQ ID NO:136)
   ERTFIAIKPDGVQRGLVGEIIKRFE (SEQ ID NO:137)
   VDLKDRPFFAGLVKYMHSGPVVAMVWEGLN (SEQ ID NO:138)
   NIIHGSDSVESAEKEIGLWFHPEELV (SEQ ID NO:139)
   KPDGVQRGLVGEII (SEQ ID NO:140)

### Making mouse respond more like a human to drug testing against cancer cells by allowing the human engrafted cancer cells in mouse to contact NME7 or NME7-AB

NME family member proteins can function to promote cancer. Mice, rodents and other animals traditionally, used in drug discovery, do not accurately mimic humans in their response to drugs being tested for efficacy or toxicity. Several 'bad drugs' have been the subject of lawsuits for causing severe adverse reactions in humans, including death, whereas the same drugs showed no toxicities in mice. One reason for the discrepancy between the effect in mice and the effect in humans is that the molecular mechanisms of disease as well as healthy functions is different in mice compared to humans.

We have discovered that human stem cells and human cancer cells grow by the same mechanism. Both cancer cells and pluripotent stem cells overexpress MUC1* which is a potent growth factor receptor (Mahanta S et al 2008). The ligand of MUC1*, NM23-H1, also called NME1, in dimeric form is alone sufficient to make human stem cells grow in the pluripotent state, without the need for feeder cells, conditioned media, or any other growth factors or cytokines (Hikita S et al 2008). We previously showed that NM23-H1 dimers are ligands of the MUC1* growth factor receptor, wherein MUC1* is the remaining transmembrane portion after most of the extra cellular domain has been cleaved and shed from the cell surface. The remaining portion of the extra cellular domain is comprised essentially of the PSMGFR sequence. NM23 dimers bind to and dimerize the MUC1* receptor. Competitive inhibition of the NME-MUC1* interaction, using a synthetic PSMGFR peptide, induced differentiation of pluripotent stem cells, which shows that pluripotent stem cell growth is mediated by the interaction between NM23 dimers and MUC1* growth factor receptor. Similarly, a large percentage of cancers grow by virtually the same mechanism. MUC1 is cleaved to the MUC1* form on over 75% of all human cancers. Like human stem cells, human cancer cells secrete NM23 where, after dimerization, it binds to the MUC1* receptor and stimulates growth of human cancer cells. Competitive inhibition of the interaction by either addition of the PSMGFR peptide or by adding the Fab of an anti-MUC1* (anti-PSMGFR) antibody caused a great reduction in the growth of human tumor cells *in vitro* and *in vivo.*

We discovered a new growth factor of the NME family, NME7, that makes human stem cells grow and inhibits their differentiation, in the absence of FGF or any other growth factor. NME7 is only reportedly expressed in the embryo but not in any adult tissue except in testes. Surprisingly, we discovered that many human cancers, especially aggressive cancers, express NME7 and secrete it in an activated form that has undergone posttranslational cleavage to produce an NME7 species that runs with an apparent molecular weight of ~33kDa. This is in contrast to full-length NME7 that has a calculated molecular weight of ~42kDa and which appears to be restricted to the cytoplasm. Secreted NME7 functions as a growth factor for human stem cells and human cancers. Like NM23 dimers, NME7 binds to and dimerizes the MUC1* growth factor receptor; however, NME7 does so as a monomer as it has two binding sites for the extracellular domain of MUC1*. Thus, human stem cells and human cancers grow via the same growth factors: NM23, also called NME1, in dimer form or NME7.

NME proteins promote growth and pluripotency of embryonic and iPS cells as well as inducing cells to revert to a stem-like state. Because much of the genetic signature of a stem-like state and a cancerous state is now shared (Kumar SM et al 2012, Liu K et al 2013, Yeo JC et al 2014, Oshima N et al 2014, Wang ML et al 2013), we conclude that NME family member proteins are also able to induce a cancerous state. In a preferred embodiment the NME family member protein is NME1 or an NME protein having greater than 30%, 35%, 40%, 45%, 50%, 55%, 60%, 65%, 70%, 75%, 80%, 85%, 90%, 95%, or 97% sequence identity to NME1, wherein said protein is a dimer. In a more preferred embodiment, the NME family member protein is NME7 or an NME protein having greater than 30%, 35%, 40%, 45%, 50%, 55%, 60%, 65%, 70%, 75%, 80%, 85%, 90%, 95%, or 97% sequence identity to at least one of the NME7 domains A or B and able to dimerize the MUC1* growth factor receptor.

Here, we report that NME1 in dimer form, a bacterial NME1 in dimer form, NME7 or NME7-AB were able to: a) fully support human ES or iPS growth and pluripotency, while inhibiting differentiation; b) revert somatic cells to a more stem-like or cancer-like state; and c) transform cancer cells to the highly metastatic cancer stem cell state, also referred to as tumor initiating cells.

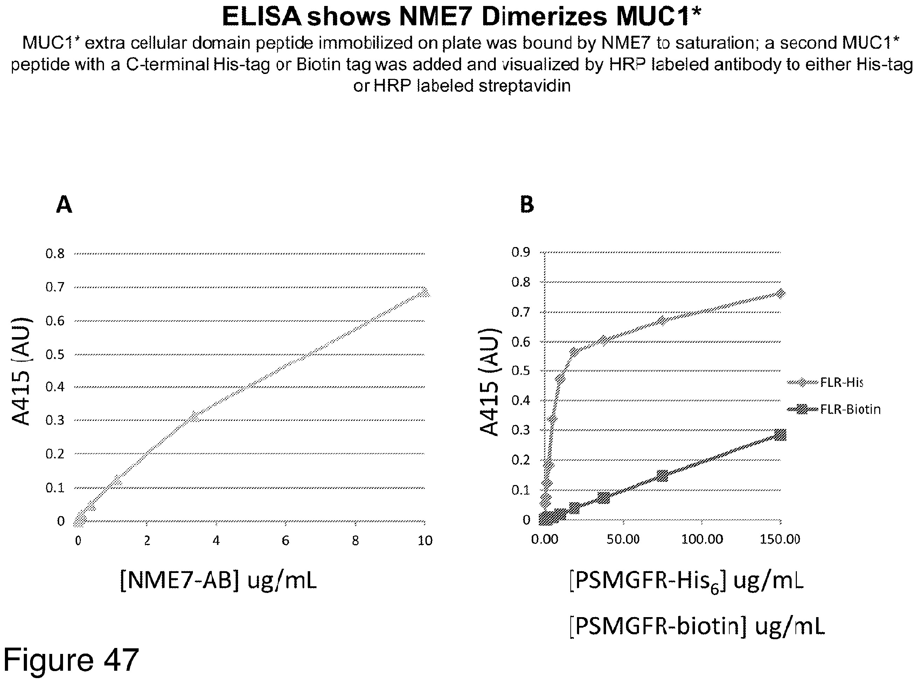

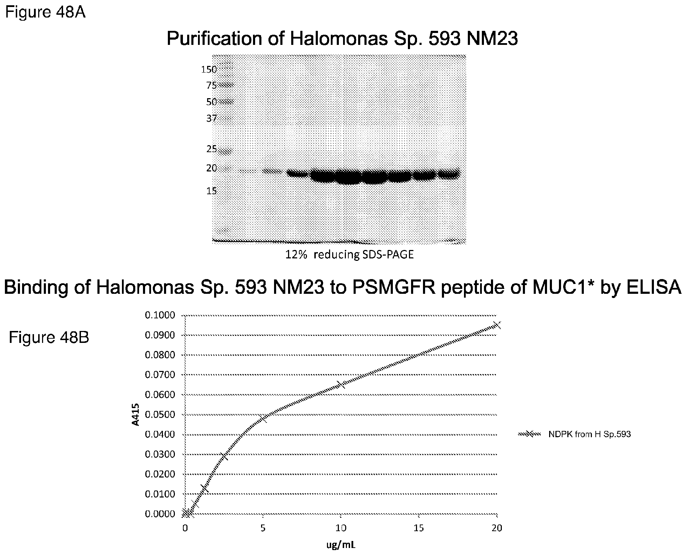

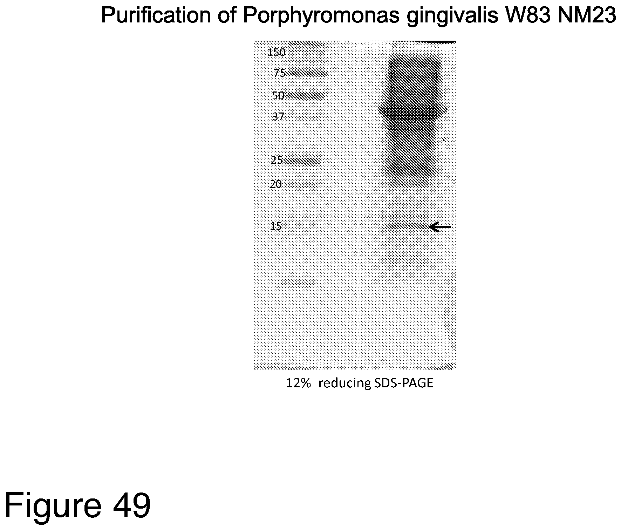

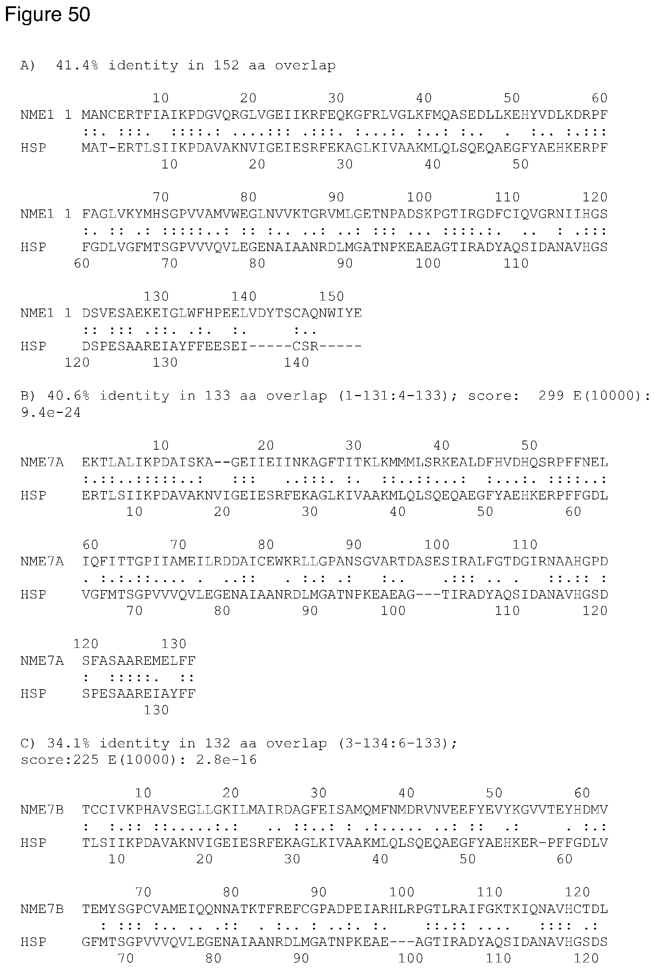

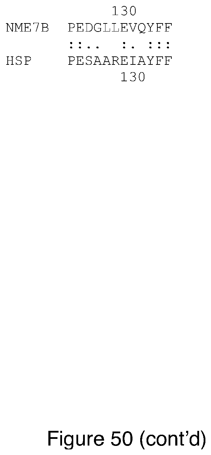

We made recombinant human NM23, also called NME1, dimers that bear the S120G mutation that stabilizes dimers. We previously reported that human NME1 dimers bind to the PSMGFR portion of the extracellular domain of the MUC1* receptor (Smagghe et al. 2013). We also made recombinant human NME7-AB that is secreted as a monomer. **Figure 47** shows that NME7-AB monomers bind to and dimerize the PSMGFR portion of the extracellular domain of the MUC1* receptor. We made recombinant bacterial NME proteins found in *Halomonas* Sp. 593 ('HSP 593') and in *Porphyromonas gingivalis* W83 that had high sequence homology to human NME1 and had been reported to exist in dimer state (**Figure 48** and **Figure 49**). HSP 593 expressed well in *E. coli* and a significant portion was present as a dimer, which population was then purified by FPLC and confirmed the dimer population (**Figure 48a**). A direct binding experiment was performed that showed that bacterial NME from *Halomonas* Sp. 593 bound to the PSMGFR peptide of the MUC1* extracellular domain, **Figure 48b****.** Sequence alignment between HSP 593 and human NME1 or human NME7 domain A or B showed that the bacterial NME that bound to MUC1* extracellular domain was 40-41% identical to human NME1 and human NME7-A, and 34% identical to NME7-B, **Figure 50A-C** .

Additional experiments were performed that showed that bacterial NMEs with greater than 30%, or more preferably 40%, identity to human NME1 or NME7 function like the human NMEs that promote cancer and stem cell growth and survival. Many of the bacterial NMEs that had this high sequence identity to the human NMEs were reported to be implicated in human cancers. Here we report that bacterial NME proteins that have high sequence identity to the human NMEs are able to function like human NMEs to promote the growth of cancers and to promote the transformation of cancer cells to cancer stem cells or more metastatic cancer cells. The bacterial NME was tested in functional assays against human NME1 and NME7.

However, it is known that mouse stem cells grow by a different mechanism than human stem cells. Mouse LIF, leukemia initiating factor, can fully support mouse stem cell growth, whereas LIF has no effect on the growth of human stem cells. The primary growth factor used in the culture of human stem cells is bFGF (Xu C et al 2005), or basic fibroblast growth factor. bFGF cannot support mouse stem cell growth. This shows that the basic biology of mouse stem cell growth is very different from that of human stem cell growth. This fact becomes very important when considering the testing of cancer drugs in mice.

The current practice for testing cancer drugs in mice and other animals is to inject human cancer cells into the animal and either immediately or after several days or weeks of engraftment, inject the animal with the test drug. However, this approach is fundamentally flawed because the host does not naturally produce the growth factors that the human cancer cells need to grow or engraft. Additionally, because the host does not produce the growth factor or the same levels of the growth factor or the human form of the growth factor, drugs being tested in the animals will not have the same effect as they would in humans. Mouse NME7 is only 84% homologous to human NME7 and is not expressed in the adult. Therefore, current xenograft methods for anti-cancer drug testing often fall short in predicting human response to those drugs. This problem could be solved by introducing NM23 dimers or NME7 into the mice so that the human tumor cells have their cognate growth factor to feed the tumor. NM23 dimers or NME7 can be introduced into an animal by a variety of methods. It can be mixed in with the tumor cells prior to implantation, or it can be injected into the animal bearing the tumor.

In a preferred embodiment, a transgenic mouse is generated that expresses human NME7 or a fragment thereof. The NME7 may be carried on an inducible promoter so that the mouse can develop naturally, but expression of the NME7 or the NME7 fragment can be turned on during implantation of human tumors or for the evaluation of drug efficacies or toxicities. In a preferred embodiment, the NME7 species that is introduced to the test animal is NME7-AB.

Alternatively, a transgenic mouse can be made wherein the animal expresses human MUC1, MUC1*, NME7 and/or NM23-H1 or -H2, a variant of H1 or H2 that prefers dimer formation, single chain constructs or other variants that form dimers. Because NME proteins and MUC1 are parts of a feedback loop in humans, wherein expression of one can cause upregulation of the other, it could be advantageous to generate transgenic mice that express human NME protein(s) and MUC1 or the cleaved form MUC1*. A natural or an engineered NME species can be introduced into mice by any of a variety of methods, including generating a transgenic animal, injecting the animal with natural or recombinant NME protein or a variant of an NME protein, wherein NME1 (NM23-H1), NME6 and NME7 proteins or variants are preferred and NME NME1 (NM23-H1), NME6 and NME7 proteins or variants that are able to dimerize MUC1*, specifically the PSMGFR peptide, are especially preferred. In a preferred embodiment, the NME species is a truncated form of NME7 having an approximate molecular weight of 33kDa. In a more preferred embodiment, the NME7 species is devoid of the DM10 domain of its N-terminus. In a still more preferred embodiment, the NME7 species is human.

NME family proteins, especially NME1, NME6 and NME7 are expressed in human cancers where they function as growth factors that promote the growth and metastasis of human cancers. Therefore, human NME protein or active forms of NME protein should be present for proper growth, evolution and evaluation of human cancers and for determining their response to compounds, biologicals or drugs.

### The presence of NME7 increases the engraftment rate of human cancer cells in non-human animals

Another problem with testing of human cancers and drugs to treat them in rodents and other animals is that many human cancers do not easily engraft in the host animal. Many cancer cell lines that are routinely used in *in vitro* testing simply have engraftment rates that are too low for reliable animal studies. For example, T47D breast cancer cell line is typically used for *in vitro* studies because of its overexpression of the oncogene MUC1. However, T47D cells are infrequently used in mouse xenograft studies because of their poor engraftment rate.

Like many human cancers, T47D cells express and secrete NME1 and NME7. A problem that arises when verifying *in vitro* results in animals bearing human tumors is that the rate of T47D engraftment into mice is usually between 25-35%. That means that one needs to start with at least four-times as many mice as the experiment requires and wait several weeks in order to identify those mice whose tumors engraft. More importantly, it is problematic that the engraftment rate is so low and raises the concern that the host is not producing an environment that mimics the human and therefore, the drug testing results may be of limited use.

Engraftment of human cancers into rodents and other test animals is greatly enhanced by the presence of an NME protein, wherein the NME protein can be NME1, NME6 or NME7. There are many methods known to those skilled in the art for introducing the NME protein into a test animal. The NME protein can be injected into the test animal or the transgenic animals that express the NME protein can be generated. In some instances, it is desirable to be able to control the timing of the expression of the NME protein. In these cases, the protein expression may be linked to an inducible genetic element such as a regulatable promoter. In a preferred embodiment, the NME protein that is introduced into a mouse to increase engraftment of human stem cells or cancer cells is human NME7. In a yet more preferred embodiment, the NME7 protein is a fragment that is ~33kDa. In a still more preferred embodiment, the NME protein is human NME7-AB.

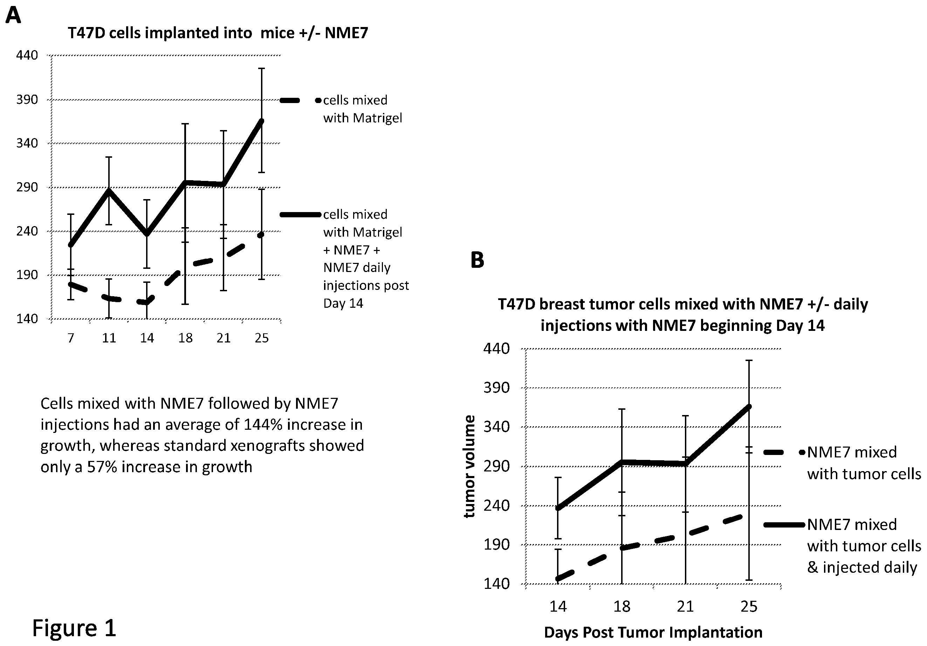

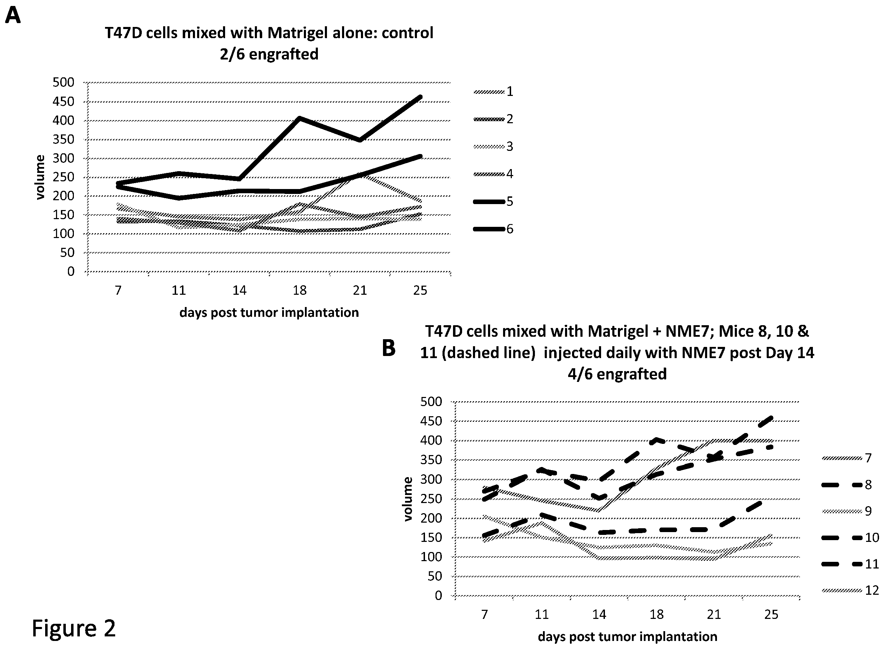

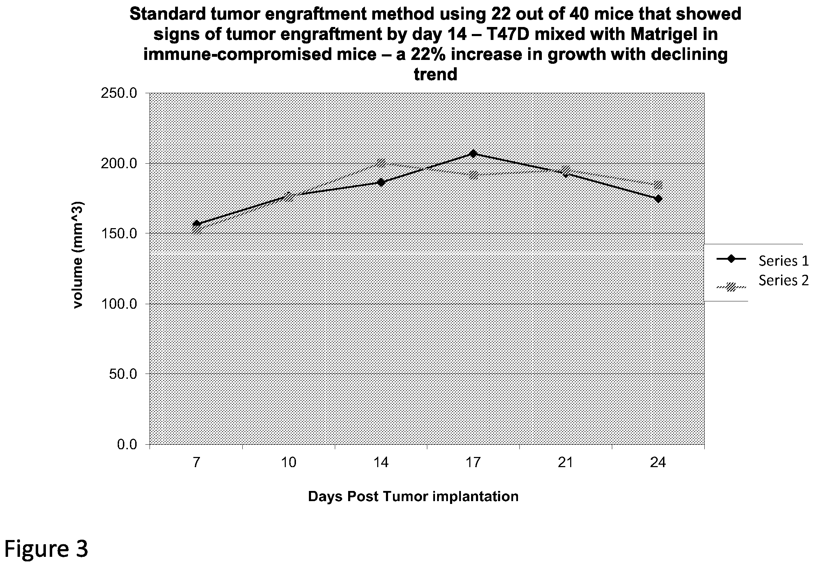

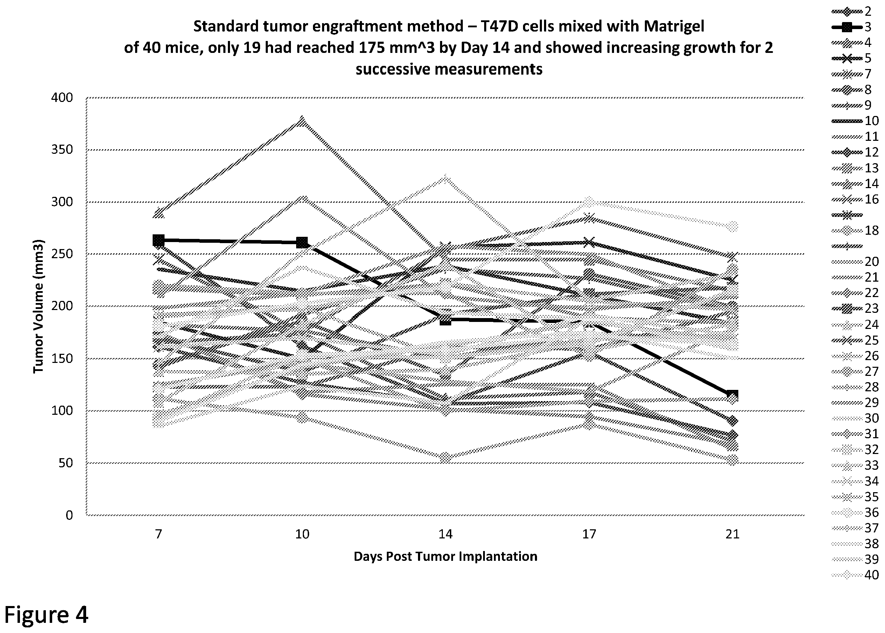

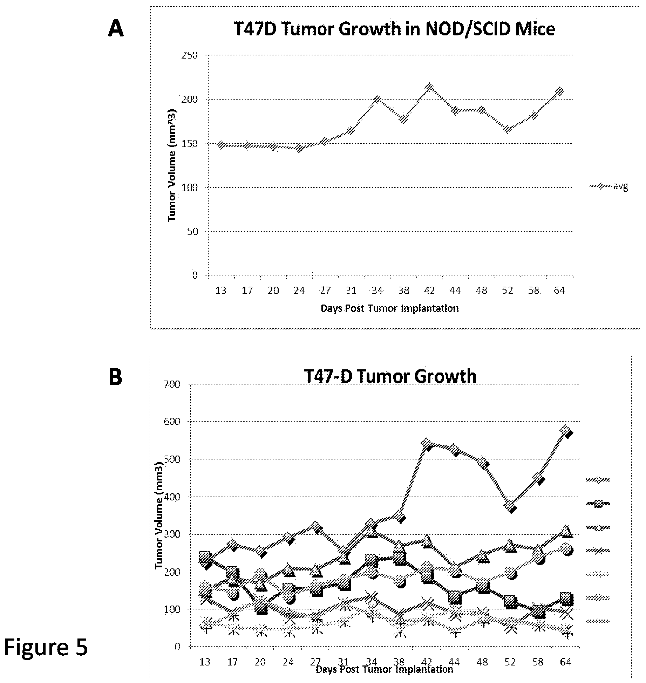

In mouse studies, animals injected with human NME7-AB displayed an increase in the engraftment rate of human tumor cells. Engraftment of MUC1*-positive tumor cells (T47D) was greatly enhanced by mixing the cancer cells with NME7 prior to implantation into the animals and further enhanced by daily injections of NME7 into the tumor-bearing mice. In one experiment, the average growth increase from Day 7 until day 21 was 144% when tumors were mixed with NME7 prior to implantation and 57% without it, see **Figure 1a****.** In the same study, cancer cells were mixed with NME7 prior to implantation alone, or plus NME7 injections into the animal after cell implantation. Results showed that the engraftment rates were 33% and 66% respectively, **Figure 1b****.** **Figure 2** shows graphs of the growth of human tumor cells in individual mice. Panel (A) shows a graph of the growth of T47D breast cancer cells that were mixed with the standard Matrigel. Only two (2) of the six (6) implanted mice showed tumor growth characteristic of engraftment. Panel (B) shows a graph of the growth of T47D breast cancer cells that were mixed with Matrigel and NME7. Four (4) of the six (6) implanted mice showed tumor growth characteristic of engraftment. Dashed lines indicate mice that were also injected with NME7 after Day 14. In another study, performed using 40 immune-compromised mice showed that cancer cells implanted without prior mixing with NME7 and without NME7 injections had about a 25% true engraftment rate. Average growth from Day 7 to Day 24 showed a modest 22% increase in growth, but with a decreasing trend in tumor size. **Figure 3** shows a graph of T47D tumor cells mixed with the standard Matrigel and xenografted into forty (40) immune compromised (nu/nu) mice. The graph shows the average of two identical groups of twenty mice each, with an average increase of 22% in tumor volume but a downward trend. **Figure 4** shows a graph of the growth of the T47D human breast tumor cells in the forty (40) individual mice, with about 25% showing tumor engraftment. **Figure 5** shows graphs of the growth of T47D breast cancer cells mixed with Matrigel and xenografted into the flanks of six (6) NOD/SCID mice. Panel (A) shows average tumor growth. Panel (B) shows tumor growth in individual mice, revealing that only one (1) of six (6) mice had good tumor engraftment using the standard method. These examples demonstrate the difficulty in having human cancer cells engraft into host animals.

### Cancer stem cells

In a related matter, there is currently no practical method for evaluating the efficacy, toxicity or dosing of compounds, biologicals or drugs on 'tumor initiating cells', which are also called 'cancer stem cells'. Cancer stem cells are the minority of cells in a tumor that have the ability to initiate tumor formation. These cells are thought to be the ones responsible for metastasis since a single cell that breaks free from a tumor could in theory give rise to a distant metastasis. Researchers have also developed evidence that cancer stem cells can escape the killing effects of chemotherapy (Fessler S et al 2009, Lissa Nurrul Abdullah and Edward Kai-Hua Chow 2013). Cancer stem cells were first identified by the presence of specific molecular markers such as CD44-high and CD24-low. More recently, the receptor CXCR4 has been identified as a metastatic receptor whose expression is elevated in cancer stem cells and metastatic cancer cells. There are now a panel of genes thought to be markers of cancer stem cells (Miki J et al 2007, Jeter CR et al 2011, Hong X et al 2012, Faber A et al 2013, Mukherjee D et al 2013, Herreros-Villanueva M et al , 2013, Sefah K et al, 2013; Su H-T et al 2013).

The experiment considered to be definitive proof of cancer stem cells is if very small numbers of the cells are able to generate a tumor in an animal. Whereas the typical number of cancer cells required for tumor engraftment into the flank of a nu/nu mouse is 4,000,000 to 6,000,000, cancer stem cells are able to initiate tumor formation from as few as 100 or so cells, albeit in most published reports these cancer stem cells were implanted into the mammary fat pad and required months to develop. Although there are methods for identifying cancer stem cells, to date, there has been no evidence that it is possible to expose cancer cells to an agent or agents which causes them to be transformed to cancer stem cells. Therefore, there is currently no practical way of testing compounds, biologicals or drugs for their ability to inhibit these metastatic cancer stem cells. A vast improvement over the current state of the art would be to develop a way to influence regular cancer cells to rapidly become cancer stem cells so that in addition to the original cancer cells, the metastatic cancer stem cells could also be screened to identify treatments that would inhibit them. In this way a patient could be treated with drugs to inhibit the primary cancer as well with drugs that would inhibit the sub-population of cancer stem cells that could kill the patient years later when the cancer stem cells metastasize.

Agents that are able to revert primed stem cells to the naive state (Nichols J, Smith A 2009, Hanna J et al 2010, Smagghe B et al 2013) or able to maintain naive stem cells in the naive state are also able to transform cancer cells into a more metastatic state also sometimes referred to as cancer stem cells or tumor initiating cells. For example, NME proteins are able to maintain stem cells in the naïve state and are able to revert primed state stem cells to the naive state. In particular, NME1 and NME6 in the dimeric form or NME7 as a monomer are able to maintain stem cells in the naïve state and are also able to transform cancer cells to a more aggressive or metastatic state. NME7 transforms cancer cells into cancer stem cells.

NME1 and NME7 have the ability to transform cancer cells to the more metastatic cancer stem cell state, also called tumor initiating cells. A panel of cancer cells were cultured in a serum-free minimal base media with human NME7-AB or human NME1 dimers ('NM23' in figures) as the only growth factor or cytokine. After several days in this media, cells began to float off the surface and continued to grow in solution. The 'floaters' were collected and separately analyzed by PCR. Cells in other wells were treated with a rho kinase inhibitor ('Ri in figures'). Quantitative PCR measurements show an increase of the cancer stem cell markers, some of which used to be thought of as stem cell markers only.

Others have reported that inhibitors called '2i' (Silva J et al 2008) and '5i' (Theunissen TW et al 2014) are able to maintain stem cells in the naive state. We reasoned that they would also be able to transform cancer cells to a cancer stem cell or metastatic state. Our experiments demonstrate that this is so. Treatment of cancer cells with human recombinant NME7-AB for 7-10 days dramatically increased the expression of markers of cancer stem cells. Similarly, culture in NME1 dimers, bacterial NME1 dimers or treatment with the '2i' inhibitors or '5i' inhibitors caused cancer cells to be transformed into cancer stem cells. 2i refers to inhibitors of the MAP kinase pathway and GSK3 inhibitors such as PD0325901 and CHIR99021. Expression of the metastasis receptor CXCR4 was greatly increased as were other markers of cancer stem cells, such as E-cadherin, Oct4 and Nanog, in some cases. Cancer cells cultured in human NME7-AB increased expression of CXCR4 by nearly 200-fold. In addition, the morphology of the cancer cells changed after exposure to NME1 dimers, NME7, bacterial NME1 dimers or inhibitors that make human primed stem cells revert to the naive state. The NME treated cells, which are normally adherent floated off the surface and grew in suspension. Analysis showed that the cells that remained adherent had less of an increase in the expression of markers of cancer stem cells.

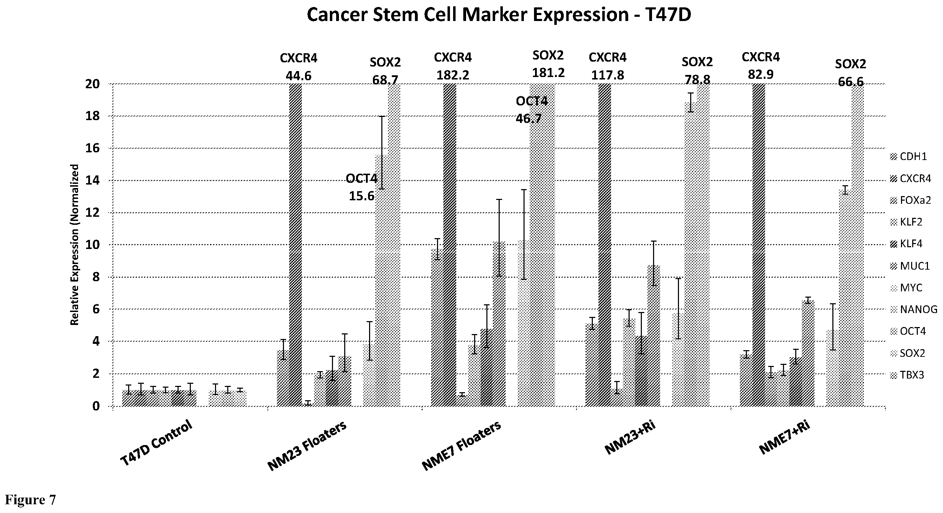

In one experiment, MUC1-positive T47D breast cancer cells were cultured in either normal RPMI growth media, or a serum free minimal media to which was added recombinant human NME7-AB as the single growth factor. A portion of those cells became non-adherent and floated off the surface and were collected while '+ Ri' refers to Rho kinase inhibitor that was added to the media so that all the cells would remain attached to the surface, thus giving an average reading of the adherent and the non-adherent cells. **Figure 6** shows a graph of the RT-PCR measurements of the expression of a panel of genes, reportedly overexpressed in some cancer stem cells or metastatic cancer cells, in the resultant cells. As is shown in the graph, the expression of the metastatic receptor CXCR4 is nearly 200-times the expression level of the T47D cells cultured in normal growth media. CDH1, also called E-cadherin, and MUC1, which have both been implicated in the progression of cancers were both elevated by about 10-fold (Hugo HJ et al 2011). Stem cell markers that have also been reported to be markers of cancer cells were also elevated. OCT4 and SOX2 were increased by about 50-times and 200-times respectively. Other stem cell markers such as NANOG, KLF2, KLF4 and TBX3 showed modest increases in expression. Like NME7, NME1 dimers, also called NM23, can fully support stem cell growth and maintains stem cells in the naive state. Here, we show that NME1 dimers also transform cancer cells to more metastatic, cancer stem cell state. **Figure 7** shows a graph of RT-PCR measurements of T47D breast cancer cells that were cultured in either normal RPMI growth media, or a serum free minimal media to which was added a human recombinant NM23, also called NME1, dimers or NME7-AB as the single growth factor. 'Floaters' refers to those cells that became non-adherent and were collected, while '+ Ri' refers to Rho kinase inhibitor that was added to some cells to make them adhere to the surface. As can be seen in the graph, cancer cells cultured in NME1 dimers or NME7-AB had dramatic increases in the expression of CXCR4, SOX2 and OCT4, followed by increases in expression of CDH1 also called E-cadherin, MUC1, and NANOG. There were modest increases in the expression of stem cell markers KLF2 and KLF4.

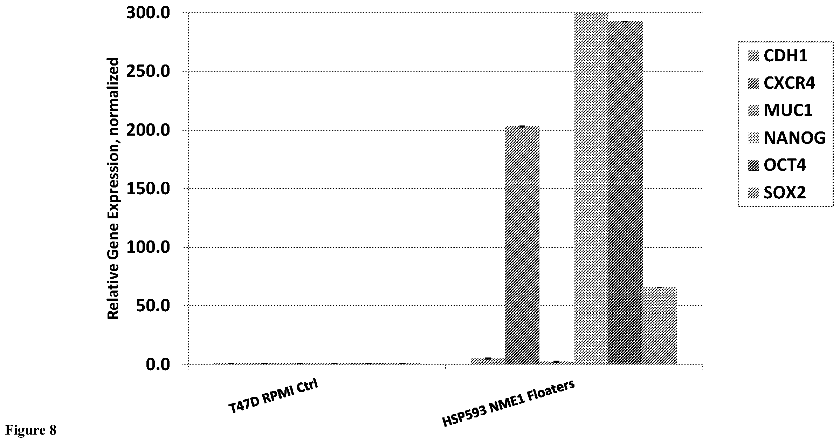

Similarly, bacterium HSP593 expresses an NME1 that forms dimers and can fully support stem cell growth while inhibiting differentiation. Further, it can maintain naive stem cells in the naive state. Here we show that bacterial NME1 also transforms cancer cells to a cancer stem cell state, see **Figure 8****.** Recombinant HSP593 bacterial NME1 added to T47D, MUC1-positive breast cancer cells also caused a morphological change in the cells and also caused the cells to become non-adherent. **Figure 8** shows a graph of RT-PCR measurements showing a dramatic increase in the expression of CXCR4, NANOG, and OCT4, followed by lesser increase in SOX2 and a 5-fold increase in CDH1, also called E-cadherin. In some cases, the proliferation of the cancer stem cells having high expression of the CXCR4 receptor was increased by adding its ligand CXCL12 (Epstein RJ et al 2004, Müller, A et al 2001), to the media.

Others previously reported that kinase inhibitors were able to revert stem cells from a primed state to a naïve state and maintain them in the naive state. 2i inhibitors, which are small molecule inhibitors of GSK3-beta and MEK of the MAP kinase signaling pathway, have been reported (Silva J et al, 2008) to revert mouse primed stem cells to the naive state. We wondered whether these inhibitors could also revert human cancer cells to the cancer stem cell state. Here we report that the 2i inhibitors induce cancer cells to become transformed to cancer stem cells or a more metastatic state. The invention envisions any combination of genes, proteins, nucleic acids or chemical agents that make human stem cells in the primed state revert to the less mature naive state can also be used to transform cancer cells into a more metastatic state often called cancer stem cells or tumor initiating cells.

Here we show that these '2i' inhibitors also transform cancer cells to a cancer stem cell state displaying markers of a metastatic state. **Figure 9** shows that 2i added to T47D breast cancer cells increased expression of the metastasis receptor CXCR4 as well as other stem cell and cancer stem cell markers. The combination of 2i and NM23 dimers or 2i plus NME7-AB increased expression of these markers. However, **Figure 10** shows that NME7-AB alone generates cancer cells with an even higher expression level of the cancer stem cell markers.

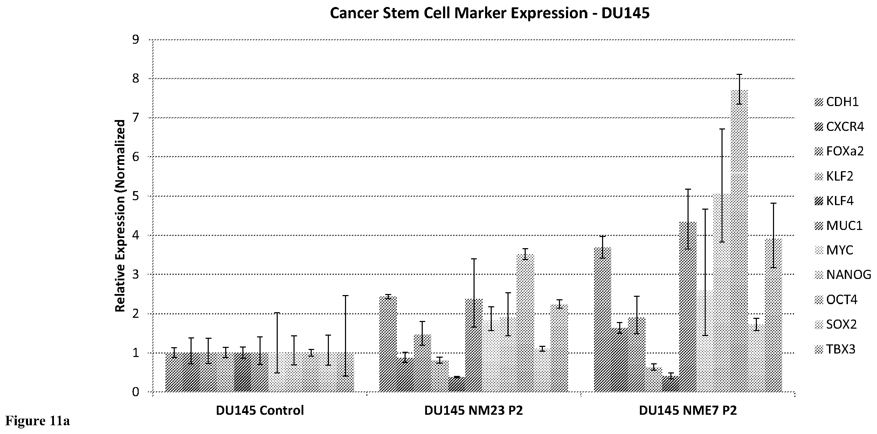

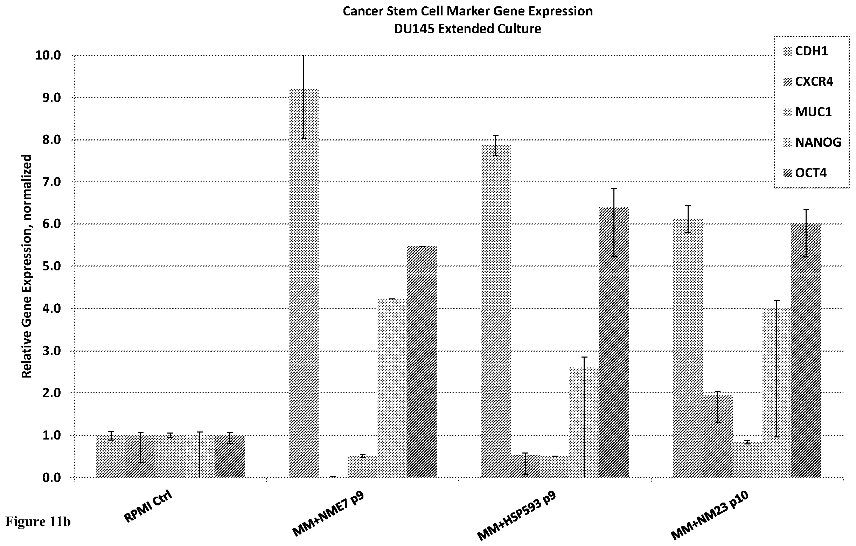

Many types of cancer cells undergo this transition to a more metastatic state when treated with agents such as NME1, NME6 dimers, NME7, 2i or 5i, which are able to revert stem cells from the primed state to the less mature naive state. In one example, DU145 prostate cancer cells that were cultured in regular cancer growth media or serum-free media to which was added one of these agents, also displayed an increase in the expression of stem cell, cancer stem cell and metastatic markers, including CDH1, also called E-cadherin is often elevated in metastatic prostate cancers. Prostate cancer cells were also cultured as described above with either human NME1 dimers, bacterial NME1 dimers, NME7-AB, or 2i inhibitors. Unlike the T47D cells, the prostate cancer cells did not become non-adherent. Thus, the RT-PCR measurements of the cancer stem cell markers are lower than that of the T47D cells, which could be explained by it being the measure of transformed cells and non-transformed cells. In the case of breast cancer cells, we were able to isolate the fully transformed cancer stem cells by collecting the floating cells, but were not able to do so here. DU145 MUC1-positive prostate cancer cells were cultured in RPMI media as a control and in minimal media to which was added recombinant human NME1/NM23 dimers, bacterial HSP593 dimers, or human NME7-AB. **Figure 11a** shows that culture in rhNME1 dimers or rhNME7-AB for 10 days resulted in modest increases in markers of cancer stem cells. There was about a 2-8-fold increase in OCT4, MUC1 and CDH1/E-cadherin. However, after serial passaging under these same culture conditions, the increases in expression of cancer stem cell markers became more pronounced. We reasoned that serial passaging allowed more time for cells to transform since we could not rapidly collect floating cells. **Figure 11b** shows that after 9-10 passages in either rhNME7-AB, bacterial HSP593 NME1 dimers or rhNME1/NM23 dimers, there was a 9-fold, 8-fold and 6-fold increase, respectively, in the expression of CDH1/E-cadherin which is often over expressed in prostate cancers. There were also significant increases in expression of NANOG and OCT4.

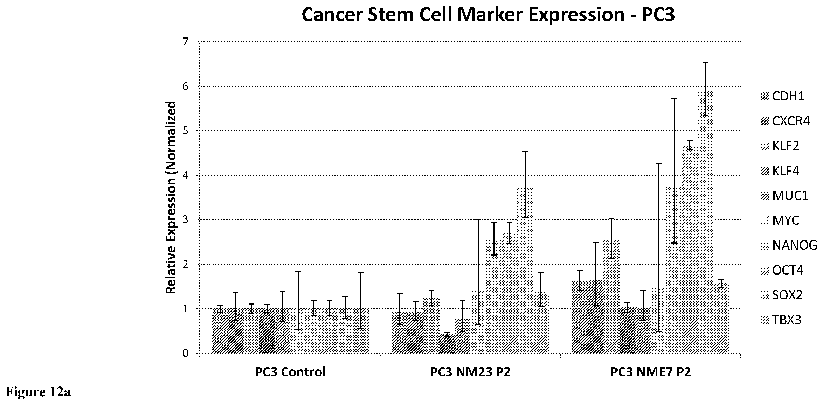

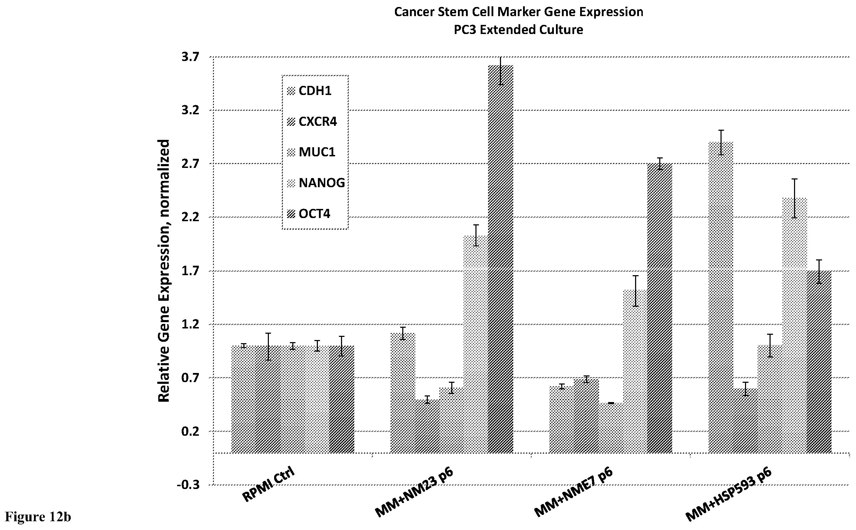

PC3 MUC1-negative prostate cancer cells were also tested for their ability to transform into cancer stem cells when cultured in agents that are able to maintain stem cells in the naïve state. PC3 cells were cultured as described above with either human NME1 dimers, bacterial NME1 dimers or NME7-AB. Like DU145 prostate cancer cells, PC3 cells did not become non-adherent. Thus, the RT-PCR measurements of the cancer stem cell markers may be lower because measurements will be the average of transformed cells and non-transformed cells. **Figure 12a** shows RT-PCR measurements of a subset of genes after passage in rhNME1/NM23 dimers or in rhNME7-AB. The graph of **Figure 12a** shows modest increases in the expression of stem cell markers SOX2, OCT4 and NANOG. Serial passaging in the media did not increase expression of cancer stem cell or stem cell markers, rather they decreased, as is shown in **Figure 12b****.**

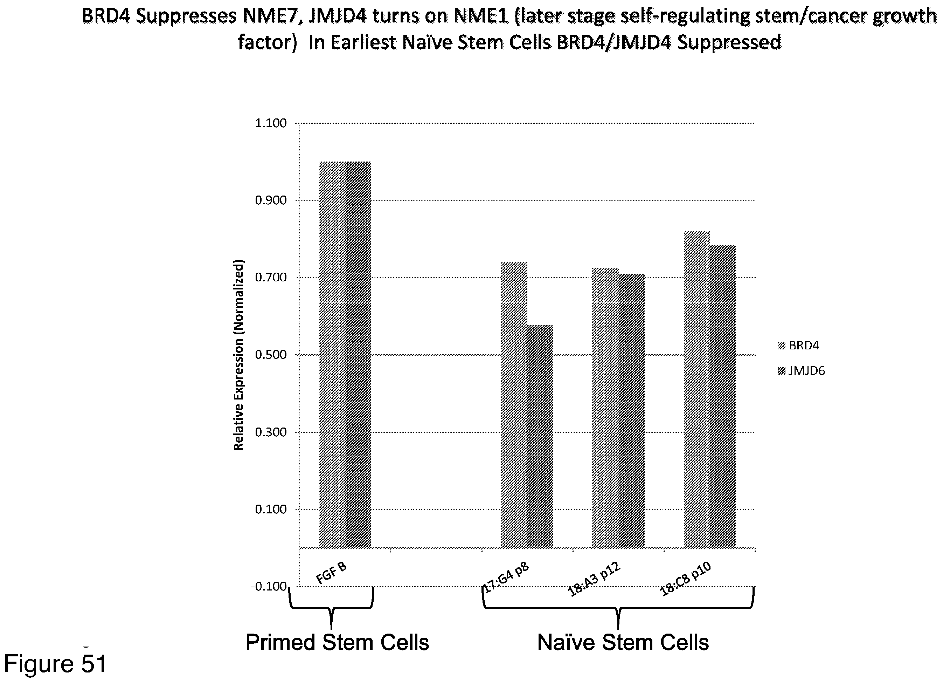

Other agents have been reported to maintain stem cells in the naïve state or revert primed stem cells to the naïve state. Chromatin re-arrangement factors MBD3 and CHD4 were recently reported to block the induction of pluripotency (Rais Y et al, 2013). For example, siRNA suppression of the chromatin re-arrangement factors MBD3 and CHD4 were shown to be key components of a method for reverting human primed stem cells to the naive state. Transcription factors BRD4 and co-factor JMJD6 reportedly suppress NME7 and up-regulate NME1 (Lui W et al, 2013). We found that these factors were expressed at lower levels in naive stem cells than they were in the later stage primed stem cells, **Figure 51****.** This result supports our hypothesis that NME7 is an earlier expressed stem cell growth factor than NME1 because the former cannot turn itself off or regulate self-replication the way NME1 does; as a dimer it activates stem cell growth but when the cells secrete more and it forms hexamers, the hexamers do not bind MUC1* and differentiation is induced.

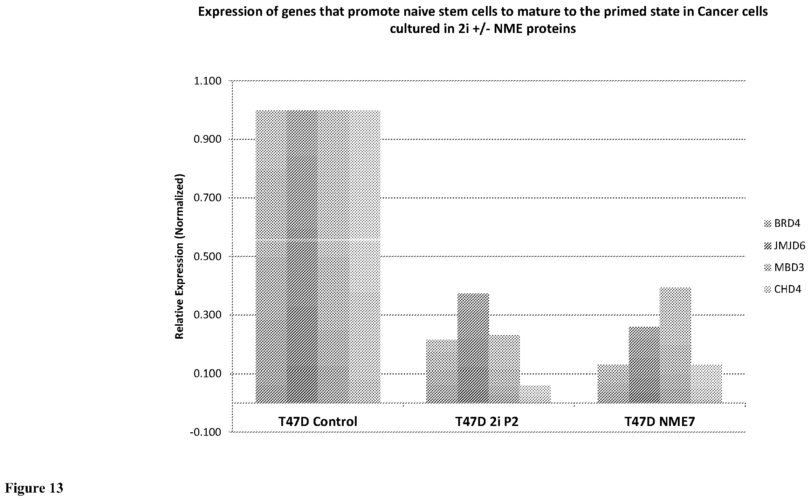

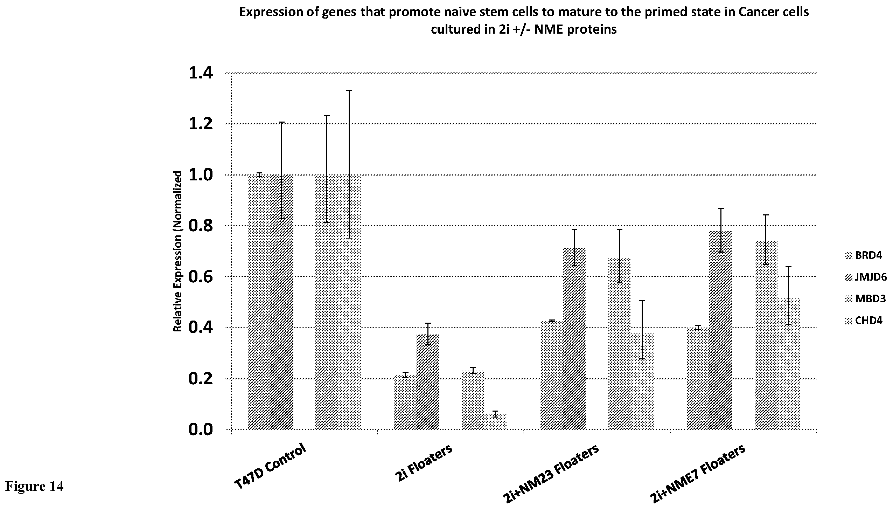

We observed that these four (4) genes, MBD3, CHD4, BRD4 and JMJD6, are naturally suppressed in cancer cells that were cultured in 2i, NME1 dimers or NME7. **Figure 13** shows a graph of one such experiment in which RT-PCR measurements were recorded of the expression of genes that code for chromatin rearrangement factors BRD4, JMJD6, MBD3 and CHD4, in MUC1-positive T47D breast cancer cells cultured in either normal RPMI growth media, or a serum free minimal media to which was added either '2i' or recombinant human NME7-AB wherein the 'floater' cells were the cells analyzed. The combination of 2i and NME1 dimers or NME7 also suppressed BRD4, JMJD6, MBD3 and CHD4 as is shown in **Figure 14****.**

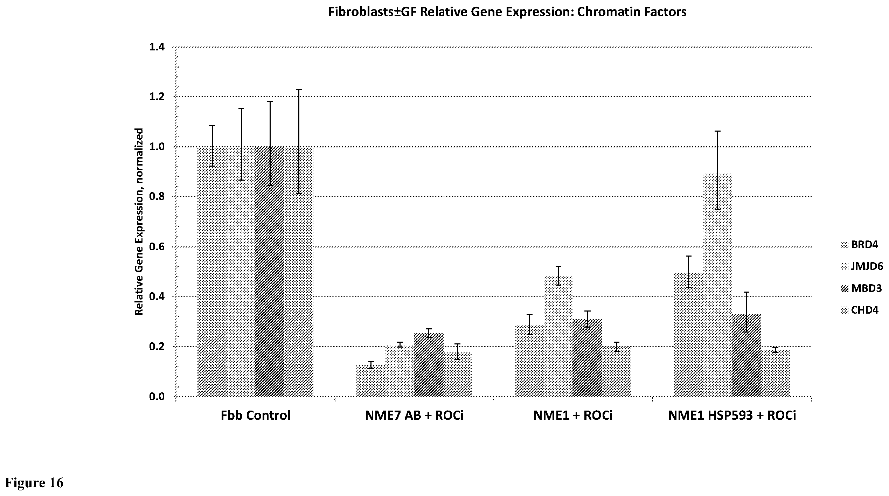

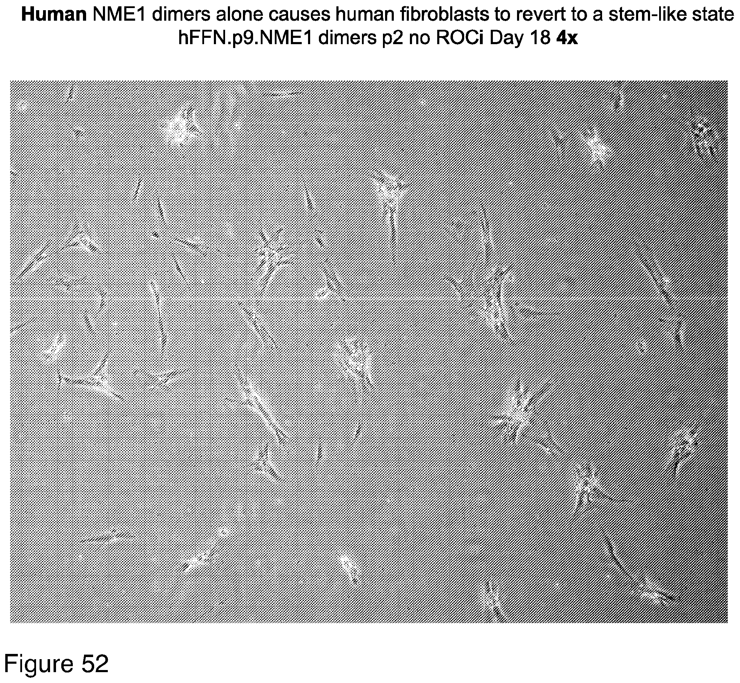

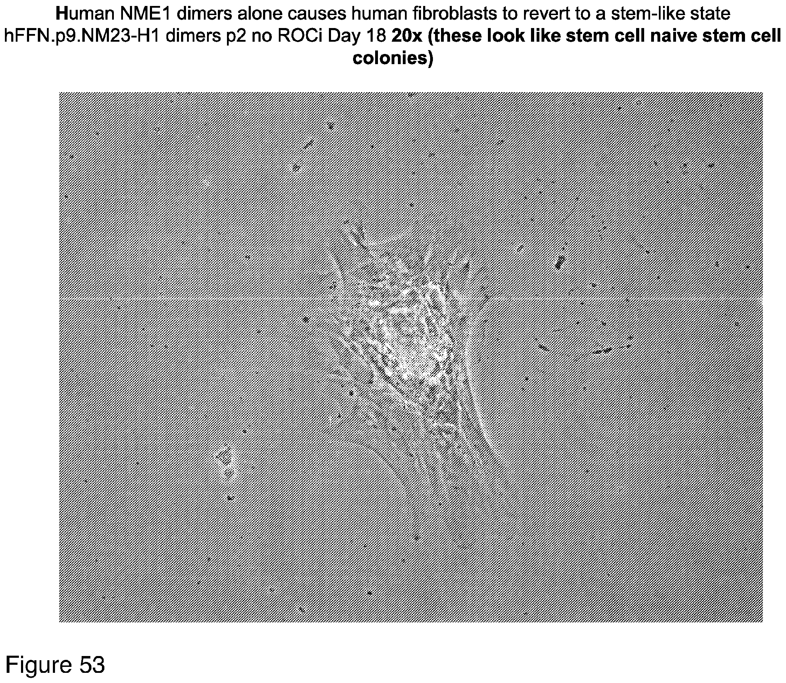

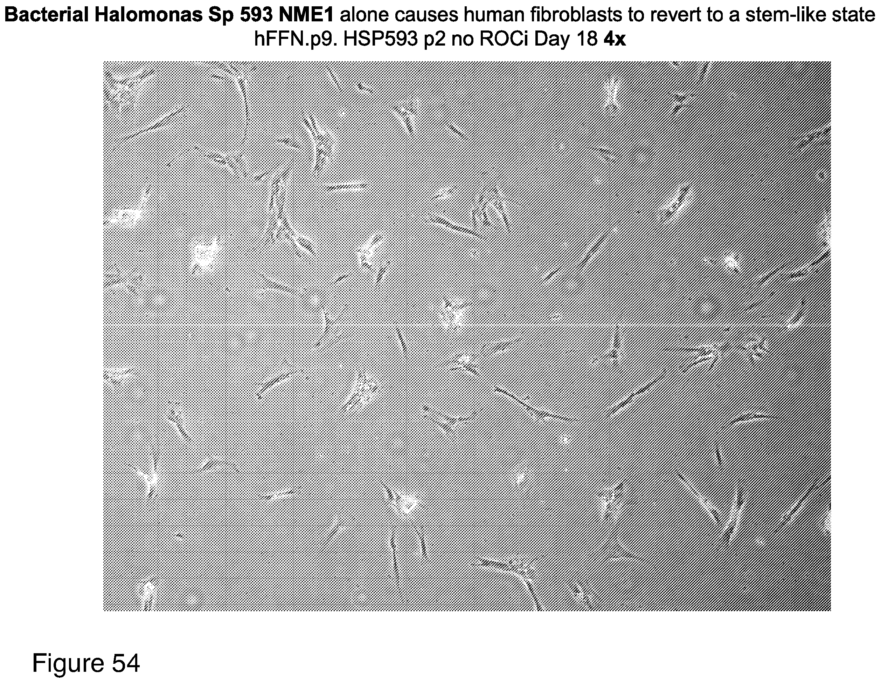

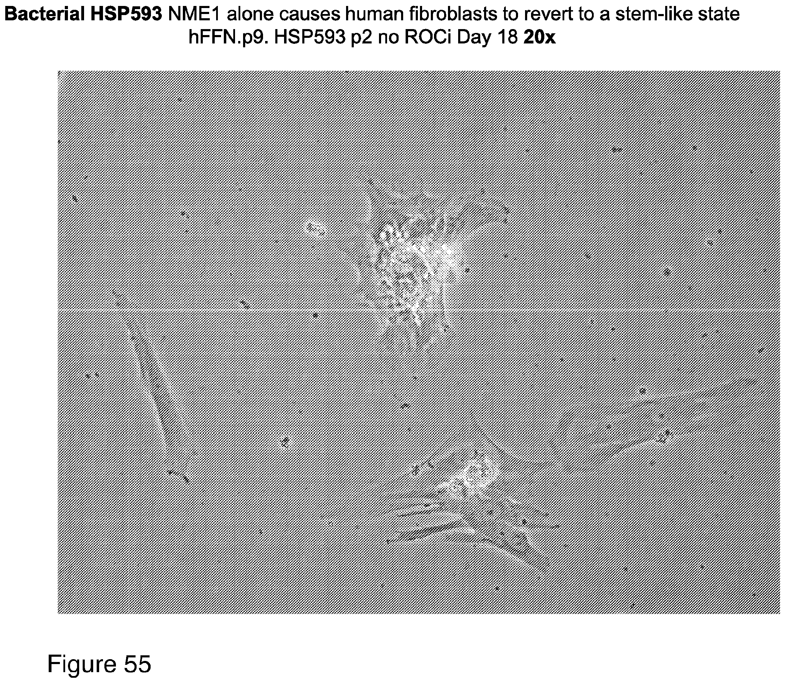

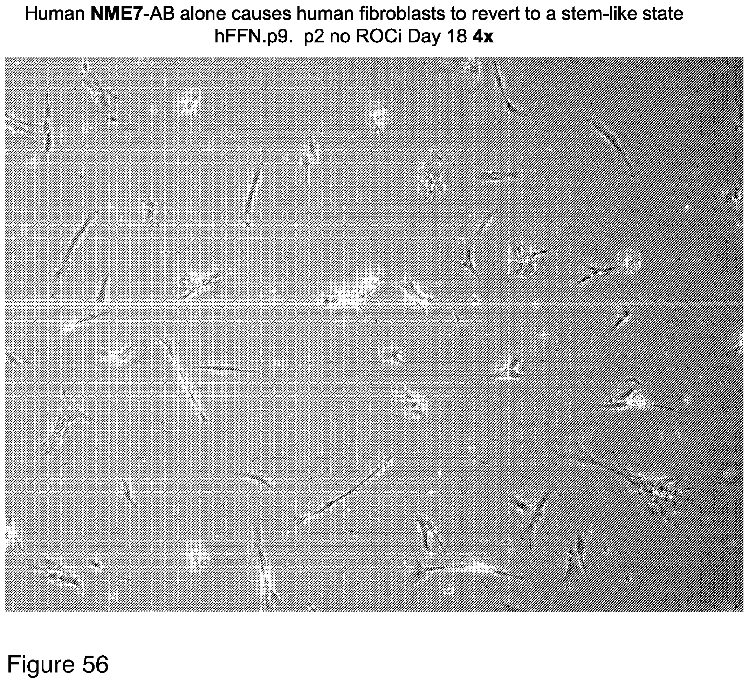

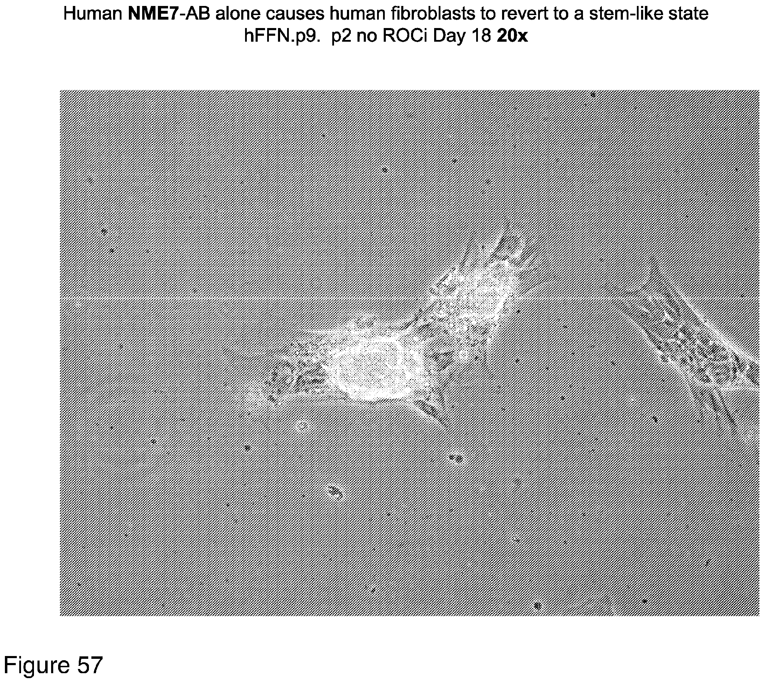

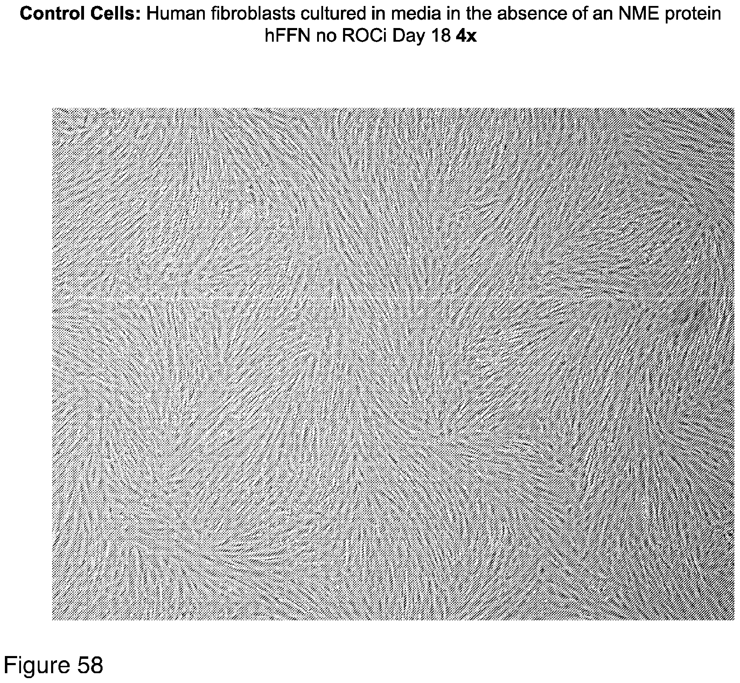

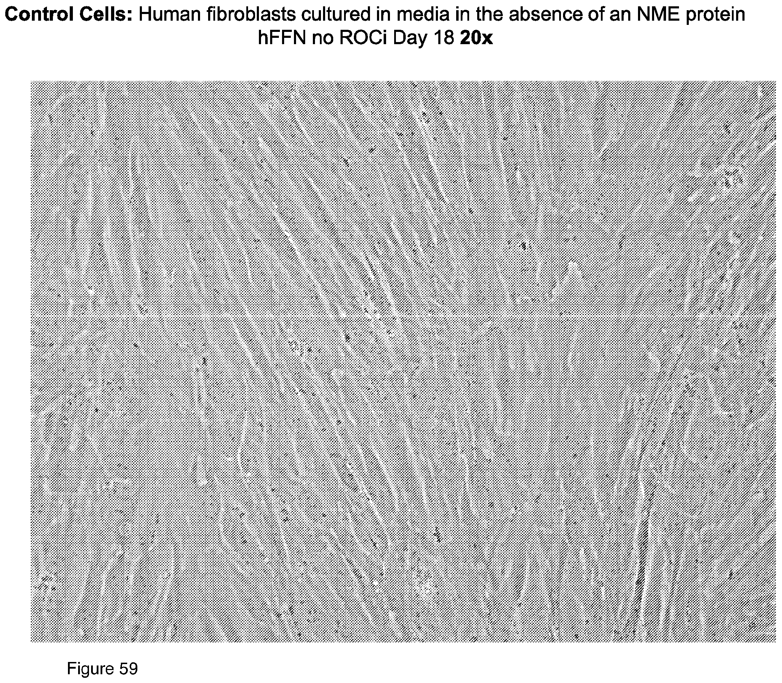

Agents that are able to maintain stem cells in the naive state are also able to transform somatic cells to a cancer or stem-like state. Human NME1 dimer or human NME7 are able to make somatic cells revert to a less mature state, expressing stem and cancer cell markers. Bacterial NME from HSP 593 was tested alongside the human homologs to determine if it could mimic their function by being able to revert somatic cells to a cancer-like state. Human fibroblasts were cultured in a serum-free minimal base media with either HSP 593, human NME1 dimers or human NME7-AB as the only growth factor or cytokine. RT-PCR measurement showed that like the human NMEs, bacterial NME1 HSP 593 reverted somatic cells to an OCT4-positive stage by Day 19. Recalling that stem cells and metastatic cancer cells can grow anchorage-independently, we repeated the experiments but this time a rho kinase inhibitor was added to one set of cells to make the cells adhere to the surface. When the floating cells were forced to adhere to the surface, RT-PCR showed that there had actually been a 7-fold increase in stem/cancer marker OCT4 and as high as a 12-fold increase in the stem/cancer markers Nanog. Photos of the experiment show the dramatic change in morphology as the fibroblasts revert when cultured in human or bacterial NME, **Figures 52-****59.** The relative order of efficiency of reverting somatic cells to a less mature state was NME7 > NME1 dimers > NME1 bacterial. RT-PCR measurements of human fibroblasts grown in the human NME1 or NME7 or bacterial NME1 show that the NME proteins suppress all four (BRD4, JMJD6, MBD3 and CHD4) blockers of pluripotency. Composite graphs of RT-PCR experiments show that the relative potency of increasing pluripotency genes and decreasing pluripotency blockers is NME7 > NME1 > HSP 593 NME. However, Bacterial NME from HSP 593 apparently up-regulates expression of human NME7 and NME1. **Figure 15** shows a graph of RT-PCR measurements of the expression of genes, reportedly overexpressed in some cancer stem cells or metastatic cancer cells, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface. 'Minus ROCi' does not refer to floaters but rather refers to cells that remained adherent in the absence of a rho kinase inhibitor. **Figure 16** shows a graph of RT-PCR measurements of the expression of genes that code for chromatin rearrangement factors BRD4, JMJD6, MBD3 and CHD4, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface. **Figure 17** shows a graph of RT-PCR measurements of the expression of genes, reportedly overexpressed in some cancer stem cells or metastatic cancer cells and genes that code for chromatin rearrangement factors BRD4, JMJD6, MBD3 and CHD4, in somatic fibroblast, 'fbb' cells that were cultured in either normal fibroblast growth media, or a serum free minimal media to which was added a human recombinant NM23, also called NME1, dimers, NME7-AB, or HSP593 bacterial NME1 dimers. '+ ROCi' refers to Rho kinase inhibitor that was added to some cells to make them adhere to the surface. 'Minus ROCi' refers to cells that became non-adherent and floated off the surface.

Agents that transform cancer cells to a more metastatic cancer stem cell state decrease expression of pluripotency-blocking genes BRD4, JMJD6, MBD3 and CHD4. These agents also decreased expression of BRD4, JMJD6, MBD3 and CHD4 in somatic cells. Thus, NME1 dimers, NME7 and bacterial NME1 dimers cause somatic cells to revert to a less mature cancer/stem-like state.

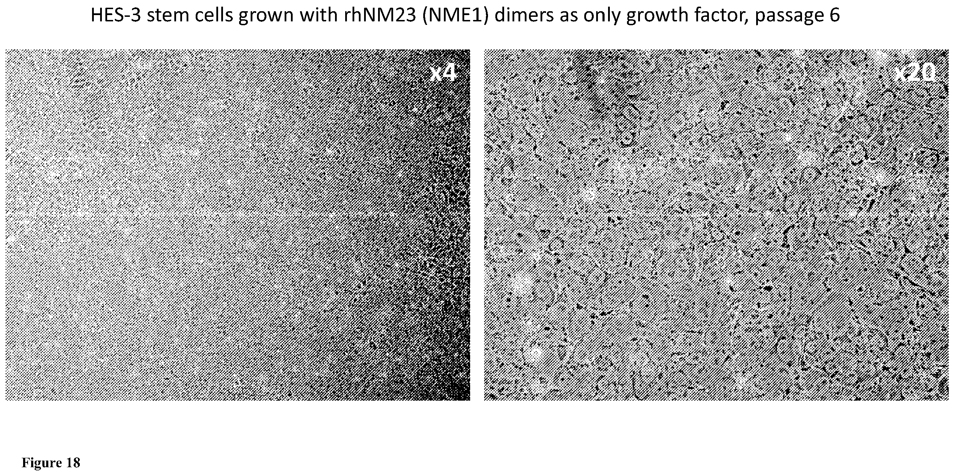

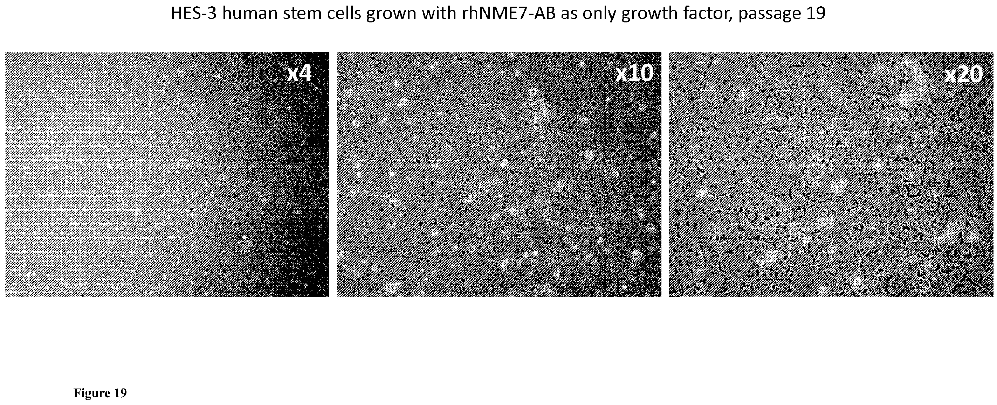

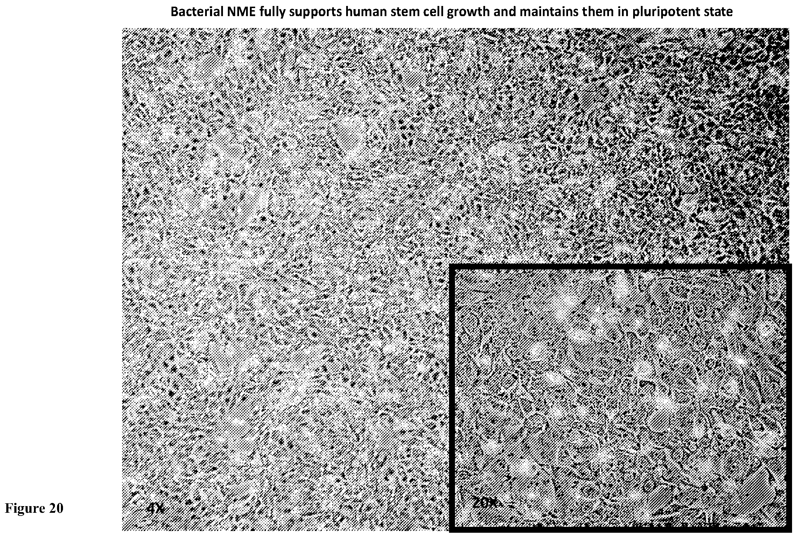

Factors that are able to maintain stem cells in the naive state are also able to transform cells to a cancer-like or stem-like state and are also able to transform cancer cells to a cancer stem cell state or a more metastatic state. Herein, we have shown several examples of bacterial NME1 being able to function like human NME1 dimers, which are also able to function like NME7 or NME7-AB. However, the primary example that these factors function in a very similar way is their ability to promote the growth of stem cells, inhibit differentiation and maintain them in the naive state. We have demonstrated that human NME1 dimers, also called NM23-H1, bacterial NME1 and NME7-AB promote the growth of embryonic stem cells and induced pluripotent stem cells, inhibit their differentiation and maintain them in a naive state as evidenced by global genetic analysis, having both X chromosomes in the active state if stem cell donor is human and by having the ability to form teratomas in a host animal. Human HES-3 embryonic stem cells were cultured in a serum-free minimal base media with either HSP 593, human NME1 or NME7- dimers or human NME7-AB as the only growth factor or cytokine. Just as human NME1 and NME7 fully supported human stem cell growth, so did bacterial NME from HSP 593. **Figure 18** shows a photograph of pluripotent human embryonic stem cells that have been serially cultured in recombinant human NME1 dimers. **Figure 19** shows a photograph of pluripotent human embryonic stem cells that have been cultured for ten (10) or more passages in recombinant human NME7-AB. **Figure 20** shows a photograph of pluripotent human embryonic stem cells that have been cultured for ten (10) or more passages in recombinant bacterial NME1.

Several examples have been presented here that indicate that contacting cells with an agent or agents that are able to revert stem cells from the primed state to the less mature naive state are also able to revert a wide variety of cell types to a less mature state: somatic cells to stem or progenitor cells or cancer cells to more metastatic state also called cancer stem cell state. Therefore, the invention is not meant to be limited to a specific cell type or a specific cancer cell type.

A method for the identification or testing of agents to treat cancer, and metastatic cancer in particular, is comprised of the steps of: 1) contacting cancer cells with an entity that is able to maintain stem cells in the naïve state; 2) contacting the cancer cells with an agent; 3) assessing the ability of the agent to inhibit the growth or metastatic potential of the cancer cells; 4) concluding that agents that inhibit the growth or metastatic potential of the cancer cells are suitable for treating a patient with cancer; and 5) administering the agent to the patient. An inhibition of the metastatic potential of cancer cells is indicated by a decrease in the expression of CXCR4, E-cadherin, MUC1, NME1, NME7 or stem cell markers OCT4, SOX2, NANOG, KLF2 or KLF4, or an increase in the expression of MBD3, CHD4, BRD4 or JMJD6.

Alternatively, cells that have been cultured in the presence of an agent that is able to maintain stem cells in a naive state are then transplanted into a test animal. Since cancer cells cultured in the presence of an agent that maintains stem cells in the naive state become cancer stem cells and more metastatic, they can be implanted at very low numbers into test animals. Drugs and drug candidates can then be tested for efficacy, toxicity or to establish dosing regimens in animals implanted with cancer stem cells generated in this way.

In one experiment, T47D breast cancer cells were cultured with human recombinant NME7-AB for 7 days. The floating cells were collected and analyzed by RT-PCR which showed an increase in the expression of genes associated with cancer stem cells. In particular the expression of the metastasis receptor CXCR4, which is a key indicator of metastatic potential, was 130-times higher than that of the parent cells. These cells were implanted into immune-deficient, nu/nu female mice. The number of cells implanted was 50, 100, 1,000 or 10,000. Recall that 4,000,000 to 6,000,000 cells are normally required to get tumor engraftment, whereas as few as 100 cancer stem cells have been known to give rise to a tumor in an immune-compromised mouse, albeit with very slow growth rates, such as several months for tumor development.

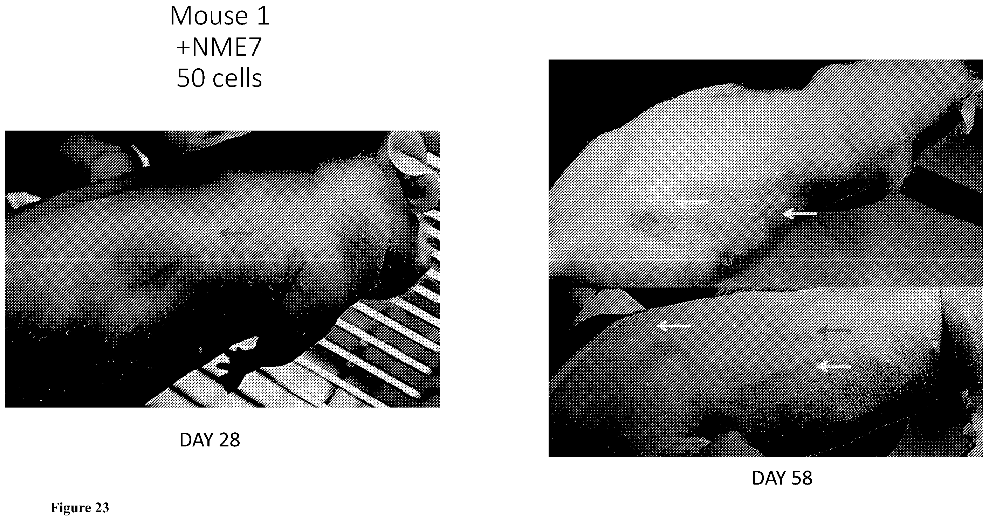

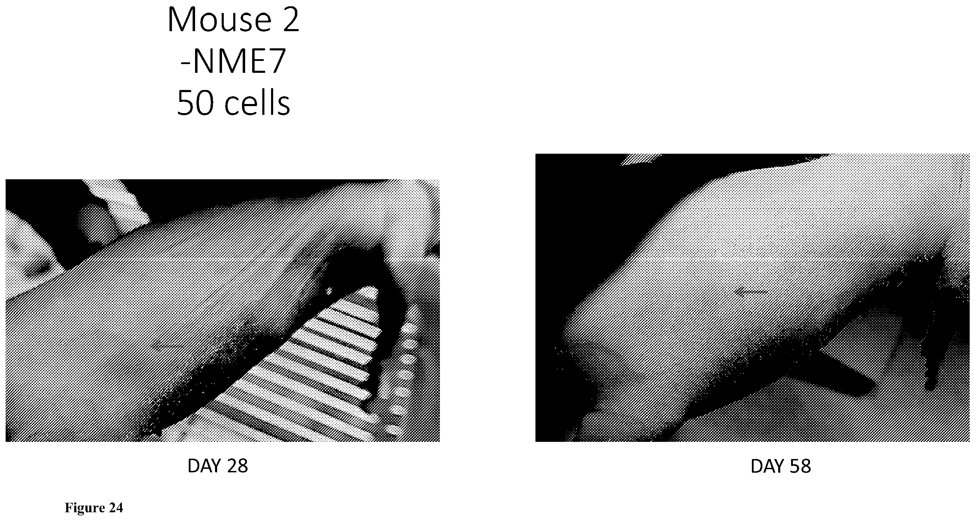

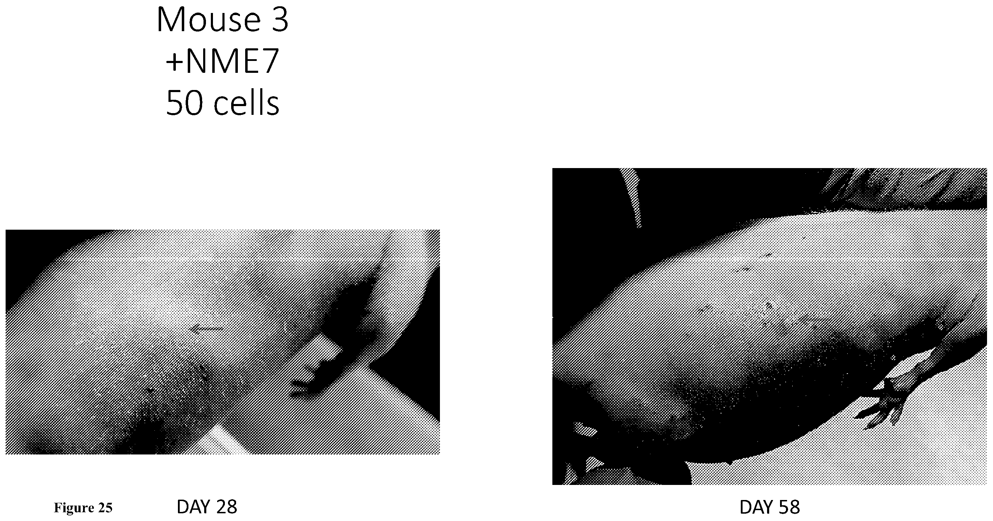

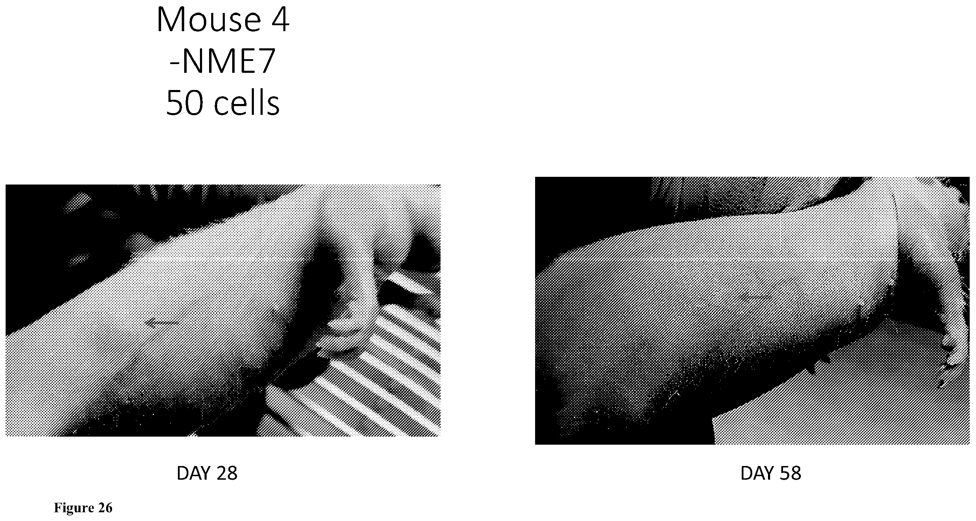

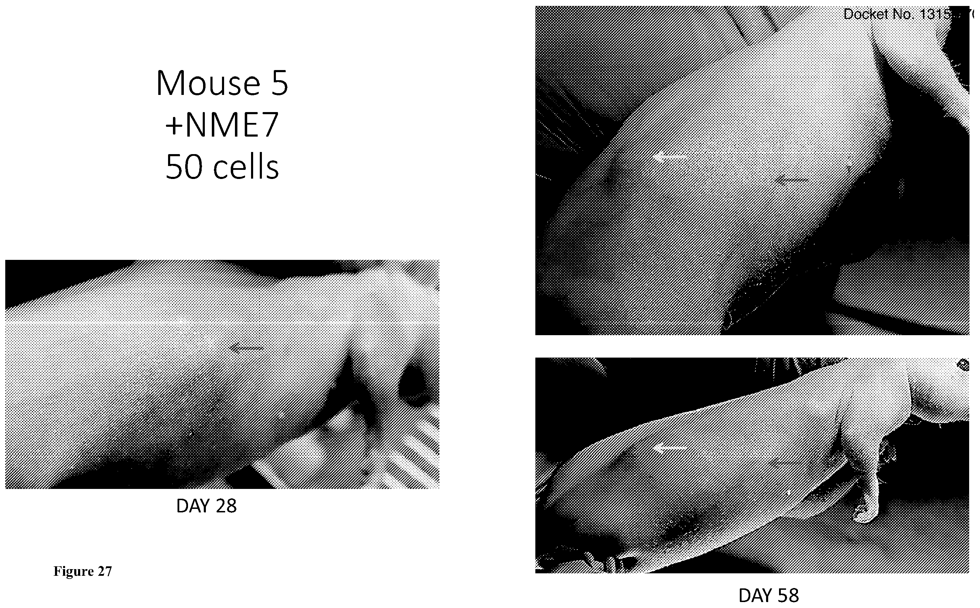

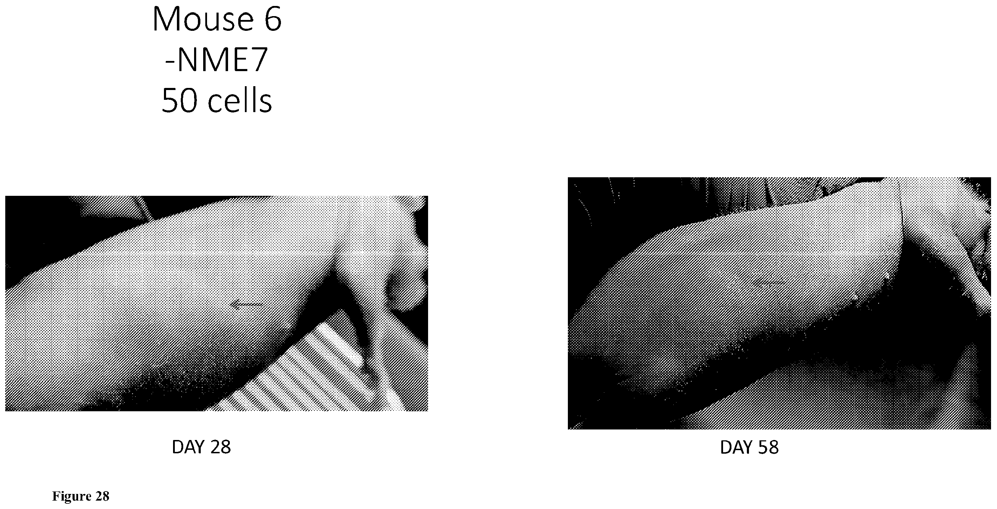

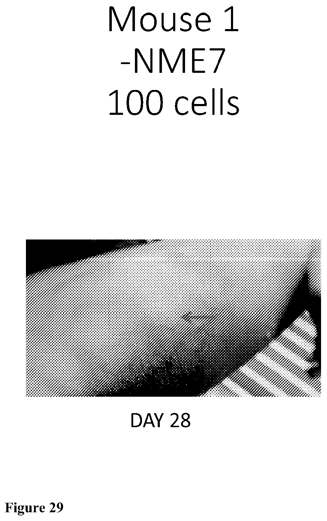

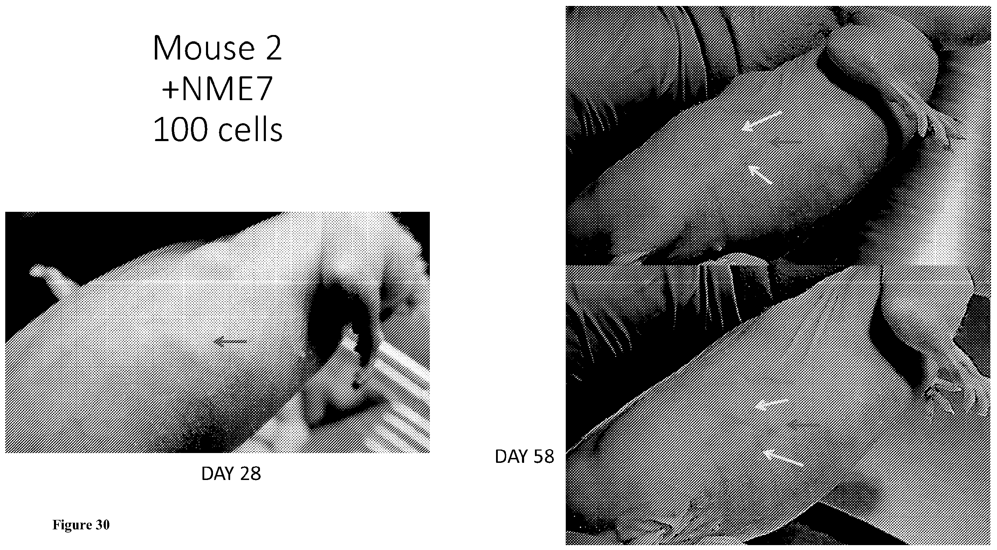

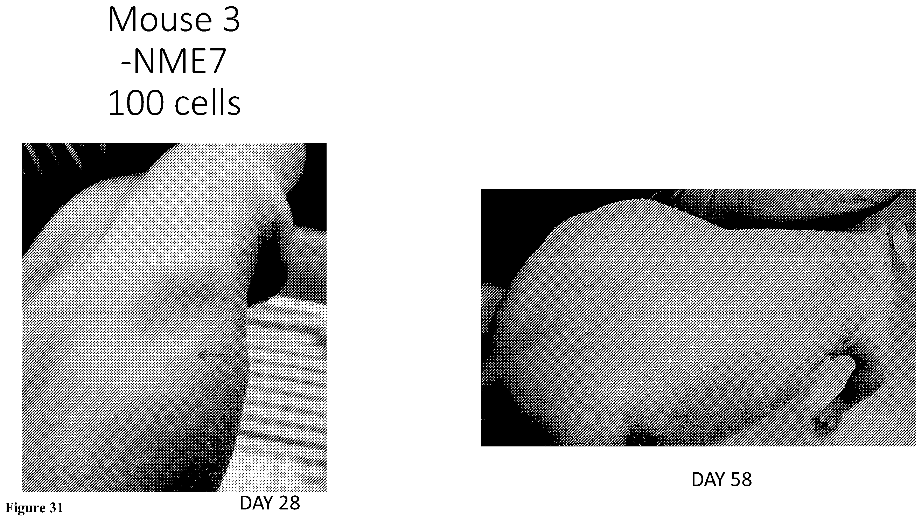

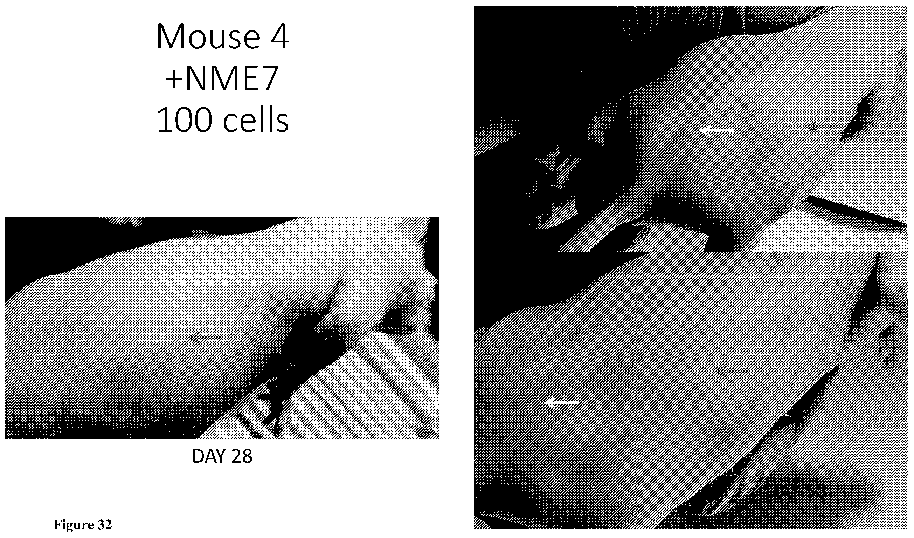

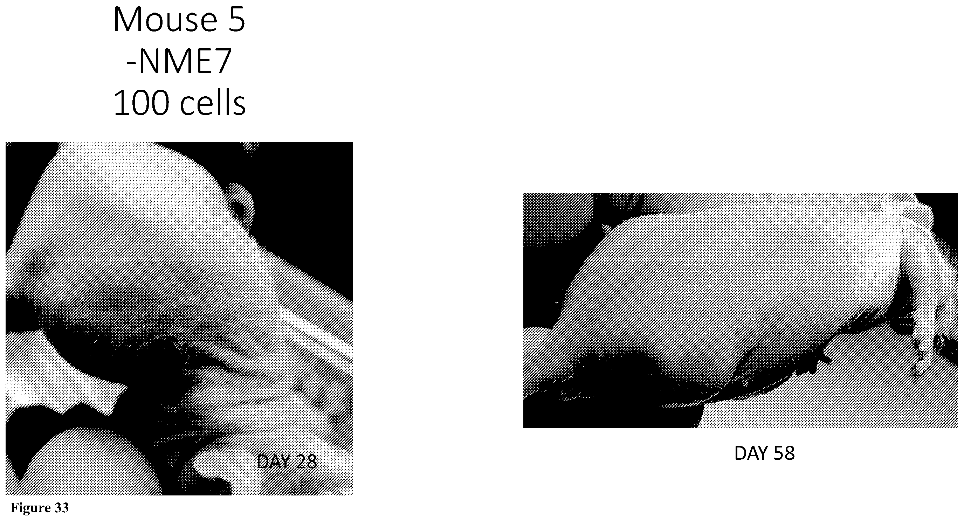

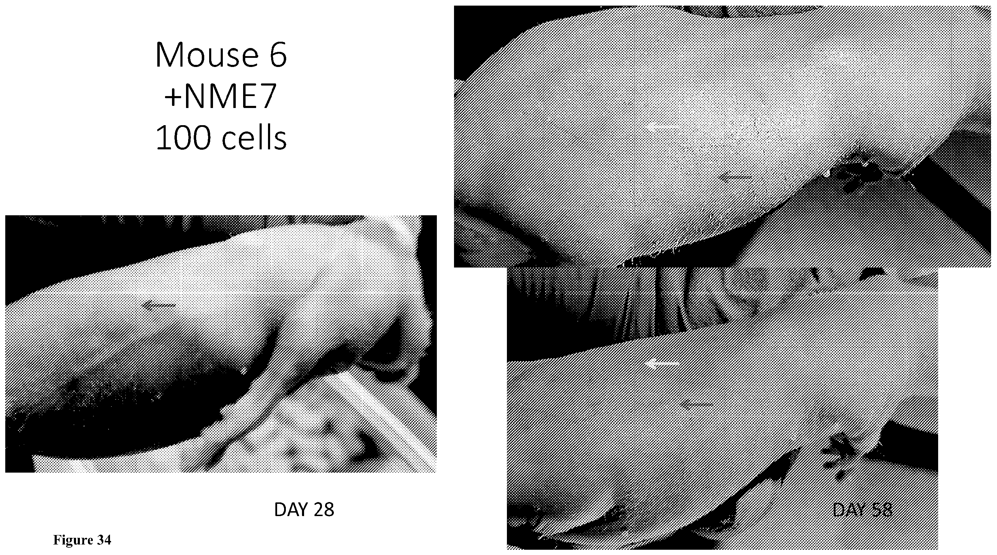

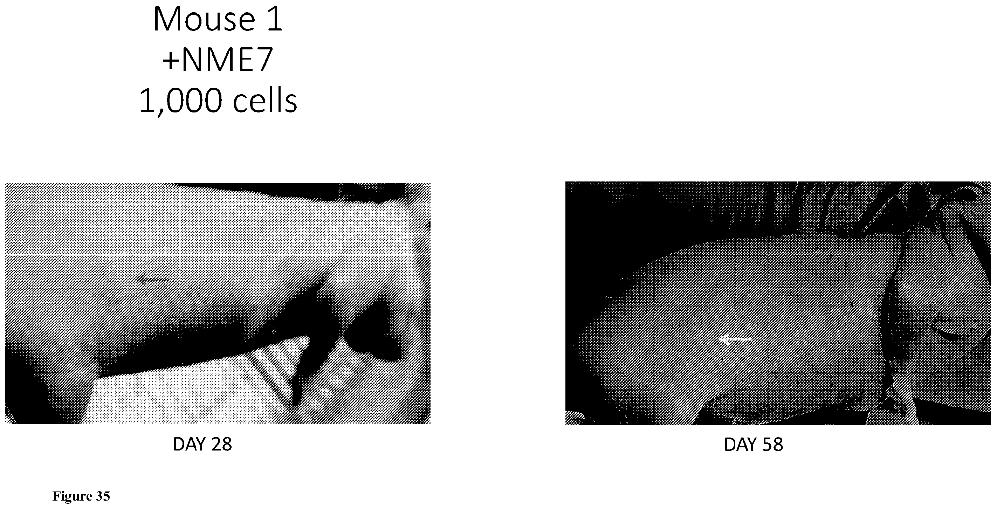

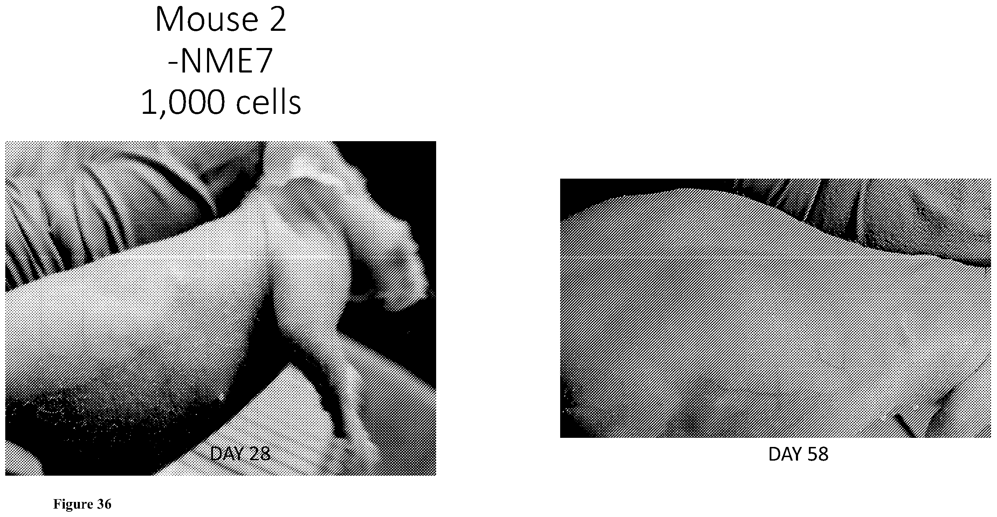

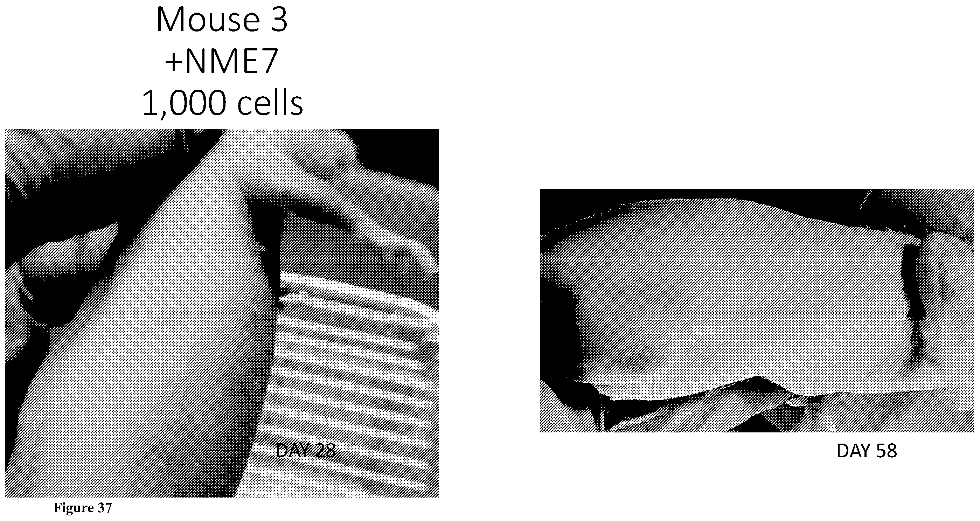

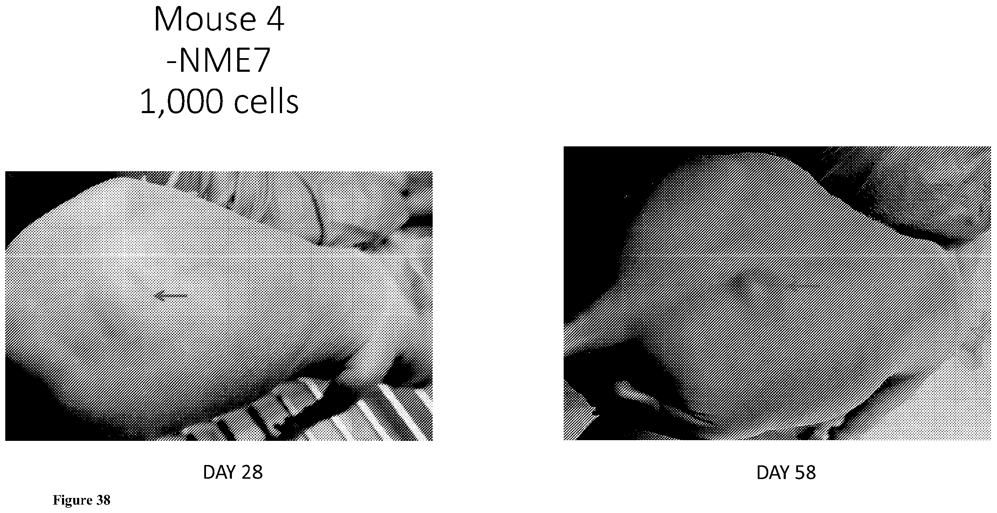

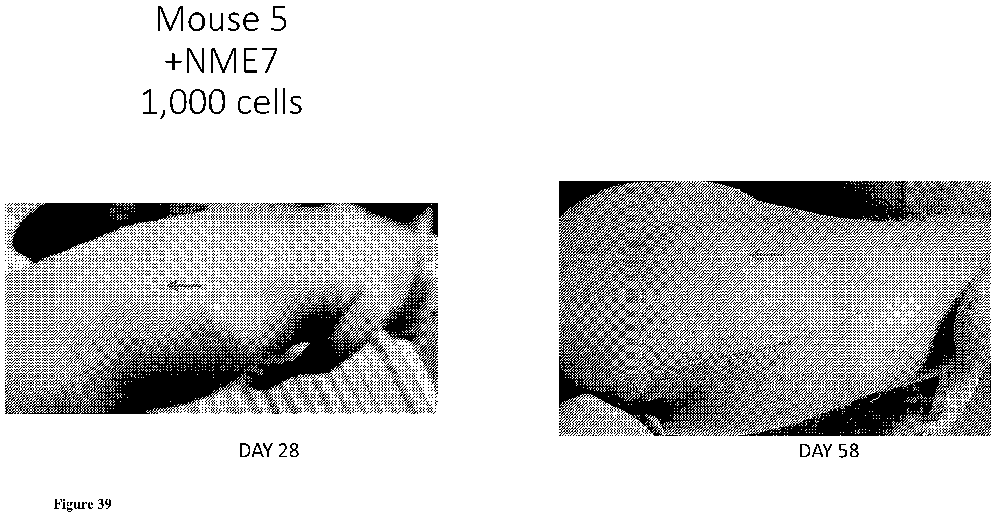

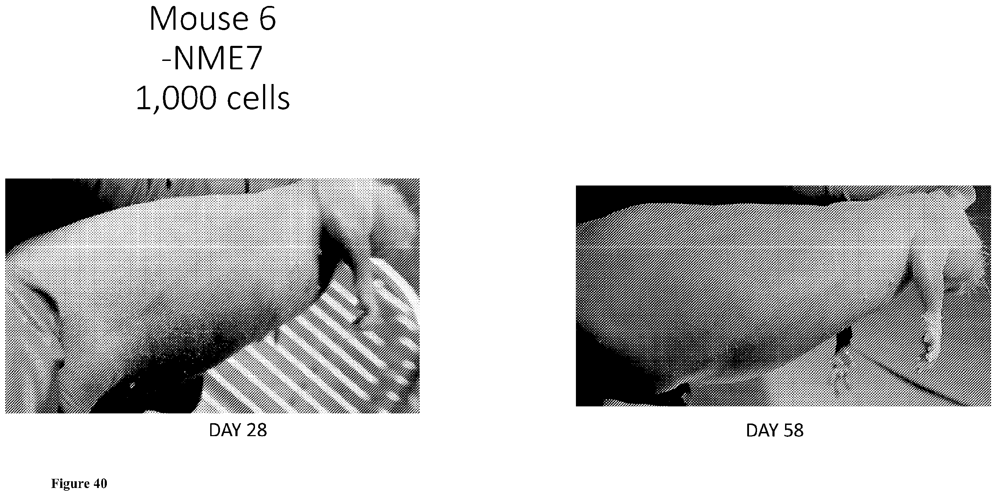

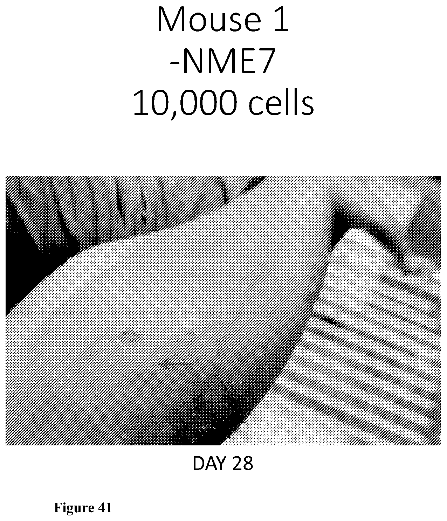

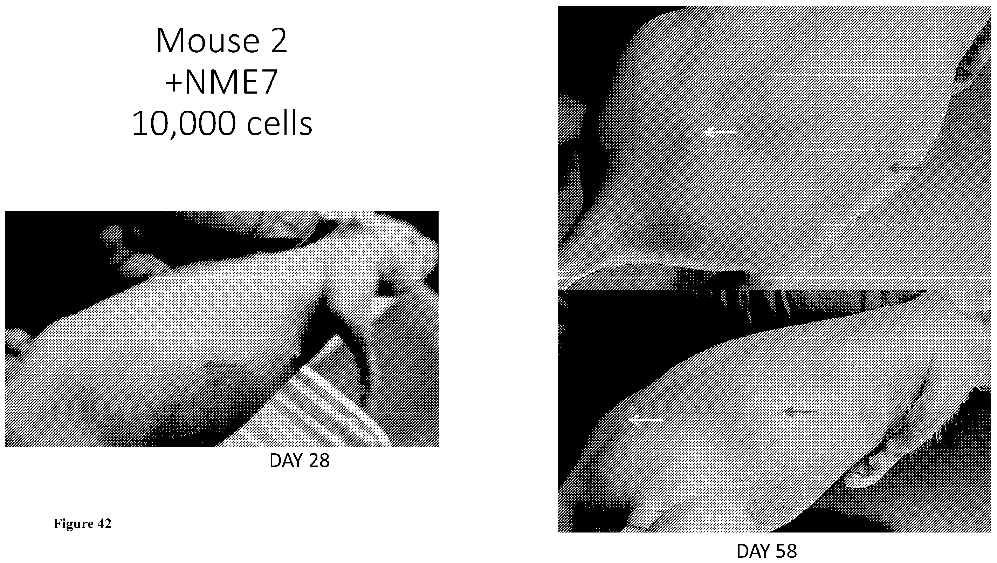

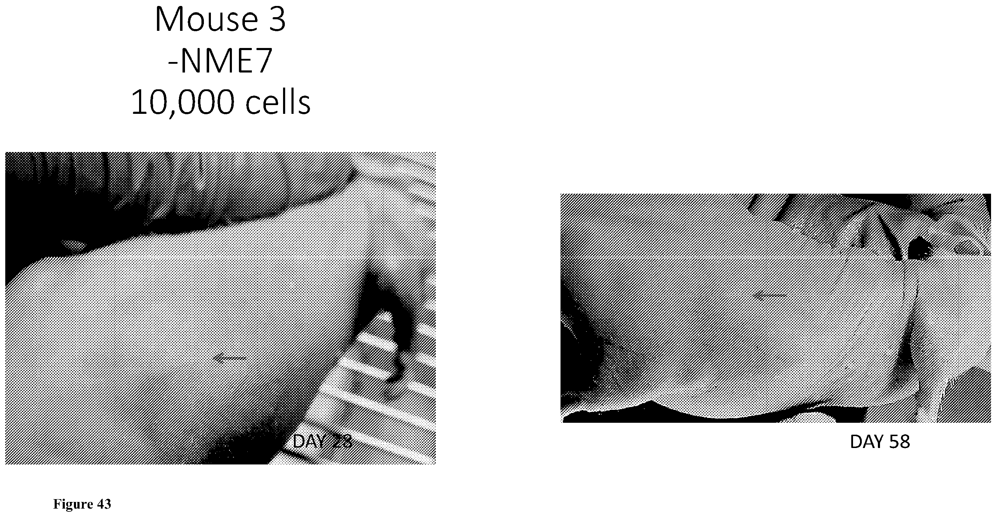

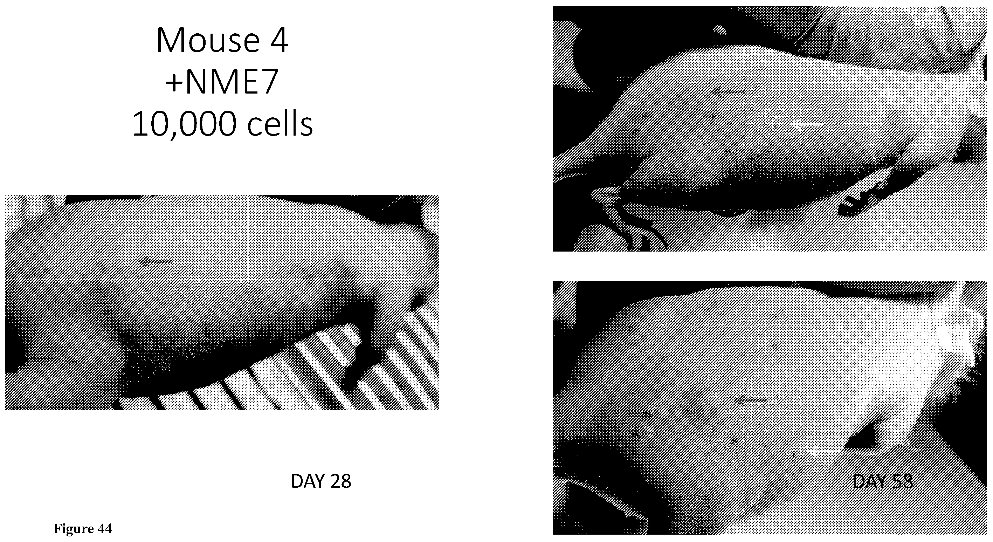

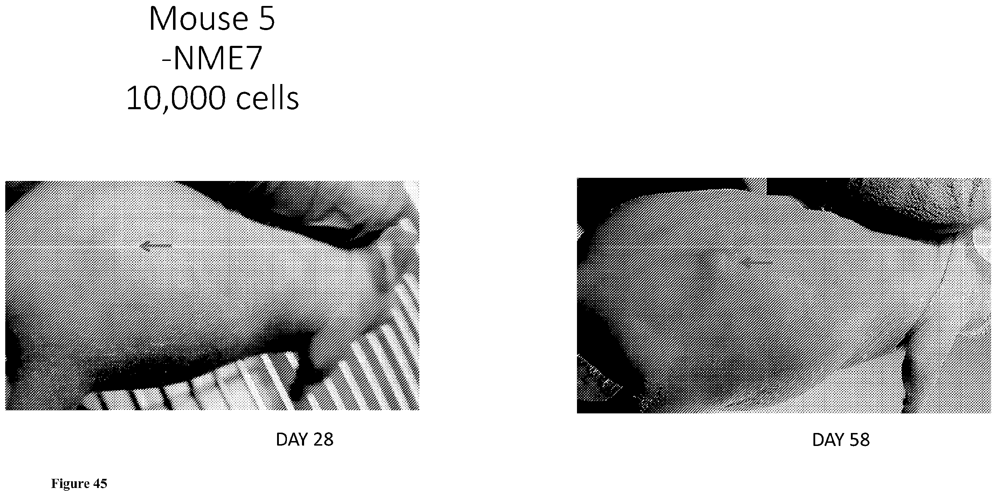

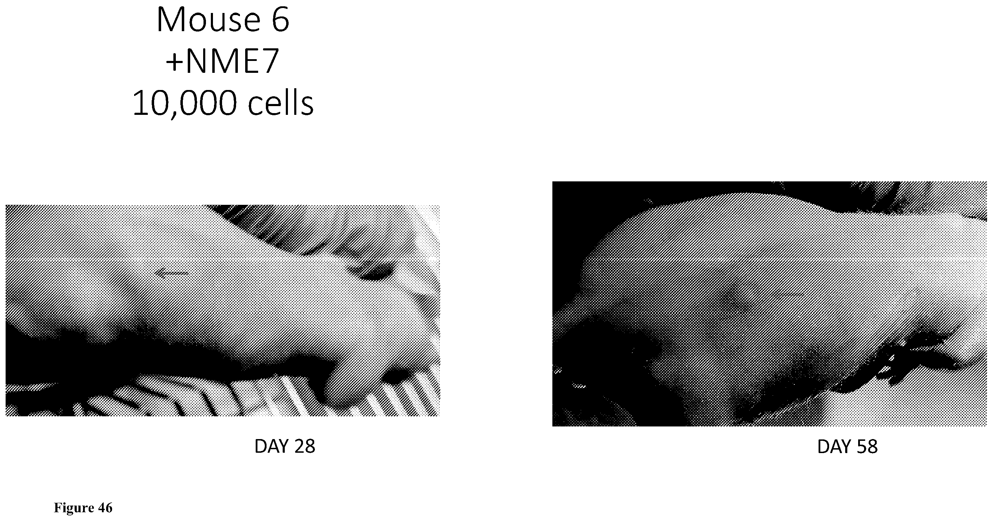

Additionally, half of the mice were injected daily with recombinant human NME7-AB. Tumor engraftment was achieved even for the group implanted with only 50 cells. Surprisingly, the groups that were injected daily with NME7 had increased rate of engraftment and some of the animals in that group also formed multiple tumors. That is to say, the cancers of the group injected with NME7 daily metastasized after about 50 days and gave rise to multiple tumors at sites remote to the initial implantation site. In this particular experiment, 67% of the 24 mice implanted with the NME7-induced cancer stem cells developed tumors. However, a closer look at the data shows that only 50% of the mice that did not having circulating NME7 formed tumors, while 83% of the mice receiving daily injections of NME7 formed tumors. Of that 83% that formed tumors, 80% developed cancer metastasis as they had multiple tumors by approximately Day 50 of the experiment. **Figure 21** is a graph of tumor volume measured 63 days after implantation of cancer cells that had been cultured in a media containing a recombinant form of NME7, NME7-AB. The number alongside each data point refers to the mouse tracking number and "M" denotes that the animal had multiple tumors. **Figure 22** is a graph of total tumor volume wherein the volumes of all the tumors in one mouse with metastatic cancer have been added together. **Figures 23-****46** show photographs of each mouse in the study at Day 28 and Day 58 to show the progression of tumor growth and in most cases where mice were injected daily with NME7-AB, to show the progression of metastasis. In **Figures 23-46** the dark arrows point to the site of injection of the initial cancer cells and the light arrows point to the distant metastases that developed between Day 28 and Day 63.

In general, the animal need not be injected *per se* with the agent that makes stem cells revert to the naive state such as NME1 dimers, NME7-AB, 2i, 5i and the like. In an alternative approach, the agent can be administered to the animal by injection, adsorption, or ingestion.

Therefore, a method for transforming cancer cells to cancer stem cells is to culture cancer cells in NME1 dimers, NME7, bacterial NME1 dimers or inhibitors that make primed stem cells revert to the naive state. In a preferred embodiment, the agents that make primed stem cells revert to the naive state are the 2i inhibitors or the 5i inhibitors. In a preferred embodiment, the NME7 is human NME7. In a more preferred embodiment the NME7 is NME7-AB. Drugs and drug candidates for the treatment of cancers or to inhibit the progression of cancers, especially metastatic cancers are then tested on these cancer stem cells. Additionally, cancer stem cells generated by these methods can be analyzed in order to identify still unknown markers of metastatic cancer cells, which would then be new targets for anti-cancer drugs.

Therefore, a method for evaluating compounds, biologicals or drugs for the treatment of cancers or for the prevention of metastasis or for the inhibition of metastasis comprises: 1) transforming cancer cells into more metastatic cancer cells by culturing the cells in a media that contains NME1 dimers that may be human or bacterial, NME7 or a fragment thereof or NME7-AB, 2i inhibitors or 5i inhibitors; 2) contacting the cells with a test agent *in vitro* or implanting the cells in an animal then treating the animal with the test agent; 3) evaluating the effect of the test agent on tumor growth or metastasis; 4) concluding that a test agent that inhibits tumor growth or metastasis to remote sites is an agent suitable for treatment of patients diagnosed with cancer, patients with cancer recurrence or metastatic cancers or patients at risk for developing cancers. In a preferred embodiment, the test animal is injected with an NME protein, 2i or 5i, or is a transgenic animal that expresses human NME or human NME7 or NME7-AB. Alternatively, the animal is a transgenic animal in which the kinases inhibited by 2i or 5i are suppressed or agents to suppress the kinases are administered to the test animal.

### Metastasis in mouse

As discussed above, low numbers of cancer cells were able to initiate tumors in animals or cancer cells metastasized in animals that were injected with NME7-AB. However, the same effect is readily accomplished by generating transgenic animals that express human NME7 or NME7-AB. In one aspect of the invention, the animal is a rodent.

A transgenic mouse expressing human NME7, human NME1 or mutants that prefer dimerization or bacterial NME, human MUC1 or MUC1* would be of great use in drug discovery, for growing cancer cells *in vivo* and for testing the effects of immunizing NME-derived peptides as elements of an anti-cancer vaccine and for generating animals that could support the growth of human stem cells in their developing organs to yield an animal, such as a mouse, with some human heart tissue, and the like. Murine NME proteins differ significantly from human NME proteins, which are required for human cancer growth and for human stem cell growth. Therefore a normal mouse does not produce the requisite proteins to support the growth of human cancer cells or human stem cells. A mouse or other mammal that would spontaneously form tumors, or respond more like a human to drugs being tested or that would better allow human tumor engraftment, would be advantageous. Transgenic animals that better support the growth of human cancer cells or human stem cells are therefore generated by making the animal express human NME genes. In a preferred embodiment the human NME gene is human NME7. In a more preferred embodiment, the human NME gene is human NME7-AB. Transgenic animals are generated using a number of methods. In general, a foreign gene is transferred into the germ cells of a host animal. The transgene can be integrated into the host animal's genome. Some of the methods for transgene integration enable site specific integration. One of the advantages of some methods of site-specific integration is that they allow the expression of the transgene to be controlled by the expression of a naturally occurring gene in the host animal. Methods for generating transgenic animals are known to those skilled in the art. Such methods include knock-in, knock-out, CRISPR, TALENS and the like. The invention envisions using any method for making the mammal express human NME1, bacterial NME1, NME7 or NME7-AB.

In a preferred embodiment, a transgenic mouse that expresses human NME7 or NME7-AB is generated. Because NME1, human or bacterial, and NME7 inhibit differentiation of stem cells, it may be advantageous to use technology in which the timing of expression of the NME protein, preferably NME7 or NME7-antibody, in the transgenic animal can be controlled. It would be advantageous to have the human NME7 on an inducible promoter, for example to avoid potential problems of NME7 expression during development of the animal. Methods for making the expression of foreign genes inducible in the host animal are known to those skilled in the art. Expression of NME7 or NME7-AB can be inducible using any one of many methods for controlling expression of transgenes that are known in the art.

Alternatively, the expression or timing of expression, of NME7 may be controlled by the expression of another gene which may be naturally expressed by the mammal. For example, it may be desirable for the NME7 or NME7 variant to be expressed in a certain tissue, such as the heart. The gene for NME7 is then operably linked to the expression of a protein expressed in the heart such as MHC. In this instance, the expression of NME7 is turned off when and where the MHC gene product is expressed. Similarly, one may want to have the expression of human NME1, NME6 or NME7 turn on or off in the prostate such that the location and timing of its expression is controlled by the expression of for example, a prostate specific protein. Similarly, the expression of human NME6 or NME7 in a non-human mammal can be controlled by genes expressed in mammary tissues. For example, in a transgenic mouse, human NME6 or human NME7 is expressed from the prolactin promoter, or a similar gene. In this way, it would be possible to induce or repress expression of the human NME protein in a site specific manner.

Animals xenografted with human tumors and also injected with human NME7 developed metastatic cancers. Therefore, an animal model for the development of cancer metastasis is generated by making a transgenic animal that expresses human NME7 or more preferably NME7-AB. Optimally the NME7 is on an inducible promoter to allow the animal to correctly develop. Alternatively, a metastatic animal model, preferably rodent, is made by making a transgenic animal that expresses human NME or human NME7 or NME7-AB. Alternatively, the animal is a transgenic animal in which the kinases inhibited by 2i or 5i are suppressed via inducible promoters or agents to suppress the kinases are administered to the test animal. Metastatic animal models are then used to study the basic science of the development or progression of cancers as well as to test the effects of compounds, biologicals, drugs and the like on the development of cancers.

Thus a method for the identification and selection of agents to treat cancer and metastatic cancer in particular is comprised of the steps of: 1) generate a transgenic animal that expresses a form of NME or NME7; 2) implant the animal with human cancer cells; 3) treat the animal with a candidate drug for the treatment of cancer or metastatic cancer; 4) evaluate the effect of the candidate drug on the size or spread of the cancer; and 5) conclude that candidate drugs that inhibited the growth or spread of the cancer in the test animal are suitable for the treatment of cancers or metastatic cancers in humans.

### Personalized drug discovery by extending the period of time that patient cancer cells proliferate to test for response to candidate drugs

Nearly all pre-clinical drug testing and drug selection is done using a very few established cancer cell lines. Those cell lines were derived from the tumor of a patient that lived and died decades ago and have been propagated through millions of generations, so that the resultant test cells bear little or no resemblance to the donor's original cancer and even less resemblance to a new patient's cancer. It would therefore be of great benefit to be able to screen drugs and even to establish dosing using the cells of the patient to be treated as the test cells. Unfortunately, it is difficult if not impossible to grow cancer cells from a patient in a dish and even more difficult to propagate patient cells in an animal. This is because human cells, unlike mouse cells, can only divide in culture a very limited number of times before they senesce. The cancer cell lines that researchers use today are either naturally immortalized cancer cells isolated from pleural effusions of a single metastatic cancer patient or induced to become immortalized by fusing to an immortalized cell line or by transfecting the cancer cells with an immortalizing gene. The latter methods significantly alter the molecular characteristics of the original or primary cancer cell.

A method that is useful for propagating cancer cells from a particular patient is to culture the cells using methods that transform cancer cells into cancer stem cells. Another method is to culture the patient cells using reagents and methods that are used to revert primed stem cells to the naive state. The resultant cancer stem cells are heartier, live longer and propagate for longer periods of time. As described in the section above, patient cancer cells can be propagated by culturing them in NME1 dimers that may be human or bacterial, NME7 or a fragment thereof or NME7-AB, 2i inhibitors or 5i inhibitors. The resultant cancer cells can be divided into two fractions: those that adhere to the surface and those that float off the surface. The adherent cells will closely resemble the original tumor cells while those that float will be cancer stem cells and will look like the metastatic cancer cells that the patient may develop in the future or in response to treatment with chemotherapy drugs. These patient cells are then used to identify and select drugs and candidate drugs that will effectively inhibit that particular patient's cancer as well as identify drugs that will inhibit the progression of their cancer to a metastatic state caused by the survival of cancer stem cells. In another aspect of the invention, a patient's cancer cells propagated in this way are used to determine optimal combinations of drugs that would effectively inhibit that particular patient's cancer or used to establish dosing of a drug or drugs.

### Personalized drug discovery by transplanting patient cancer cells into multiple animals for drug testing due to small number of required cells for engraftment

Recall that cancer cells that have been cultured in NME1 dimers that may be human or bacterial, NME7 or a fragment thereof or NME7-AB, 2i inhibitors or 5i inhibitors are able to initiate tumors in test animals with very low numbers of cells implanted. Patient cancer cells cultured using these methods are then able to form tumors in multiple test animals because as few as 50 or 100 cells are required per mouse rather than millions. The reduced numbers of cells required to initiate a tumor then makes it feasible to implant a patient's cancer cells into test animals, some of which can be injected with NME1 dimers that may be human or bacterial, NME7 or a fragment thereof or NME7-AB, 2i inhibitors or 5i inhibitors or may be transgenic animals as described above, for example such that the animal expresses human NME7 or NME7-AB.

For example, the host animal is injected with candidate drugs or compounds and efficacy is assessed in order to predict the patient's response to treatment with the candidate drug or compound. In another instance, the first line treatments or drugs that are being administered to the patient or are being considered for treatment of the patient, are administered to the animal bearing the patient's cancer cells which are being reverted to a less mature state. The first line treatments likely influence which mutations the cancer cells adopt in order to escape the first line treatments. The resultant cancer cells can then be removed from the host animal and analyzed or characterized to identify mutations that are likely to occur in response to certain treatments. Alternatively, the cancer cells can remain in the host animal and the host animal is then treated with other therapeutic agents to determine which agents inhibit or kill the resistant cells or cancer stem cells.

Thus, disclosed therein, but not part of the claimed invention, is a method for identifying suitable treatments to inhibit the growth or spread of a patient's cancer comprises the steps of: 1) obtaining cancer cells from a patient; 2) culturing the patient's cancer cells in the presence of NME1 dimers that may be human or bacterial, NME7 or a fragment thereof or NME7-AB, 2i inhibitors or 5i inhibitors; 3) contacting said cells with a test agent for the inhibition of cancer or metastasis; 4) evaluating the efficacy, toxicity or dosing of a compound, biological or drug on the patient's cancer cells or evaluating the effect of the test agent on the levels of cancer stem cell markers expressed by the cells; and 5) determining that test agents that reduce viability of the cancer cells or reduce expression of genes known as cancer stem cell markers are suitable treatments for the patient.

Another method disclosed herein but not part of the claimed invention is a method for identifying suitable treatments to inhibit the growth or spread of a patient's cancer comprises the steps of: 1) obtaining cancer cells from a patient; 2) culturing the patient's cancer cells in the presence of NME1 dimers that may be human or bacterial, NME7 or a fragment thereof or NME7-AB, 2i inhibitors or 5i inhibitors; 3) implanting the cells in a test animal that may also be injected with NME1 dimers that may be human or bacterial, NME7 or a fragment thereof or NME7-AB, 2i inhibitors or 5i inhibitors or is a transgenic animal that expresses NME proteins, NME7, NME7-AB or suppresses expression of the targets of 2i or 5i; 4) administering to the test animal a test agent for the inhibition of cancer or metastasis; 5) evaluating the efficacy, toxicity or dosing of a compound, biological or drug on the ability of the patient's cells to engraft in the animal, tumor size, or development of metastasis; and 6) determining that test agents that reduce engraftment rate, tumor growth rate or development of metastasis are suitable treatments for the patient.

Alternatively, the methods described above that enable the proliferation or implantation of patient cancer cells may be used for the benefit of patients other than the donor. Patient cancer cells proliferated or implanted according to the methods of the invention will more accurately resemble a naturally occurring cancer, so could replace standard cancer cell lines in use today which have been artificially immortalized by transfecting with immortalizing genes. In particular, cancer cells cultured in NME1 dimers, NME7, NME7-AB, 2i inhibitors or 5i inhibitors that express high levels of stem cell markers, cancer stem cell markers or CXCR4 can be used for the discovery, testing and selection of drugs to treat metastatic cancers *in vitro, ex vivo* or *in vivo.*

### Generation of animals that express human tissue

Other applications are envisioned wherein a non-human transgenic for human NME1, bacterial NME1 or human NME7, preferable NME7-AB, is implanted or engrafted with human cells which may be stem cells or progenitor cells. For example in some cases it is desirable to generate an animal, such as a mouse, that will grow human tissue in its heart, liver, skin or other organ. One method for doing so is to generate a kind of chimeric animal by implanting human stem cells into an animal that has been made to express human NME7 or human NME7-AB. The human stem cells or progenitor cells can be implanted at various stages of the animal's development, including *in vitro* and *in vivo,* at the blastocyst, embryo or fetus stage of development. Because NME7 inhibits differentiation, the NME7 or NME7-AB transgene would be linked to a method by which the timing of its expression is controllable. Methods are known to those skilled in the art which could be used such that expression of the human NME7 or NME7-AB is turned off or decreased at times or locations where it is desirable to have differentiation or maturation occur. One method for making the transgene, preferably NME7, inducible or repressible is to link its expression or repression to the expression of a gene that is only expressed later in development. In such cases, one would make a transgenic animal in which expression of NME7 or NME7-AB is linked to the expression of a later gene expressed in heart or in heart progenitor cells. Thus, the expression or timing of expression, of NME7 is controlled by the expression of another gene which may be naturally expressed by the mammal. For example, it may be desirable for the NME7 or NME7 variant to be expressed in a certain tissue, such as the heart. The gene for the NME7 is then operably linked to the expression of a protein expressed in the heart such as MHC. In this instance, the expression of NME7 is decreased or turned off when and where the MHC gene product is expressed. Similarly, one may want to have the expression of human NME1, NME6 or NME7 turn on in the prostate such that the location and timing of its expression is controlled by the expression of for example, a prostate specific protein. Similarly, the expression of human NME1 or NME7 in a non-human mammal can be controlled by genes expressed in mammary tissues. For example, in a transgenic mouse, human NME1 or human NME7 can be expressed or repressed by the prolactin promoter, or a similar gene.

In this way, an animal transgenic for human NME7 or NME7-AB can be allowed to grow to a point, then implanted with human stem or progenitor cells, where they proliferate because of contact with human NME protein. The expression of the human NME is then turned off such that a specific organ or part of an organ in the animal would develop as a human tissue.

Many applications of non-human animals transgenic for human NME1, bacterial NME1 or human NME7, or NME7-7AB can be contemplated. For example, human stem or progenitor cells are implanted in the NME transgenic animal or germ cells of what will be a transgenic animal. Expression of the NME may be inducible or repressible. Depending on the site and timing of the implantation of the stem or progenitor cells, the resulted animal can be made to express human heart, liver, neuronal cells or skin.

Thus human tissues can be generated in a transgenic non-human mammal, wherein the mammal expresses human MUC1 or MUC1* or NME protein in the germ cells or somatic cells, wherein the germ cells or somatic cells contain a recombinant human MUC1 or MUC1* or NME gene sequence introduced into said mammal, wherein the expression of the gene sequence can be induced or repressed either by introduction of an external composition or by linking its expression or repression to the expression or repression of a naturally occurring gene of the host animal. Stem cells or progenitor cells that are xenogeneic in origin to the non-human mammal are transferred to the transgenic animal such that the gene is induced to be expressed so as to multiply the stem or progenitor cells and then repressing the gene expression so as to generate tissue from the xenografted stem cells. One method by which repression of the transgene is carried out is by contacting the stem cell or progenitor cells with a tissue differentiation factor. Transgene repression is also carried out naturally in the mammal in response to naturally produced host tissue differentiation factors.

These animals can be used for drug discovery. They can also be used for toxicity testing, to use an animal to determine the effects of a compound, biological or drug on human tissue or on the development of human tissue. Alternatively, the transgenic animal implanted with human stem or progenitor cells is used to grow human tissue for transplant into a human patient. In some cases, the stem or progenitor cells that are implanted are from a patient who will be the recipient of the human tissue harvested from the transgenic animal.

In one aspect, the MUC1, MUC1* or NME protein expression may be induced until the amount of transferred stem or progenitor cells are sufficiently large. The MUC1, MUC1* or NME protein expression may then be shut down by injecting the host mammal with a substance that represses the expression of MUC1, MUC1* or NME protein. The population of stem or progenitor cells may be induced to differentiate by either natural methods such as by the expression in the mouse of a differentiation inducing factor for a particular tissue or organ type, or chemical or protein substances may be injected into the host at the site of stem or progenitor cell transference to cause differentiation to desired tissue type.

Induction, differentiation/transformation agents for endoderm cell tissue may include without limitation the following agents: hepatocyte growth factor, oncostatin-M, epidermal growth factor, fibroblast growth factor-4, basic-fibroblast growth factor, insulin, transferrin, selenius acid, BSA, linoleic acid, ascorbate 2-phosphate, VEGF, and dexamethasone, for the following cell types: liver, lung, pancreas, thyroid, and intestine cells.

Induction, differentiation/transformation agents for mesoderm tissue include without limitation the following agents: insulin, transferrin, selenous acid, BSA, linoleic acid, TGF-β1, TGF-β3, ascorbate 2-phosphate, dexamethasone, β-glycerophosphate, ascorbate 2-phosphate, BMP, and indomethacine, for the following cell types: cartilage, bone, adipose, muscle, and blood cells.

Induction, differentiation/transformation agents for ectoderm tissue include without limitation the following agents: dibutyryl cyclin AMP, isobutyl methylxanthine, human epidermal growth factor, basic fibroblast growth factor, fibroblast growth factor-8, brain-derived neurotrophic factor, and/or other neurotrophic growth factor, for the following cell types: neural, skin, brain, and eye cells.

### Animals transgenic for NME protein for discovery and testing of vaccines

A non-human transgenic animal expressing human NME, especially NME7-AB, would also be useful for assessing which immunizing peptides could safely be used for the generation of antibodies against NME proteins, including NME1, bacterial NME and NME7. For example, mice transgenic for human NME1, NME7, or NME7-AB could be immunized with one or more of the immunizing peptides set forth as in **Figures 61-63****,** peptide numbers 1-53. Control group mice are analyzed to ensure that anti-NME antibodies are produced. Human tumor cells would then be implanted into the transgenic mouse, wherein expression of the human NME protein in the host animal is induced, if using an inducible promoter. The efficacy and potential toxicities of the immunizing peptides is then assessed by comparing the tumor engraftment, tumor growth rate and tumor initiating potential of cells transplanted into the transgenic mouse compared to the control mouse or a mouse wherein the inducible NME promoter was not turned on. Toxicities are assessed by examining organs such as heart, liver and the like, in addition to determining overall bone marrow numbers, number and type of circulating blood cells and response time to regeneration of bone marrow cells in response to treatment with agents cytotoxic to bone marrow cells. Immunizing peptides derived from those listed in **Figures 61-63****,** peptide numbers 1-53 that significantly reduced tumor engraftment, tumor growth rate, or tumor initiating potential with tolerable side effects are selected as immunizing peptides for the generation of antibodies outside of the patient or in a human as an anti-cancer treatment, preventative or vaccine.

### Regulators of NME protein or downstream effectors of NME protein can substitute for the NME protein

These studies have shown that one way in which NME proteins function to promote stem-like or cancer-like growth is by binding to a clipped form of the MUC1 transmembrane protein, herein referred to as MUC1*, which consists primarily of the PSMGFR sequence. Dimerization of the MUC1* extracellular domain stimulates growth and de-differentiation of somatic cells, stem cells and cancer cells, making them more metastatic.

Another way that NME proteins exert their effects is by being transported to the nucleus where they function directly or indirectly to stimulate or suppress other genes. It has been previously reported (Boyer et al, 2005) that OCT4 and SOX2 bind to the promoter sites of MUC1 and its cleavage enzyme MMP16. The same study reported that SOX2 and NANOG bind to the promoter site of NME7. We conclude, on the basis of our experiments that these 'Yamanaka' pluripotency factors (Takahashi and Yamanaka, 2006) up-regulate MUC1, its cleavage enzyme MMP16 and its activating ligand NME7. It has also been previously reported that BRD4 suppresses NME7, while its co-factor JMJD6 up-regulates NME1 (Thompson et al), which we have demonstrated is a self-regulating stem cell growth factor that is expressed later than NME7 in embryogenesis. Still others recently reported that siRNA suppression of Mbd3 or Chd4 greatly reduced resistance to iPS generation (Rais Y et al 2013 et al.) and was able to maintain stem cells in the naive state. Evidence presented here shows that there is a reciprocal feedback loop wherein NME7 suppresses BRD4 and JMJD6, while also suppressing inhibitors of pluripotency Mbd3 and CHD4. We note that in naive human stem cells, these four factors BRD4, JMJD6, Mbd3 and CHD4 are suppressed compared to their expression in later stage 'primed' stem cells. We also note that the 2i inhibitors (inhibitors of Gsk3β and MEK) that revert mouse primed stem cells to the naive state, also down regulated the same four factors BRD4, JMJD6, Mbd3 and CHD4.

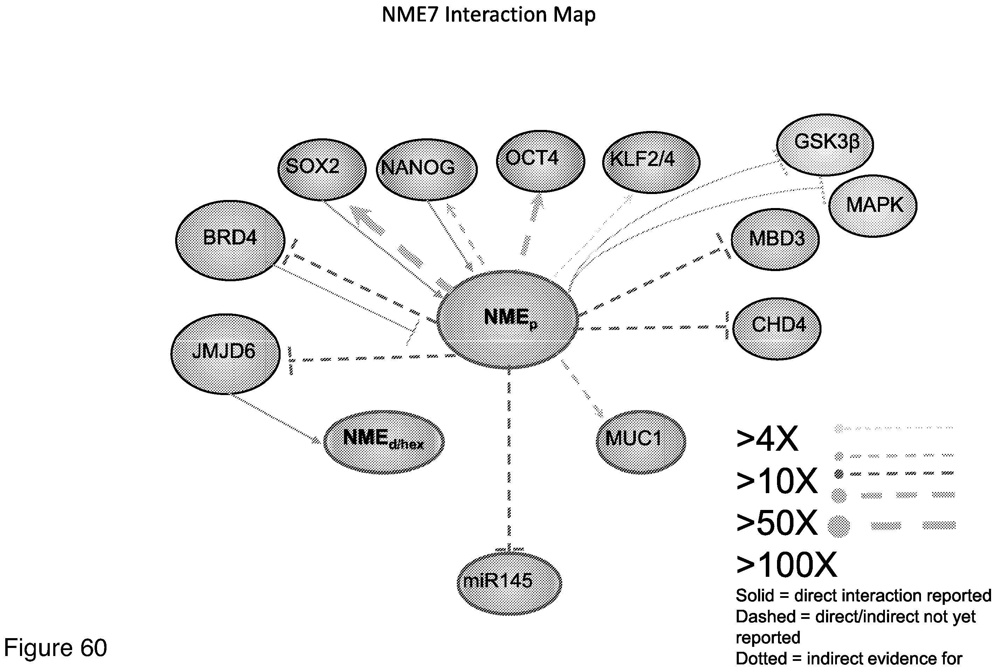

We have also discovered that NME7 up-regulates SOX2 (>150X), NANOG (~10X), OCT4 (~50X), KLF4 (4X) and MUC1 (10X). Importantly, we have shown that NME7 up-regulates cancer stem cell markers including CXCR4 (~200X) and E-cadherin (CDH1). Taken together these multiple lines of evidence point to the conclusion that NME7 is the most primitive stem cell growth and pluripotency mediator and that it is a powerful factor in the transformation of somatic cells to a cancerous state as well as transforming cancer cells to the more metastatic cancer stem cells. **Figure 60** is a cartoon of the interaction map of NME7 and the associated regulators of the stem/cancer state as evidenced by the experiments described herein. NME1 in dimer form functioned approximately the same as NME7 in being able to convert somatic cells to a stem/cancer-like state and being able to transform cancer cells to metastatic cancer stem cells, albeit to a slightly lesser degree. Similarly, bacterial NME dimers with high homology to human NME1 or NME7 such as Halomonas Sp 593 was, like NME1 dimers and NME7 monomers, able to fully support human stem cell growth, pluripotency and survival, cancer cell growth and survival, reverted somatic cells to a cancer/stem cell state and transformed cancer cells to the more metastatic cancer stem cells.

Therefore, disclosed herein and not part of the claimed invention, it is contemplated substituting genes and gene products that increase expression of NME7 for NME7. Similarly, the invention contemplates substituting downstream effectors of NME7 for NME7. For example, alone or in combination, agents that suppress MBD3, CHD4, BRD4 or JMJD6 can be substituted in any of the methods described herein, for NME7, which we have shown suppresses MBD3, CHD4, BRD4 or JMJD6.

### EXAMPLES

**Example 1.** Immune-compromised nu/nu mice between 40-60 days old were implanted with 90-day estrogen release pellets to foster the engraftment and growth of breast tumor cells. Human T47D breast cancer cells were mixed 1:2 with Matrigel then injected into the flank of the mouse (4 million each mouse), six (6) mice per group. In a second group, cancer cells were mixed with Matrigel at the same 1:2 ratio (100uL cells: 200ul Matrigel) except, human recombinant NME7-AB was added into the cell/Matrigel mix to a final NME7 concentration of 16nM (6 mice). Of those six (6) mice, three (3) were randomly chosen to receive NME7 injections daily (100 uL at 32nM), after day 14. **Figure 1** shows graphs of tumor cell growth in mice. Panel **(A)** shows a graph of the growth of T47D breast tumor cells mixed with either the standard Matrigel or Matrigel plus NME7 and xenografted into immune compromised (nu/nu) mice. After Day 14, the mice whose tumor cells were mixed with NME7 were also injected once daily with human recombinant NME7. Panel (B) shows a graph of the growth of T47D breast tumor cells mixed with Matrigel plus NME7 and xenografted into immune compromised mice. After Day 14, half of the mice were also injected once daily with human recombinant NME7. **Figure 2** shows graphs of the growth of human tumor cells in individual mice. Panel (A) shows a graph of the growth of T47D breast cancer cells that were mixed with the standard Matrigel. Only two (2) of the six (6) implanted mice showed tumor growth characteristic of engraftment. Panel (B) shows a graph of the growth of T47D breast cancer cells that were mixed with Matrigel and NME7. Four (4) of the six (6) implanted mice showed tumor growth characteristic of engraftment. Dashed lines indicate mice that were also injected with NME7 after Day 14.

**Example 2.** Several groups of immune-compromised mice between 40-60 days old were implanted with 90-day estrogen release pellets to foster the engraftment and growth of breast tumor cells and implanted into the flank of each mouse. Human T47D breast cancer cells were mixed 1:2 with Matrigel (100uL cells: 200ul Matrigel) then injected into one flank of each mouse (4 million each mouse). Animals were untreated and tumor growth was measured to track the rate of tumor growth and assess the percentage tumor engraftment of this cell line. **Figure 3** shows a graph of T47D tumor cells mixed with the standard Matrigel and xenografted into forty (40) immune compromised (nu/nu) mice. The graph shows the average of two identical groups of twenty mice each, with an average increase of 22% in tumor volume but a downward trend. **Figure 4** shows a graph of the growth of the T47D human breast tumor cells in the forty (40) individual mice, with about 25% showing tumor engraftment. **Figure 5** shows graphs of the growth of T47D breast cancer cells mixed with Matrigel and xenografted into the flanks of six (6) NOD/SCID mice. Panel (A) shows average tumor growth. Panel (B) shows tumor growth in individual mice, revealing that only one (1) of six (6) mice had good tumor engraftment using the standard method.

**Example 3.** Agents that are able to maintain stem cells in the naive state transform cancer cells into cancer stem cells wherein only a small number of cells are required for engraftment. Cancer cells were cultured according to standard practice. In the controls, the cancer cells were cultured in their normal media: RPMI for T47D breast cancer cells, DU145 and PC3 prostate cancer cells. However, the test agents, recombinant proteins human NME1 dimers, bacterial HSP593 NME1 dimers, human NME7-AB and 2i, were added to a serum-free minimal media to eliminate potential interference from the many growth factors and cytokines in serum. As a further control, cancer cells were cultured in minimal media that did not contain NME1, NME7 or 2i, but did not proliferate nor did the resultant cells up-regulate markers of cancer stem cells,

Serum-free Minimal Media 500 mL includes the following components:
394mL DMEM/F12, GlutaMAX; 100 mL Knockout^{™} Serum Replacement;
5.0 mL 100x MEM Non-essential Amino Acid Solution;
0.9 mL β-mercaptoethanol, 55 mM stock.

To this serum-free minimal media, rhNME1 (aka NM23) was added to a final concentration of 8nM, or bacterial HSP593 recombinant NME1 was added to a final concentration of 8nM, or rhNME7-AB was added to a final concentration of 4nM, or 2i inhibitors, PD0325901 and CHIR99021, which inhibit the MAP kinase pathway and GSK3, respectively, were added to a final concentration of 3µM and 1µM.

When a rho kinase inhibitor, "Ri" or "ROCi", was added it was Y27632 from Stemgent (Cambridge, MA), added immediately before use to a final concentration of 10uM.

**Example 3a.** Breast cancer T47D cells were cultured either in the traditional RPMI growth media or in a minimal stem cell media to which was added either human NME1 dimers, also known as NM23-H1, bacterial HSP593 NME1 dimers, human NME7-AB, or kinase inhibitors known as '2i'. What these agents have in common is that they are each able to promote the growth of stem cells in the naive state or are able to revert primed state stem cells to the less mature naive state. We observed that some of the T47D cancer cells cultured in any of these agents became non-adherent. These 'floaters' were also morphologically different. The addition of a rho kinase inhibitor, called 'Ri' or 'ROCi' here caused most of the cells to remain attached to the surface. RT-PCR measurements shown in the graphs of **Figures 6-10** show that the floating cells are the ones that have the highest expression of the cancer stem cell markers or stem cell markers. When a rho kinase inhibitor is added, all the cells remain attached but the RT-PCR measurements indicate that the resultant measure is of a mixture of the cells that were transformed and those that were not or were not yet. The results are shown in **Figures 6-10****.**

**Figure 6** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added recombinant human NME7-AB as the single growth factor. A portion of those cells became non-adherent and floated off the surface and were collected while '+ Ri' refers to Rho kinase inhibitor that was added to the media so that all the cells would remain attached to the surface, thus giving an average reading of the adherent and the non-adherent cells. As is shown in the graph, the expression of the metastatic receptor CXCR4 is nearly 200-times the expression level of the T47D cells cultured in normal growth media. CDH1, also called E-cadherin, and MUC1, which have both been implicated in the progression of cancers were both elevated by about 10-fold. Stem cell markers that have also been reported to be markers of cancer cells were also elevated. OCT4 and SOX2 were increased by about 50-times and 200-times respectively. Other stem cell markers such as NANOG, KLF2, KLF4 and TBX3 showed modest increases in expression.

**Figure 7** shows a graph of RT-PCR measurements of the expression of a panel of genes, reportedly overexpressed in some cancer stem cells or metastatic cancer cells, in MUC1-positive T47D breast cancer cells that were cultured in either normal RPMI growth media, or a serum free minimal media to which was added a human recombinant NM23, also called NME1, dimers or NME7-AB as the single growth factor. 'Floaters' refers to those cells that became non-adherent and were collected, while '+ Ri' refers to Rho kinase inhibitor that was added to some cells to make them adhere to the surface. As can be seen in the graph, cancer cells cultured in NME1 dimers or NME7-AB had dramatic increases in the expression of CXCR4, SOX2 and OCT4, followed by increases in expression of CDH1 also called E-cadherin, MUC1, and NANOG. There were modest increases in the expression of stem cell markers KLF2 and KLF4.

In **Figure 8** we see that bacterial NME1 acts in a way similar to that of human NME1. Recombinant HSP593 bacterial NME1 added to T47D, MUC1-positive breast cancer cells also caused a morphological change in the cells and also caused the cells to become non-adherent. **Figure 8** shows a graph of RT-PCR measurements showing a dramatic increase in the expression of CXCR4, NANOG, and OCT4, followed by lesser increase in SOX2 and a 5-fold increase in CDH1, also called E-cadherin.

The result of culturing T47D breast cancer cells in the presence of the '2i' kinase inhibitors is shown in **Figure 9****.** 2i are biochemical inhibitors of MAP kinase and GSK3, previously shown to revert stem cells in the primed state to the earlier naive state. 2i caused T47D cells to robustly increase expression of CXCR4, SOX2, OCT4, MUC1 and CDH1/E-cadherin, in that order. If NME1 dimers or NME7-AB were added to the 2i, there was an increase in the expression of these cancer stem cell markers, with NME7-AB having the greatest effect. However, **Figure 10** shows that NME7-AB alone generates cancer cells with an even higher expression level of the cancer stem cell markers.

**Example 3b.** Prostate cancer cells were also cultured as described above with either human NME1 dimers, bacterial NME1 dimers, NME7-AB, or 2i inhibitors. Unlike the T47D cells, the prostate cancer cells did not become non-adherent. Thus, the RT-PCR measurements of the cancer stem cell markers are lower than that of the T47D cells, which could be explained by it being the measure of transformed cells and non-transformed cells. In the case of breast cancer cells, we were able to isolate the fully transformed cancer stem cells by collecting the floating cells, but were not able to do so here. DU145 MUC1-positive prostate cancer cells were cultured in RPMI media as a control and in minimal media to which was added recombinant human NME1/NM23 dimers, bacterial HSP593 dimers, or human NME7-AB. **Figure 11a** shows that culture in rhNME1 dimers or rhNME7-AB for 10 days resulted in modest increases in markers of cancer stem cells. There was about a 2-8-fold increase in OCT4, MUC1 and CDH1/E-cadherin. However, after serial passaging under these same culture conditions, the increases in expression of cancer stem cell markers became more pronounced. We reasoned that serial passaging allowed more time for cells to transform since we could not rapidly collect floating cells. **Figure 11b** shows that after 9-10 passages in either rhNME7-AB, bacterial HSP593 NME1 dimers or thNME1/NM23 dimers, there was a 9-fold, 8-fold and 6-fold increase, respectively, in the expression of CDH1/E-cadherin which is often over expressed in prostate cancers. There were also significant increases in expression of NANOG and OCT4.

PC3 MUC1-negative prostate cancer cells were also tested for their ability to transform into cancer stem cells when cultured in agents that are able to maintain stem cells in the naïve state. PC3 cells were cultured as described above with either human NME1 dimers, bacterial NME1 dimers or NME7-AB. Like DU145 prostate cancer cells, PC3 cells did not become non-adherent. Thus, the RT-PCR measurements of the cancer stem cell markers may be lower because measurements will be the average of transformed cells and non-transformed cells. **Figure 12a** shows RT-PCR measurements of a subset of genes after passage in rhNME1/NM23 dimers or in rhNME7-AB. The graph of **Figure 12a** shows modest increases in the expression of stem cell markers SOX2, OCT4 and NANOG. Serial passaging in the media, did not increase expression of cancer stem cell or stem cell markers, rather they decreased, as is shown in **Figure 12b****.**

**Example 3c.** In this example, the effect of '2i' inhibitors on cancer cells was tested. Recall that 2i is the name given to 2 small molecules, CHIR99021 and PD0325901 that inhibit the MAP kinase pathway and GSK3 respectively. In stem cell experiments, it was demonstrated that 2i is able to revert stem cells in the primed state back to the less mature naïve state. In this experiment, we cultured MUC1-positive T47D breast cancer cells in minimal media to which was added either 2i or NME7-AB. RT-PCR measurements were taken that showed that 2i as well as NME7-AB suppressed expression of chromatin re-arrangement factors MBD3 and CHD4, see **Figure 13****.** siRNA suppression of MBD3 and CHD4 were reported to be key additives of a media that also reverted stem cells to the naïve state. MBD3 and CHD4 were similarly suppressed when cultured in 2i plus either rhNME1/NM23 or rhNME7-AB, **Figure 14****.**

**Example 3d.** In this example, fibroblast cells were cultured in the presence of recombinant human NME1/NM23 dimers, bacterial HSP593 NME1 dimers or NME7-AB. The fibroblasts were cultured in their normal media as a control, which is for 500 mL, 445 mL DMEM high glucose base media, 5 mL GlutaMAX and 50 mL of fetal bovine serum (FBS). After 15-20 days in culture with either NME1/NM23 dimers, bacterial HSP593 NME1 dimers or NME7-AB, the morphology of the cells completely changed so that they no longer were recognizable as fibroblasts. RT-PCR showed that the resultant cells greatly increased expression of stem cell marker genes OCT4 and NANOG, see **Figure 15****.** Just as the cancer cells had, they also decreased expression of BRD4, JMJD6, MBD3 and CHD4.

**Figure 16** shows a graph of RT-PCR measurements of the expression of genes that code for the chromatin rearrangement factors BRD4, JMJD6, MBD3 and CHD4. **Figure 17** shows a graph of RT-PCR measurements of the expression of genes, reportedly overexpressed in some cancer stem cells or metastatic cancer cells and genes that code for chromatin rearrangement factors BRD4, JMJD6, MBD3 and CHD4. Here, 'minus ROCi' refers to cells that became non-adherent and floated off the surface.

**Example 4.** Cancer stem cells generated by culture in NME7-AB initiate tumors in mice with implantation of as few as 50 cells.

T47D breast cancer cells were cultured as described in Example 3a above. After 7-10 days culture in minimal media to which was added rhNME7-AB to a final concentration of 4nM, floating cells were collected. RT-PCR measurements showed that expression of CXCR4 was increased 130-times over the control cells that were cultured in RPMI media. The floating cells were counted and re-suspended in PBS. The NME7-induced cancer stem cells were mixed 1:2 with reduced growth factor Matrigel; that is 2 parts Matrigel to 1 part cells. A range of ratios of cancer cells to NME7 or the injection schedule of NME7 is expected to vary from one mouse strain to another and from one tumor type to another.

The cancer stem cells were implanted in the flank of female nu/nu mice which had previously been implanted with a 90-day release estrogen pellet in the shoulder area. Four (4) groups of six (6) mice each were implanted with either 50, 100, 1,000 or 10,000 T47D cancer stem cells. In each case the total volume injected into each mouse was 100uL. Half of the mice in each group were injected daily with 200uL of recombinant human NME7-AB in the flank near but not at the cancer stem cell implantation site.

Animals were monitored for food intake and body weight, which were normal and stable. Two (2) independent and blinded tumor measurements were taken once a week and recorded.

Tumor engraftment was achieved even for the group implanted with only 50 cells. Surprisingly, the groups that were injected daily with NME7 had increased rate of engraftment and some of the animals in that group also formed multiple tumors. That is to say, the cancers of the group injected with NME7 daily metastasized after about 50 days and gave rise to multiple tumors at sites remote to the initial implantation site. In this particular experiment, 67% of the 24 mice implanted with the NME7-induced cancer stem cells developed tumors. However, a closer look at the data shows that only 50% of the mice that did not having circulating NME7 formed tumors, while 83% of the mice receiving daily injections of NME7 formed tumors. Of that 83% that formed tumors, 80% developed cancer metastasis as they had multiple tumors by approximately Day 50 of the experiment. **Figure 21** is a graph of tumor volume measured 63 days after implantation of cancer cells that had been cultured in a media containing a recombinant form of NME7, NME7-AB. The number alongside each data point refers to the mouse tracking number and "M" denotes that the animal had multiple tumors. **Figure 22** is a graph of total tumor volume wherein the volumes of all the tumors in one mouse with metastatic cancer have been added together. **Figures 23-****46** show photographs of each mouse in the study at Day 28 and Day 58 to show the progression of tumor growth and in most cases where mice were injected daily with NME7-AB, to show the progression of metastasis. In **Figures 23-46** the dark arrows point to the site of injection of the initial cancer cells and the light arrows point to the distant metastases that developed between Day 28 and Day 63.

### Cited References List

Clarke MF, Dick JE, Dirks PB, Eaves CJ, Jamieson CH, Jones DL, Visvader J, Weissman IL, Wahl GM. (2006) Cancer stem cells--perspectives on current status and future directions: AACR Workshop on cancer stem cell.. Cancer Res. Oct 1;66(19):9339-44. Epub 2006 Sep 21.
Chen K, Huang YH, Chen JL. (2013) Understanding and targeting cancer stem cells: therapeutic implications and challenges. Acta Pharmacologica Sinica 34: 732-740; Review
Darash-Yahana M, Pikarsky E, Abramovitch R, Zeira E, Pal B, Karplus R, Beider K, Avniel S, Kasem S, Galun E, Peled A (2004) Role of high expression levels of CXCR4 in tumor growth, vascularization, and metastasis. FASEB J 18(11): 1240-1242
Mahanta S, Fessler S, Park J, Bamdad C. A Minimal Fragment of MUC1 Mediates Growth of Cancer Cells, 2008 PLoS ONE 3:e2054-2065.
Hikita S, Clegg O, Kosik K, Bamdad C. MUC1* Mediates the Growth of Human Pluripotent Stem Cells, 2008 PLoS ONE 3:e3312-3325.
Kumar SM, Liu S, Lu H, Zhang H, Zhang PJ, Gimotty PA, Guerra M, Guo W, Xu X. (2012) Acquired cancer stem cell phenotypes through Oct4-mediated dedifferentiation. Oncogene. Nov 22;31(47):4898-911.
Liu K, Lin B, Zhao M, Yang X, Chen M, Gao A, Liu F, Que J, Lan X. (2013) The multiple roles for Sox2 in stem cell maintenance and tumorigenesis. Cellular Signaling May;25(5):1264-71. Review
Yeo JC, Jiang J, Tan ZY, Yim GR, Ng JH, Göke J, Kraus P, Liang H, Gonzales KA, Chong HC, Tan CP, Lim YS, Tan NS, Lufkin T, Ng HH. (2014) K1f2 is an essential factor that sustains ground state pluripotency. Cell Stem Cell. Jun 5;14(6):864-72.
Oshima N, Yamada Y, Nagayama S, Kawada K, Hasegawa S, Okabe H, Sakai Y, Aoi T. (2014) Induction of cancer stem cell properties in colon cancer cells by defined factors. PLoS One. Jul 9;9(7):e101735
Wang ML, Chiou SH, Wu CW. (2013) Targeting cancer stem cells: emerging role of Nanog transcription factor. Onco targets and Therapy. Sep 4;6:1207-20. Review.
Xu C, Rosler E, Jiang J, Lebkowski JS, Gold JD, et al. (2005) Basic Fibroblast Growth Factor Supports Undifferentiated Human Embryonic Stem Cell Growth Without Conditioned Medium. STEM CELLS 23: 315-323.
Fessler S, Wotkowicz M, Mahanta S, Bamdad C (2009) MUC1* is a determinant of trastuzumab (Herceptin) resistance in breast cancer cells, Breast Cancer Res Treat 118:113-124 DOI 10.1007/s10549-009-0412-3
Lissa Nurrul Abdullah and Edward Kai-Hua Chow (2013) Mechanisms of chemoresistance in cancer stem cells. Clinical and Translational Medicine Jan 17;2(1):3
Miki J, Furusato B, Li H, Gu Y, Takahashi H, Egawa S, Sesterhenn IA, McLeod DG, Srivastava S, Rhim JS. Identification of putative stem cell markers, CD133 and CXCR4, in hTERT-immortalized primary nonmalignant and malignant tumor-derived human prostate epithelial cell lines and in prostate cancer specimens. Cancer Res. 2007 Apr 1;67(7):3153-61.
Jeter CR, Liu B, Liu X, Chen X, Liu C, Calhoun-Davis T, Repass J, Zaehres H, Shen JJ, Tang DG. NANOG promotes cancer stem cell characteristics and prostate cancer resistance to androgen deprivation. Oncogene. 2011 Sep 8;30(36):3833-45. PMCID:
Hong X, Chedid K, Kalkanis SN. Glioblastoma cell line-derived spheres in serum-containing medium versus serum-free medium: a comparison of cancer stem cell properties. Int. J. Oncol. 2012 Nov;41(5):1693-700.
Faber A, Goessler UR, Hoermann K, Schultz JD, Umbreit C, Stern-Straeter J. SDF-1-CXCR4 axis: cell trafficking in the cancer stem cell niche of head and neck squamous cell carcinoma. Oncol. Rep. 2013 Jun;29(6):2325-31.
Mukherjee D, Zhao J. The Role of chemokine receptor CXCR4 in breast cancer metastasis. Am J Cancer Res. 2013;3(1):46-57. PMCID: PMC3555200
Herreros-Villanueva M, Zhang J-S, Koenig A, Abel EV, Smyrk TC, Bamlet WR, de Narvajas AA-M, Gomez TS, Simeone DM, Bujanda L, Billadeau DD. SOX2 promotes dedifferentiation and imparts stem cell-like features to pancreatic cancer cells. Oncogenesis. 2013;2:e61. PMCID: PMC3759123
Sefah K, Bae K-M, Phillips JA, Siemann DW, Su Z, McClellan S, Vieweg J, Tan W. Cell-based selection provides novel molecular probes for cancer stem cells. Int. J. Cancer. 2013 Jun 1;132(11):2578-88.
Su H-T, Weng C-C, Hsiao P-J, Chen L-H, Kuo T-L, Chen Y-W, Kuo K-K, Cheng K-H. Stem cell marker nestin is critical for TGF-β1-mediated tumor progression in pancreatic cancer. Mol. Cancer Res. 2013 Jul;11(7):768-79.
Nichols J, Smith A (2009) Naive and primed pluripotent states. Cell Stem Cell 4: 487-492.
Hanna J, Cheng AW, Saha K, Kim J, Lengner CJ, et al. (2010) Human embryonic stem cells with biological and epigenetic characteristics similar to those of mouse ESCs. Proc Natl Acad Sci U S A 107: 9222-9227.
Smagghe, B.J. Stewart A.K., Carter M.G., Shelton L.S., Bernier K.J., Hartman E.J., Calhoun A.K., Hatziioannou V.M., Lillacci G., Kirk B.A., DiNardo B.A., Kosik K.S., Bamdad C. (2013) MUC1* Ligand, NM23-H1, Is a Novel Growth Factor That Maintains Human Stem Cells in a More Naive State. PLoS ONE 8(3): e58601
Silva J, Barrandon O, Nichols J, Kawaguchi J, Theunissen TW, Smith A (2008). Promotion of reprogramming to ground state pluripotency by signal inhibition. PLoS Biol. 21;6(10)
Theunissen TW, Powell BE, Wang H, Mitalipova M, Faddah DA, Reddy J, Fan ZP, Maetzel D, Ganz K, Shi L, Lungjangwa T, Imsoonthornruksa S, Stelzer Y, Rangarajan S, D'Alessio A, Zhang J, Gao Q, Dawlaty MM, Young RA, Gray NS, Jaenisch R. (2014) Systematic Identification of Culture Conditions for Induction and Maintenance of Naive Human Pluripotency. Cell Stem Cell. 2014 Jul 24, S1934-5909(14)00298-7.
Hugo HJ, Kokkinos MI, Blick T, et al. Defining the E-cadherin repressor interactome in epithelial-mesenchymal transition: the PMC42 model as a case study. (2011) Cells Tissues Organs;193:23-40
Epstein RJ (2004) The CXCL12-CXCR4 chemotactic pathway as a target of adjuvant breast cancer therapies. Nat Rev Cancer 4(11): 901-909
Müller, A.; Homey, B.; Soto, H.; Ge, N.; Catron, D.; Buchanan, M. E.; McClanahan, T.; Murphy, E.; Yuan, W.; Wagner, S. N.; Barrera, J. L.; Mohar, A.; Verástegui, E.; Zlotnik, A. (2001) Involvement of chemokine receptors in breast cancer metastasis. Nature, 410 (6824), 50-56.
Rais Y1, Zviran A, Geula S, Gafni O, Chomsky E, Viukov S, Mansour AA, Caspi I, Krupalnik V, Zerbib M, Maza I, Mor N, Baran D, Weinberger L, Jaitin DA, Lara-Astiaso D, Blecher-Gonen R, Shipony Z, Mukamel Z, Hagai T, Gilad S, Amann-Zalcenstein D, Tanay A, Amit I, Novershtern N, Hanna JH (2013). Deterministic direct reprogramming of somatic cells to pluripotency. , 502(7469):65-70.
Liu W, Ma Q, Wong K, Li W, Ohgi K, Zhang J, Aggarwal AK, Rosenfeld MG. Brd4 and JMJD6-Associated Anti-Pause Enhancers in Regulation of Transcriptional Pause Release. Cell. 2013 Dec 19;155(7):1581-95. PMCID: PMC3886918.
Amit M, Carpenter MK, Inokuma MS, Chiu C-P, Harris CP, et al. (2000) Clonally Derived Human Embryonic Stem Cell Lines Maintain Pluripotency and Proliferative Potential for Prolonged Periods of Culture. Developmental Biology 227: 271-278.
Ludwig TE, Levenstein ME, Jones JM, Berggren WT, Mitchen ER, et al. (2006) Derivation of human embryonic stem cells in defined conditions. Nat Biotechnol 24: 185-187.
Xu RH, Peck RM, Li DS, Feng X, Ludwig T, et al. (2005) Basic FGF and suppression of BMP signaling sustain undifferentiated proliferation of human ES cells. Nat Methods 2: 185-190.
Liu W, Ma Q, Wong K, Li W, Ohgi K, Zhang J, Aggarwal AK, Rosenfeld MG. Brd4 and JMJD6-Associated Anti-Pause Enhancers in Regulation of Transcriptional Pause Release. Cell. 2013 Dec 19;155(7):1581-95. PMCID: PMC3886918.
Rais Y, Zviran A, Geula S, Gafni O, Chomsky E, Viukov S, Mansour AA, Caspi I, Krupalnik V, Zerbib M, Maza I, Mor N, Baran D, Weinberger L, Jaitin DA, Lara-Astiaso D, Blecher-Gonen R, Shipony Z, Mukamel Z, Hagai T, Gilad S, Amann-Zalcenstein D, Tanay A, Amit I, Novershtern N, Hanna JH. Deterministic direct reprogramming of somatic cells to pluripotency. 2013 Sep 18, Nature 502, 65-70 DOI: 10.1038/nature12587
Herreros-Villanueva M, Zhang J-S, Koenig A, Abel EV, Smyrk TC, Bamlet WR, de Narvajas AA-M, Gomez TS, Simeone DM, Bujanda L, Billadeau DD. SOX2 promotes dedifferentiation and imparts stem cell-like features to pancreatic cancer cells. Oncogenesis. 2013;2:e61. PMCID: PMC3759123
Hong X, Chedid K, Kalkanis SN. Glioblastoma cell line-derived spheres in serum-containing medium versus serum-free medium: a comparison of cancer stem cell properties. Int. J. Oncol. 2012 Nov;41(5):1693-700.
Silva J, Barrandon O, Nichols J, Kawaguchi J, Theunissen TW, Smith A. Promotion of reprogramming to ground state pluripotency by signal inhibition. PLoS Biol. 2008 Oct 21;6(10):e253. PMCID: PMC2570424
Boyer et al, 2005, "Core Transcriptional Regulatory Circuitry in Human Embryonic Stem Cells", Cell, Vol. 122, 947-956
Takahashi K and Yamanaka S (2006) Induction of pluripotent stem cells from mouse embryonic and adult fibroblast cultures by defined factors. Cell 126(4):663-676 .
Porter D et al. (2011) Chimeric antigen receptor-modified T cells in chronic lymphoid leukemia. N Engl J Med 365:725-733 DOI: 10.1056/NEJMoa1103849
Tiller T et al. (2013) A fully synthetic human Fab antibody library based on fixed VH/VL framework pairings with favorable biophysical properties. MABs 9:5(3) PMID: 23571156
Webb PA, Perisic O, Mendola CE, Backer JM and Williams RL. The crystal structure of a human nucleoside diphosphate kinase, NM23-H2. J Mol Biol. 1995, 251:574-587.
Min K, Song HK, Chang C, Kim SY, Lee KJ and Suh SW. Crystal structure of human nucleoside diphosphate kinase A, a metastasis suppressor. Proteins. 2002, 46:340-342.

## Claims

1. A method for engrafting human tumors in a mouse, the method comprising:
(i) injecting or implanting human tumor cells into the mouse, and
(ii) contacting the tumor cells with an NME protein that maintains stem cells in the naive state or reverts primed stem cells to the naive state.

2. The method according to claim 1, wherein the NME protein is NME1 dimer, NME7 monomer, NME7-AB, NME6 dimer, or bacterial NME.

3. The method according to claim 1, wherein the NME protein is NME1 dimer.

4. The method according to claim 1, wherein the NME protein is NME7-AB.

5. The method according to claim 1, wherein the mouse is transgenic and expresses a human MUC1, MUC1*, or NME protein in the germ cells or somatic cells.

6. The method according to claim 5, wherein the mouse expresses a human NME protein in its germ cells or somatic cells, and wherein the germ cells and somatic cells contain a recombinant human NME gene sequence introduced into the mouse.

7. The method according to claim 6, wherein the recombinant human NME gene sequence that expresses the human NME protein is under control of an inducible promoter.

8. The method according to claim 7, wherein, the inducible promoter is responsive to a naturally occurring protein in the mouse.

9. The method according to claim 1, wherein the amount of cells implanted into the mouse is at least about 30, about 30 to about 1,000,000, about 50 to about 500,000, about 50 to 100,000, or from about 1,000 to about 1,000,000.

10. The method according to claim 1, wherein the agent that maintains stem cells in the naive state or reverts primed stem cells to the naive state is contacted with the cancer cells before carrying out step (i).

11. The method according to claim 1, wherein the agent that maintains stem cells in the naive state or reverts primed stem cells to the naive state is contacted with the cancer cells after carrying out step (i).

12. The method according to claim 1, wherein the agent that maintains stem cells in the naive state or reverts primed stem cells to the naive state is contacted with the cancer cells before and after carrying out step (i).

13. The method according to claim 10, wherein the NME protein is present in serum-free media as the single growth factor.

## Patentansprüche

1. Verfahren zum Übertragen eines menschlichen Tumors in eine Maus, wobei das Verfahren Folgendes umfasst:
(i) Injizieren oder Implantieren von menschlichen Tumorzellen in die Maus und
(ii) In-Kontakt-Bringen der Tumorzellen mit einem NME-Protein, das Stammzellen im naiven Zustand hält oder geprimte Stammzellen in den naiven Zustand zurücksetzt.

2. Verfahren nach Anspruch 1, wobei das NME-Protein ein NME1-Dimer, NME7-Monomer, NME7-AB, NME6-Dimer oder bakterielles NME ist.

3. Verfahren nach Anspruch 1, wobei das NME-Protein ein NME1-Dimer ist.

4. Verfahren nach Anspruch 1, wobei das NME-Protein NME7-AB ist.

5. Verfahren nach Anspruch 1, wobei die Maus eine transgene Maus ist und ein menschliches MUC1-, MUC1*- oder NME-Protein in den Keimzellen oder somatischen Zellen exprimiert.

6. Verfahren nach Anspruch 5, wobei die Maus ein menschliches NME-Protein in ihren Keimzellen oder somatischen Zellen exprimiert und wobei die Keimzellen und somatischen Zellen eine rekombinante menschliche NME-Gensequenz enthalten, die in die Maus eingebracht wurde.

7. Verfahren nach Anspruch 6, wobei die rekombinante menschliche NME-Gensequenz, die das menschliche NME-Protein exprimiert, durch einen induzierbaren Promotor gesteuert wird.

8. Verfahren nach Anspruch 7, wobei der induzierbare Promotor auf ein natürlich vorkommendes Protein in der Maus anspricht.

9. Verfahren nach Anspruch 1, wobei die Menge an in die Maus übertragenen Zellen zumindest etwa 30, etwa 30 bis etwa 1.000.000, etwa 50 bis etwa 500.000, etwa 50 bis 100.000 oder etwa 1.000 bis etwa 1.000.000 beträgt.

10. Verfahren nach Anspruch 1, wobei das Mittel, das die Stammzellen im naiven Zustand hält oder geprimte Stammzellen in den naiven Zustand zurücksetzt, vor Ausführung von Schritt (i) mit den Krebszellen in Kontakt gebracht wird.

11. Verfahren nach Anspruch 1, wobei das Mittel, das die Stammzellen im naiven Zustand hält oder geprimte Stammzellen in den naiven Zustand zurücksetzt, nach Ausführung von Schritt (i) mit den Krebszellen in Kontakt gebracht wird.

12. Verfahren nach Anspruch 1, wobei das Mittel, das die Stammzellen im naiven Zustand hält oder geprimte Stammzellen in den naiven Zustand zurücksetzt, vor und nach Ausführung von Schritt (i) mit den Krebszellen in Kontakt gebracht wird.

13. Verfahren nach Anspruch 10, wobei das NME-Protein in serumfreiem Medium als einziger Wachstumsfaktor vorhanden ist.

## Revendications

1. Procédé de greffe de tumeurs humaines chez une souris, le procédé comprenant les étapes consistant à :
(i) injecter ou implanter des cellules tumorales humaines dans la souris, et
(ii) mettre en contact les cellules tumorales avec une protéine NME qui maintient les cellules souches à l'état naïf ou rétablit des cellules souches amorcées à l'état naïf.

2. Procédé selon la revendication 1, dans lequel la protéine NME est un dimère de NME1, un monomère de NME7, NME7-AB, un dimère de NME6 ou une NME bactérienne.

3. Procédé selon la revendication 1, dans lequel la protéine NME est un dimère de NME1.

4. Procédé selon la revendication 1, dans lequel la protéine NME est NME7-AB.

5. Procédé selon la revendication 1, dans lequel la souris est transgénique et exprime une protéine MUC1, MUC1* ou NME humaine dans les cellules germinales ou les cellules somatiques.

6. Procédé selon la revendication 5, dans lequel la souris exprime une protéine NME humaine dans ses cellules germinales ou ses cellules somatiques, et dans lequel les cellules germinales et les cellules somatiques contiennent une séquence de gène de NME humaine recombinante introduite dans la souris.

7. Procédé selon la revendication 6, dans lequel la séquence de gène de NME humaine recombinante qui exprime la protéine NME humaine est sous la commande d'un promoteur inductible.

8. Procédé selon la revendication 7, dans lequel le promoteur inductible est sensible à une protéine d'origine naturelle chez la souris.

9. Procédé selon la revendication 1, dans lequel la quantité de cellules implantées dans la souris est d'au moins environ 30, d'environ 30 à environ 1 000 000, d'environ 50 à environ 500 000, d'environ 50 à environ 100 000, ou d'environ 1 000 à environ 1 000 000.

10. Procédé selon la revendication 1, dans lequel l'agent qui maintient des cellules souches dans l'état naïf ou rétablit les cellules souches amorcées dans l'état naïf est mis en contact avec les cellules cancéreuses avant la mise en œuvre de l'étape (i).

11. Procédé selon la revendication 1, dans lequel l'agent qui maintient des cellules souches dans l'état naïf ou rétablit des cellules souches amorcées dans l'état naïf est mis en contact avec les cellules cancéreuses après exécution de l'étape (i).

12. Procédé selon la revendication 1, dans lequel l'agent qui maintient les cellules souches dans l'état naïf ou rétablit les cellules souches amorcées dans l'état naïf est mis en contact avec les cellules cancéreuses avant et après la mise en œuvre de l'étape (i).

13. Procédé selon la revendication 10, dans lequel la protéine NME est présente dans un milieu exempt de sérum en tant que facteur de croissance unique.


### SEQUENCE TABLE

<110> MINERVA BIOTECHNOLOGIES
<120> METHOD FOR ENHANCING TUMOR GROWTH
<130> 13150-70129PCT
<160> 158
<170> PatentIn version 3.5
<210> 1
   <211> 1255
   <212> PRT
   <213> Artificial Sequence
<220>
   <223> full-length MUC1 Receptor
<400> 1
   Met Thr Pro Gly Thr Gln Ser Pro Phe Phe Leu Leu Leu Leu Leu Thr
   1               5                   10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Val Ser Met Thr Ser Ser Val Leu Ser Ser His
       50                  55                  60
   Ser Pro Gly Ser Gly Ser Ser Thr Thr Gln Gly Gln Asp Val Thr Leu
   65                  70                  75                  80
   Ala Pro Ala Thr Glu Pro Ala Ser Gly Ser Ala Ala Thr Trp Gly Gln
                   85                  90                  95
   Asp Val Thr Ser Val Pro Val Thr Arg Pro Ala Leu Gly Ser Thr Thr
               100                 105                 110
   Pro Pro Ala His Asp Val Thr Ser Ala Pro Asp Asn Lys Pro Ala Pro
           115                 120                 125
   Gly Ser Thr Ala Pro Pro Ala His Gly Val Thr Ser Ala Pro Asp Thr
       130                 135                 140
   Arg Pro Ala Pro Gly Ser Thr Ala Pro Pro Ala His Gly Val Thr Ser
   145                 150                 155                 160
   Ala Pro Asp Thr Arg Pro Ala Pro Gly Ser Thr Ala Pro Pro Ala His
                   165                 170                 175
   Gly Val Thr Ser Ala Pro Asp Thr Arg Pro Ala Pro Gly Ser Thr Ala
               180                 185                 190
   Pro Pro Ala His Gly Val Thr Ser Ala Pro Asp Thr Arg Pro Ala Pro
           195                 200                 205
   Gly Ser Thr Ala Pro Pro Ala His Gly Val Thr Ser Ala Pro Asp Thr
       210                 215                 220
   Arg Pro Ala Pro Gly Ser Thr Ala Pro Pro Ala His Gly Val Thr Ser
   225                 230                 235                 240
   Ala Pro Asp Thr Arg Pro Ala Pro Gly Ser Thr Ala Pro Pro Ala His
                   245                 250                 255
   Gly Val Thr Ser Ala Pro Asp Thr Arg Pro Ala Pro Gly Ser Thr Ala
               260                 265                 270
   Pro Pro Ala His Gly Val Thr Ser Ala Pro Asp Thr Arg Pro Ala Pro
           275                 280                 285
   Gly Ser Thr Ala Pro Pro Ala His Gly Val Thr Ser Ala Pro Asp Thr
       290                 295                 300
   Arg Pro Ala Pro Gly Ser Thr Ala Pro Pro Ala His Gly Val Thr Ser
   305                 310                 315                 320
   Ala Pro Asp Thr Arg Pro Ala Pro Gly Ser Thr Ala Pro Pro Ala His
                   325                 330                 335
   Gly Val Thr Ser Ala Pro Asp Thr Arg Pro Ala Pro Gly Ser Thr Ala
               340                 345                 350
   Pro Pro Ala His Gly Val Thr Ser Ala Pro Asp Thr Arg Pro Ala Pro
           355                 360                 365
   Gly Ser Thr Ala Pro Pro Ala His Gly Val Thr Ser Ala Pro Asp Thr
       370                 375                 380
   Arg Pro Ala Pro Gly Ser Thr Ala Pro Pro Ala His Gly Val Thr Ser
   385                 390                 395                 400
   Ala Pro Asp Thr Arg Pro Ala Pro Gly Ser Thr Ala Pro Pro Ala His
                   405                 410                 415
   Gly Val Thr Ser Ala Pro Asp Thr Arg Pro Ala Pro Gly Ser Thr Ala
               420                 425                 430
   Pro Pro Ala His Gly Val Thr Ser Ala Pro Asp Thr Arg Pro Ala Pro
           435                 440                 445
   Gly Ser Thr Ala Pro Pro Ala His Gly Val Thr Ser Ala Pro Asp Thr
       450                 455                 460
   Arg Pro Ala Pro Gly Ser Thr Ala Pro Pro Ala His Gly Val Thr Ser
   465                 470                 475                 480
   Ala Pro Asp Thr Arg Pro Ala Pro Gly Ser Thr Ala Pro Pro Ala His
                   485                 490                 495
   Gly Val Thr Ser Ala Pro Asp Thr Arg Pro Ala Pro Gly Ser Thr Ala
               500                 505                 510
   Pro Pro Ala His Gly Val Thr Ser Ala Pro Asp Thr Arg Pro Ala Pro
           515                 520                 525
   Gly Ser Thr Ala Pro Pro Ala His Gly Val Thr Ser Ala Pro Asp Thr
       530                 535                 540
   Arg Pro Ala Pro Gly Ser Thr Ala Pro Pro Ala His Gly Val Thr Ser
   545                 550                 555                 560
   Ala Pro Asp Thr Arg Pro Ala Pro Gly Ser Thr Ala Pro Pro Ala His
                   565                 570                 575
   Gly Val Thr Ser Ala Pro Asp Thr Arg Pro Ala Pro Gly Ser Thr Ala
               580                 585                 590
   Pro Pro Ala His Gly Val Thr Ser Ala Pro Asp Thr Arg Pro Ala Pro
           595                 600                 605
   Gly Ser Thr Ala Pro Pro Ala His Gly Val Thr Ser Ala Pro Asp Thr
       610                 615                 620
   Arg Pro Ala Pro Gly Ser Thr Ala Pro Pro Ala His Gly Val Thr Ser
   625                 630                 635                 640
   Ala Pro Asp Thr Arg Pro Ala Pro Gly Ser Thr Ala Pro Pro Ala His
                   645                 650                 655
   Gly Val Thr Ser Ala Pro Asp Thr Arg Pro Ala Pro Gly Ser Thr Ala
               660                 665                 670
   Pro Pro Ala His Gly Val Thr Ser Ala Pro Asp Thr Arg Pro Ala Pro
           675                 680                 685
   Gly Ser Thr Ala Pro Pro Ala His Gly Val Thr Ser Ala Pro Asp Thr
       690                 695                 700
   Arg Pro Ala Pro Gly Ser Thr Ala Pro Pro Ala His Gly Val Thr Ser
   705                 710                 715                 720
   Ala Pro Asp Thr Arg Pro Ala Pro Gly Ser Thr Ala Pro Pro Ala His
                   725                 730                 735
   Gly Val Thr Ser Ala Pro Asp Thr Arg Pro Ala Pro Gly Ser Thr Ala
               740                 745                 750
   Pro Pro Ala His Gly Val Thr Ser Ala Pro Asp Thr Arg Pro Ala Pro
           755                 760                 765
   Gly Ser Thr Ala Pro Pro Ala His Gly Val Thr Ser Ala Pro Asp Thr
       770                 775                 780
   Arg Pro Ala Pro Gly Ser Thr Ala Pro Pro Ala His Gly Val Thr Ser
   785                 790                 795                 800
   Ala Pro Asp Thr Arg Pro Ala Pro Gly Ser Thr Ala Pro Pro Ala His
                   805                 810                 815
   Gly Val Thr Ser Ala Pro Asp Thr Arg Pro Ala Pro Gly Ser Thr Ala
               820                 825                 830
   Pro Pro Ala His Gly Val Thr Ser Ala Pro Asp Thr Arg Pro Ala Pro
           835                 840                 845
   Gly Ser Thr Ala Pro Pro Ala His Gly Val Thr Ser Ala Pro Asp Thr
       850                 855                 860
   Arg Pro Ala Pro Gly Ser Thr Ala Pro Pro Ala His Gly Val Thr Ser
   865                 870                 875                 880
   Ala Pro Asp Thr Arg Pro Ala Pro Gly Ser Thr Ala Pro Pro Ala His
                   885                 890                 895
   Gly Val Thr Ser Ala Pro Asp Thr Arg Pro Ala Pro Gly Ser Thr Ala
   900 905 910
   Pro Pro Ala His Gly Val Thr Ser Ala Pro Asp Thr Arg Pro Ala Pro
   915 920 925
   Gly Ser Thr Ala Pro Pro Ala His Gly Val Thr Ser Ala Pro Asp Asn
   930 935 940
   Arg Pro Ala Leu Gly Ser Thr Ala Pro Pro Val His Asn Val Thr Ser
   945 950 955 960
   Ala Ser Gly Ser Ala Ser Gly Ser Ala Ser Thr Leu Val His Asn Gly
   965 970 975
   Thr Ser Ala Arg Ala Thr Thr Thr Pro Ala Ser Lys Ser Thr Pro Phe
   980 985 990
   Ser Ile Pro Ser His His Ser Asp Thr Pro Thr Thr Leu Ala Ser His
   995 1000 1005
   Ser Thr Lys Thr Asp Ala Ser Ser Thr His His Ser Ser Val Pro
   1010 1015 1020
   Pro Leu Thr Ser Ser Asn His Ser Thr Ser Pro Gln Leu Ser Thr
   1025 1030 1035
   Gly Val Ser Phe Phe Phe Leu Ser Phe His Ile Ser Asn Leu Gln
   1040 1045 1050
   Phe Asn Ser Ser Leu Glu Asp Pro Ser Thr Asp Tyr Tyr Gln Glu
   1055 1060 1065
   Leu Gln Arg Asp Ile Ser Glu Met Phe Leu Gln Ile Tyr Lys Gln
   1070 1075 1080
   Gly Gly Phe Leu Gly Leu Ser Asn Ile Lys Phe Arg Pro Gly Ser
   1085 1090 1095
   Val Val Val Gln Leu Thr Leu Ala Phe Arg Glu Gly Thr Ile Asn
   1100 1105 1110
   Val His Asp Val Glu Thr Gln Phe Asn Gln Tyr Lys Thr Glu Ala
   1115 1120 1125
   Ala Ser Arg Tyr Asn Leu Thr Ile Ser Asp Val Ser Val Ser Asp
                   1130                1135                1140
   Val Pro Phe Pro Phe Ser Ala Gln Ser Gly Ala Gly Val Pro Gly
       1145                1150                1155
   Trp Gly Ile Ala Leu Leu Val Leu Val Cys Val Leu Val Ala Leu
       1160                1165                1170
   Ala Ile Val Tyr Leu Ile Ala Leu Ala Val Cys Gln Cys Arg Arg
       1175                1180                1185
   Lys Asn Tyr Gly Gln Leu Asp Ile Phe Pro Ala Arg Asp Thr Tyr
       1190                1195                1200
   His Pro Met Ser Glu Tyr Pro Thr Tyr His Thr His Gly Arg Tyr
       1205                1210                1215
   Val Pro Pro Ser Ser Thr Asp Arg Ser Pro Tyr Glu Lys Val Ser
       1220                1225                1230
   Ala Gly Asn Gly Gly Ser Ser Leu Ser Tyr Thr Asn Pro Ala Val
       1235                1240                1245
   Ala Ala Ala Ser Ala Asn Leu
       1250                1255
<210> 2
   <211> 19
   <212> PRT
   <213> Artificial Sequence
<220>
   <223> N-terminal MUC-1 signaling sequence
<400> 2
   Met Thr Pro Gly Thr Gln Ser Pro Phe Phe Leu Leu Leu Leu Leu Thr
   1               5                   10                  15
   Val Leu Thr
<210> 3
   <211> 23
   <212> PRT
   <213> Artificial Sequence
<220>
   <223> N-terminal MUC-1 signaling sequence
<400> 3
   Met Thr Pro Gly Thr Gln Ser Pro Phe Phe Leu Leu Leu Leu Leu Thr
   1               5                   10                  15
   Val Leu Thr Val Val Thr Ala
               20
<210> 4
   <211> 23
   <212> PRT
   <213> Artificial Sequence
<220>
   <223> N-terminal MUC-1 signaling sequence
<400> 4
   Met Thr Pro Gly Thr Gln Ser Pro Phe Phe Leu Leu Leu Leu Leu Thr
   1               5                   10                  15
   Val Leu Thr Val Val Thr Gly
               20
<210> 5
   <211> 146
   <212> PRT
   <213> Artificial Sequence
<220>
   <223> truncated MUC1 receptor isoform having nat-PSMGFR at its N-
   terminus
<400> 5
   Gly Thr Ile Asn Val His Asp Val Glu Thr Gln Phe Asn Gln Tyr Lys
   1               5                   10                  15
   Thr Glu Ala Ala Ser Arg Tyr Asn Leu Thr Ile Ser Asp Val Ser Val
               20                  25                  30
   Ser Asp Val Pro Phe Pro Phe Ser Ala Gln Ser Gly Ala Gly Val Pro
           35                  40                  45
   Gly Trp Gly Ile Ala Leu Leu Val Leu Val Cys Val Leu Val Ala Leu
       50                  55                  60
   Ala Ile Val Tyr Leu Ile Ala Leu Ala Val Cys Gln Cys Arg Arg Lys
   65                  70                  75                  80
   Asn Tyr Gly Gln Leu Asp Ile Phe Pro Ala Arg Asp Thr Tyr His Pro
                   85                  90                  95
   Met Ser Glu Tyr Pro Thr Tyr His Thr His Gly Arg Tyr Val Pro Pro
               100                 105                 110
   Ser Ser Thr Asp Arg Ser Pro Tyr Glu Lys Val Ser Ala Gly Asn Gly
           115                 120                 125
   Gly Ser Ser Leu Ser Tyr Thr Asn Pro Ala Val Ala Ala Ala Ser Ala
       130                 135                 140
   Asn Leu
   145
<210> 6
   <211> 45
   <212> PRT
   <213> Artificial Sequence
<220>
   <223> Native Primary Sequence of the MUC1 Growth Factor Receptor
<400> 6
   Gly Thr Ile Asn Val His Asp Val Glu Thr Gln Phe Asn Gln Tyr Lys
   1               5                   10                  15
   Thr Glu Ala Ala Ser Arg Tyr Asn Leu Thr Ile Ser Asp Val Ser Val
               20                  25                  30
   Ser Asp Val Pro Phe Pro Phe Ser Ala Gln Ser Gly Ala
           35                  40                  45
<210> 7
   <211> 44
   <212> PRT
   <213> Artificial Sequence
<220>
   <223> Native Primary Sequence of the MUC1 Growth Factor Receptor
<400> 7
   Thr Ile Asn Val His Asp Val Glu Thr Gln Phe Asn Gln Tyr Lys Thr
   1               5                   10                  15
   Glu Ala Ala Ser Arg Tyr Asn Leu Thr Ile Ser Asp Val Ser Val Ser
               20                  25                  30
   Asp Val Pro Phe Pro Phe Ser Ala Gln Ser Gly Ala
           35                  40
<210> 8
   <211> 45
   <212> PRT
   <213> Artificial Sequence
<220>
   <223> "SPY" functional variant of the native Primary Sequence of the MUC1 Growth Factor Receptor
<400> 8
   Gly Thr Ile Asn Val His Asp Val Glu Thr Gln Phe Asn Gln Tyr Lys
   1               5                   10                  15
   Thr Glu Ala Ala Ser Pro Tyr Asn Leu Thr Ile Ser Asp Val Ser Val
               20                  25                  30
   Ser Asp Val Pro Phe Pro Phe Ser Ala Gln Ser Gly Ala
           35                  40                  45
<210> 9
   <211> 44
   <212> PRT
   <213> Artificial Sequence
<220>
   <223> SPY" functional variant of the native Primary Sequence of the MUC1 Growth Factor Receptor
<400> 9
   Thr Ile Asn Val His Asp Val Glu Thr Gln Phe Asn Gln Tyr Lys Thr
   1               5                   10                  15
   Glu Ala Ala Ser Pro Tyr Asn Leu Thr Ile Ser Asp Val Ser Val Ser
               20                  25                  30
   Asp Val Pro Phe Pro Phe Ser Ala Gln Ser Gly Ala
           35                  40
<210> 10
   <211> 216
   <212> DNA
   <213> Artificial Sequence
<220>
   <223> MUC1 cytoplasmic domain nucleotide sequence
<400> 10
   tgtcagtgcc gccgaaagaa ctacgggcag ctggacatct ttccagcccg ggatacctac       60
   catcctatga gcgagtaccc cacctaccac acccatgggc gctatgtgcc ccctagcagt      120
   accgatcgta gcccctatga gaaggtttct gcaggtaacg gtggcagcag cctctcttac      180
   acaaacccag cagtggcagc cgcttctgcc aacttg                                216
<210> 11
   <211> 72
   <212> PRT
   <213> Artificial Sequence
<220>
   <223> MUC1 cytoplasmic domain amino acid sequence
<400> 11
   Cys Gln Cys Arg Arg Lys Asn Tyr Gly Gln Leu Asp Ile Phe Pro Ala
   1               5                   10                  15
   Arg Asp Thr Tyr His Pro Met Ser Glu Tyr Pro Thr Tyr His Thr His
               20                  25                  30
   Gly Arg Tyr Val Pro Pro Ser Ser Thr Asp Arg Ser Pro Tyr Glu Lys
           35                  40                  45
   Val Ser Ala Gly Asn Gly Gly Ser Ser Leu Ser Tyr Thr Asn Pro Ala
       50                  55                  60
   Val Ala Ala Ala Ser Ala Asn Leu
   65                  70
<210> 12
   <211> 854
   <212> DNA
   <213> Artificial Sequence
<220>
   <223> NME7 nucleotide sequence
<400> 12
   gagatcctga gacaatgaat catagtgaaa gattcgtttt cattgcagag tggtatgatc      60
   caaatgcttc acttcttcga cgttatgagc ttttatttta cccaggggat ggatctgttg     120
   aaatgcatga tgtaaagaat catcgcacct ttttaaagcg gaccaaatat gataacctgc     180
   acttggaaga tttatttata ggcaacaaag tgaatgtctt ttctcgacaa ctggtattaa     240
   ttgactatgg ggatcaatat acagctcgcc agctgggcag taggaaagaa aaaacgctag     300
   ccctaattaa accagatgca atatcaaagg ctggagaaat aattgaaata ataaacaaag     360
   ctggatttac tataaccaaa ctcaaaatga tgatgctttc aaggaaagaa gcattggatt     420
   ttcatgtaga tcaccagtca agaccctttt tcaatgagct gatccagttt attacaactg     480
   gtcctattat tgccatggag attttaagag atgatgctat atgtgaatgg aaaagactgc     540
   tgggacctgc aaactctgga gtggcacgca cagatgcttc tgaaagcatt agagccctct     600
   ttggaacaga tggcataaga aatgcagcgc atggccctga ttcttttgct tctgcggcca     660
   gagaaatgga gttgtttttt ccttcaagtg gaggttgtgg gccggcaaac actgctaaat     720
   ttactaattg tacctgttgc attgttaaac cccatgctgt cagtgaaggt atgttgaata     780
   cactatattc agtacatttt gttaatagga gagcaatgtt tattttcttg atgtacttta     840
   tgtatagaaa ataa                                                       854
<210> 13
   <211> 283
   <212> PRT
   <213> Artificial Sequence
<220>
   <223> NME7 amino acid sequence
<400> 13
   Asp Pro Glu Thr Met Asn His Ser Glu Arg Phe Val Phe Ile Ala Glu
   1               5                   10                  15
   Trp Tyr Asp Pro Asn Ala Ser Leu Leu Arg Arg Tyr Glu Leu Leu Phe
               20                  25                  30
   Tyr Pro Gly Asp Gly Ser Val Glu Met His Asp Val Lys Asn His Arg
           35                  40                  45
   Thr Phe Leu Lys Arg Thr Lys Tyr Asp Asn Leu His Leu Glu Asp Leu
       50                  55                  60
   Phe Ile Gly Asn Lys Val Asn Val Phe Ser Arg Gln Leu Val Leu Ile
   65                  70                  75                  80
   Asp Tyr Gly Asp Gln Tyr Thr Ala Arg Gln Leu Gly Ser Arg Lys Glu
                   85                  90                  95
   Lys Thr Leu Ala Leu Ile Lys Pro Asp Ala Ile Ser Lys Ala Gly Glu
               100                 105                 110
   Ile Ile Glu Ile Ile Asn Lys Ala Gly Phe Thr Ile Thr Lys Leu Lys
           115                 120                 125
   Met Met Met Leu Ser Arg Lys Glu Ala Leu Asp Phe His Val Asp His
       130                 135                 140
   Gln Ser Arg Pro Phe Phe Asn Glu Leu Ile Gln Phe Ile Thr Thr Gly
   145                 150                 155                 160
   Pro Ile Ile Ala Met Glu Ile Leu Arg Asp Asp Ala Ile Cys Glu Trp
                   165                 170                 175
   Lys Arg Leu Leu Gly Pro Ala Asn Ser Gly Val Ala Arg Thr Asp Ala
               180                 185                 190
   Ser Glu Ser Ile Arg Ala Leu Phe Gly Thr Asp Gly Ile Arg Asn Ala
           195                 200                 205
   Ala His Gly Pro Asp Ser Phe Ala Ser Ala Ala Arg Glu Met Glu Leu
       210                 215                 220
   Phe Phe Pro Ser Ser Gly Gly Cys Gly Pro Ala Asn Thr Ala Lys Phe
   225                 230                 235                 240
   Thr Asn Cys Thr Cys Cys Ile Val Lys Pro His Ala Val Ser Glu Gly
                   245                 250                 255
   Met Leu Asn Thr Leu Tyr Ser Val His Phe Val Asn Arg Arg Ala Met
               260                 265                 270
   Phe Ile Phe Leu Met Tyr Phe Met Tyr Arg Lys
           275                 280
<210> 14
   <211> 534
   <212> DNA
   <213> Artificial Sequence
<220>
   <223> NM23-H1 nucleotide sequence
<400> 14
   atggtgctac tgtctacttt agggatcgtc tttcaaggcg aggggcctcc tatctcaagc      60
   tgtgatacag gaaccatggc caactgtgag cgtaccttca ttgcgatcaa accagatggg     120
   gtccagcggg gtcttgtggg agagattatc aagcgttttg agcagaaagg attccgcctt     180
   gttggtctga aattcatgca agcttccgaa gatcttctca aggaacacta cgttgacctg     240
   aaggaccgtc cattctttgc cggcctggtg aaatacatgc actcagggcc ggtagttgcc     300
   atggtctggg aggggctgaa tgtggtgaag acgggccgag tcatgctcgg ggagaccaac     360
   cctgcagact ccaagcctgg gaccatccgt ggagacttct gcatacaagt tggcaggaac     420
   attatacatg gcagtgattc tgtggagagt gcagagaagg agatcggctt gtggtttcac     480
   cctgaggaac tggtagatta cacgagctgt gctcagaact ggatctatga atga           534
<210> 15
   <211> 177
   <212> PRT
   <213> Artificial Sequence
<220>
   <223> NM23-H1 describes amino acid sequence
<400> 15
   Met Val Leu Leu Ser Thr Leu Gly Ile Val Phe Gln Gly Glu Gly Pro
   1               5                   10                  15
   Pro Ile Ser Ser Cys Asp Thr Gly Thr Met Ala Asn Cys Glu Arg Thr
               20                  25                  30
   Phe Ile Ala Ile Lys Pro Asp Gly Val Gln Arg Gly Leu Val Gly Glu
           35                  40                  45
   Ile Ile Lys Arg Phe Glu Gln Lys Gly Phe Arg Leu Val Gly Leu Lys
       50                  55                  60
   Phe Met Gln Ala Ser Glu Asp Leu Leu Lys Glu His Tyr Val Asp Leu
   65                  70                  75                  80
   Lys Asp Arg Pro Phe Phe Ala Gly Leu Val Lys Tyr Met His Ser Gly
                   85                  90                  95
   Pro Val Val Ala Met Val Trp Glu Gly Leu Asn Val Val Lys Thr Gly
               100                 105                 110
   Arg Val Met Leu Gly Glu Thr Asn Pro Ala Asp Ser Lys Pro Gly Thr
           115                 120                 125
   Ile Arg Gly Asp Phe Cys Ile Gln Val Gly Arg Asn Ile Ile His Gly
       130                 135                 140
   Ser Asp Ser Val Glu Ser Ala Glu Lys Glu Ile Gly Leu Trp Phe His
   145                 150                 155                 160
   Pro Glu Glu Leu Val Asp Tyr Thr Ser Cys Ala Gln Asn Trp Ile Tyr
                   165                 170                 175
   Glu
<210> 16
   <211> 534
   <212> DNA
   <213> Artificial Sequence
<220>
   <223> NM23-H1 S120G mutant nucleotide sequence
<400> 16
   atggtgctac tgtctacttt agggatcgtc tttcaaggcg aggggcctcc tatctcaagc        60
   tgtgatacag gaaccatggc caactgtgag cgtaccttca ttgcgatcaa accagatggg       120
   gtccagcggg gtcttgtggg agagattatc aagcgttttg agcagaaagg attccgcctt       180
   gttggtctga aattcatgca agcttccgaa gatcttctca aggaacacta cgttgacctg       240
   aaggaccgtc cattctttgc cggcctggtg aaatacatgc actcagggcc ggtagttgcc       300
   atggtctggg aggggctgaa tgtggtgaag acgggccgag tcatgctcgg ggagaccaac       360
   cctgcagact ccaagcctgg gaccatccgt ggagacttct gcatacaagt tggcaggaac       420
   attatacatg gcggtgattc tgtggagagt gcagagaagg agatcggctt gtggtttcac       480
   cctgaggaac tggtagatta cacgagctgt gctcagaact ggatctatga atga             534
<210> 17
   <211> 177
   <212> PRT
   <213> Artificial Sequence
<220>
   <223> NM23-H1 S120G mutant amino acid sequence
<400> 17 Glu
<210> 18
   <211> 459
   <212> DNA
   <213> Artificial Sequence
<220>
   <223> NM23-H2 nucleotide sequence
<400> 18
   atggccaacc tggagcgcac cttcatcgcc atcaagccgg acggcgtgca gcgcggcctg      60
   gtgggcgaga tcatcaagcg cttcgagcag aagggattcc gcctcgtggc catgaagttc     120
   ctccgggcct ctgaagaaca cctgaagcag cactacattg acctgaaaga ccgaccattc     180
   ttccctgggc tggtgaagta catgaactca gggccggttg tggccatggt ctgggagggg     240
   ctgaacgtgg tgaagacagg ccgagtgatg cttggggaga ccaatccagc agattcaaag     300
   ccaggcacca ttcgtgggga cttctgcatt caggttggca ggaacatcat tcatggcagt     360
   gattcagtaa aaagtgctga aaaagaaatc agcctatggt ttaagcctga agaactggtt     420
   gactacaagt cttgtgctca tgactgggtc tatgaataa                            459
<210> 19
   <211> 152
   <212> PRT
   <213> Artificial Sequence
<220>
   <223> NM23-H2 amino acid sequence
<400> 19
   Met Ala Asn Leu Glu Arg Thr Phe Ile Ala Ile Lys Pro Asp Gly Val
   1               5                   10                  15
   Gln Arg Gly Leu Val Gly Glu Ile Ile Lys Arg Phe Glu Gln Lys Gly
               20                  25                  30
   Phe Arg Leu Val Ala Met Lys Phe Leu Arg Ala Ser Glu Glu His Leu
           35                  40                  45
   Lys Gln His Tyr Ile Asp Leu Lys Asp Arg Pro Phe Phe Pro Gly Leu
       50                  55                  60
   Val Lys Tyr Met Asn Ser Gly Pro Val Val Ala Met Val Trp Glu Gly
   65                  70                  75                  80
   Leu Asn Val Val Lys Thr Gly Arg Val Met Leu Gly Glu Thr Asn Pro
                   85                  90                  95
   Ala Asp Ser Lys Pro Gly Thr Ile Arg Gly Asp Phe Cys Ile Gln Val
               100                 105                 110
   Gly Arg Asn Ile Ile His Gly Ser Asp Ser Val Lys Ser Ala Glu Lys
           115                 120                 125
   Glu Ile Ser Leu Trp Phe Lys Pro Glu Glu Leu Val Asp Tyr Lys Ser
       130                 135                 140
   Cys Ala His Asp Trp Val Tyr Glu
   145                 150
<210> 20
   <211> 1023
   <212> DNA
   <213> Artificial Sequence
<220>
   <223> Human NM23-H7-2 sequence optimized for E. coli expression
<400> 20
   atgcatgacg ttaaaaatca ccgtaccttt ctgaaacgca cgaaatatga taatctgcat 60
   ctggaagacc tgtttattgg caacaaagtc aatgtgttct ctcgtcagct ggtgctgatc 120
   gattatggcg accagtacac cgcgcgtcaa ctgggtagtc gcaaagaaaa aacgctggcc 180
   ctgattaaac cggatgcaat ctccaaagct ggcgaaatta tcgaaattat caacaaagcg 240
   ggtttcacca tcacgaaact gaaaatgatg atgctgagcc gtaaagaagc cctggatttt 300
   catgtcgacc accagtctcg cccgtttttc aatgaactga ttcaattcat caccacgggt 360
   ccgattatcg caatggaaat tctgcgtgat gacgctatct gcgaatggaa acgcctgctg 420
   ggcccggcaa actcaggtgt tgcgcgtacc gatgccagtg aatccattcg cgctctgttt 480
   ggcaccgatg gtatccgtaa tgcagcacat ggtccggact cattcgcatc ggcagctcgt 540
   gaaatggaac tgtttttccc gagctctggc ggttgcggtc cggcaaacac cgccaaattt 600
   accaattgta cgtgctgtat tgtcaaaccg cacgcagtgt cagaaggcct gctgggtaaa 660
   attctgatgg caatccgtga tgctggcttt gaaatctcgg ccatgcagat gttcaacatg 720
   gaccgcgtta acgtcgaaga attctacgaa gtttacaaag gcgtggttac cgaatatcac 780
   gatatggtta cggaaatgta ctccggtccg tgcgtcgcga tggaaattca gcaaaacaat 840
   gccaccaaaa cgtttcgtga attctgtggt ccggcagatc cggaaatcgc acgtcatctg 900
   cgtccgggta ccctgcgcgc aatttttggt aaaacgaaaa tccagaacgc tgtgcactgt 960
   accgatctgc cggaagacgg tctgctggaa gttcaatact ttttcaaaat tctggataat 1020
   tga 1023
<210> 21
   <211> 340
   <212> PRT
   <213> Artificial Sequence
<220>
   <223> Human NM23-H7-2 sequence optimized for E. coli expression
<400> 21
   Met His Asp Val Lys Asn His Arg Thr Phe Leu Lys Arg Thr Lys Tyr
   1               5                   10                  15
   Asp Asn Leu His Leu Glu Asp Leu Phe Ile Gly Asn Lys Val Asn Val
               20                  25                  30
   Phe Ser Arg Gln Leu Val Leu Ile Asp Tyr Gly Asp Gln Tyr Thr Ala
           35                  40                  45
   Arg Gln Leu Gly Ser Arg Lys Glu Lys Thr Leu Ala Leu Ile Lys Pro
       50                  55                  60
   Asp Ala Ile Ser Lys Ala Gly Glu Ile Ile Glu Ile Ile Asn Lys Ala
   65                  70                  75                  80
   Gly Phe Thr Ile Thr Lys Leu Lys Met Met Met Leu Ser Arg Lys Glu
                   85                  90                  95
   Ala Leu Asp Phe His Val Asp His Gln Ser Arg Pro Phe Phe Asn Glu
               100                 105                 110
   Leu Ile Gln Phe Ile Thr Thr Gly Pro Ile Ile Ala Met Glu Ile Leu
           115                 120                 125
   Arg Asp Asp Ala Ile Cys Glu Trp Lys Arg Leu Leu Gly Pro Ala Asn
       130                 135                 140
   Ser Gly Val Ala Arg Thr Asp Ala Ser Glu Ser Ile Arg Ala Leu Phe
   145                 150                 155                 160
   Gly Thr Asp Gly Ile Arg Asn Ala Ala His Gly Pro Asp Ser Phe Ala
                   165                 170                 175
   Ser Ala Ala Arg Glu Met Glu Leu Phe Phe Pro Ser Ser Gly Gly Cys
               180                 185                 190
   Gly Pro Ala Asn Thr Ala Lys Phe Thr Asn Cys Thr Cys Cys Ile Val
           195                 200                 205
   Lys Pro His Ala Val Ser Glu Gly Leu Leu Gly Lys Ile Leu Met Ala
       210                 215                 220
   Ile Arg Asp Ala Gly Phe Glu Ile Ser Ala Met Gln Met Phe Asn Met
   225                 230                 235                 240
   Asp Arg Val Asn Val Glu Glu Phe Tyr Glu Val Tyr Lys Gly Val Val
                   245                 250                 255
   Thr Glu Tyr His Asp Met Val Thr Glu Met Tyr Ser Gly Pro Cys Val
               260                 265                 270
   Ala Met Glu Ile Gln Gln Asn Asn Ala Thr Lys Thr Phe Arg Glu Phe
           275                 280                 285
   Cys Gly Pro Ala Asp Pro Glu Ile Ala Arg His Leu Arg Pro Gly Thr
       290                 295                 300
   Leu Arg Ala Ile Phe Gly Lys Thr Lys Ile Gln Asn Ala Val His Cys
   305                 310                 315                 320
   Thr Asp Leu Pro Glu Asp Gly Leu Leu Glu Val Gln Tyr Phe Phe Lys
                   325                 330                 335
   Ile Leu Asp Asn
               340
<210> 22
   <211> 399
   <212> DNA
   <213> Artificial Sequence
<220>
   <223> Human NME7-A
<400> 22
   atggaaaaaa cgctagccct aattaaacca gatgcaatat caaaggctgg agaaataatt      60
   gaaataataa acaaagctgg atttactata accaaactca aaatgatgat gctttcaagg     120
   aaagaagcat tggattttca tgtagatcac cagtcaagac cctttttcaa tgagctgatc     180
   cagtttatta caactggtcc tattattgcc atggagattt taagagatga tgctatatgt     240
   gaatggaaaa gactgctggg acctgcaaac tctggagtgg cacgcacaga tgcttctgaa     300
   agcattagag ccctctttgg aacagatggc ataagaaatg cagcgcatgg ccctgattct     360
   tttgcttctg cggccagaga aatggagttg tttttttga                             399
<210> 23
   <211> 132
   <212> PRT
   <213> Artificial Sequence
<220>
   <223> Human NME7-A
<400> 23
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe
       130
<210> 24
   <211> 444
   <212> DNA
   <213> Artificial Sequence
<220>
   <223> Human NME7-A1
<400> 24
   atggaaaaaa cgctagccct aattaaacca gatgcaatat caaaggctgg agaaataatt        60
   gaaataataa acaaagctgg atttactata accaaactca aaatgatgat gctttcaagg       120
   aaagaagcat tggattttca tgtagatcac cagtcaagac cctttttcaa tgagctgatc      180
   cagtttatta caactggtcc tattattgcc atggagattt taagagatga tgctatatgt       240
   gaatggaaaa gactgctggg acctgcaaac tctggagtgg cacgcacaga tgcttctgaa       300
   agcattagag ccctctttgg aacagatggc ataagaaatg cagcgcatgg ccctgattct       360
   tttgcttctg cggccagaga aatggagttg ttttttcctt caagtggagg ttgtgggccg       420
   gcaaacactg ctaaatttac ttga                                              444
<210> 25
   <211> 147
   <212> PRT
   <213> Artificial Sequence
<220>
   <223> Human NME7-A1
<400> 25
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe Pro Ser Ser Gly Gly Cys Gly Pro Ala Asn Thr Ala
       130                 135                 140
   Lys Phe Thr
   145
<210> 26
   <211> 669
   <212> DNA
   <213> Artificial Sequence
<220>
   <223> Human NME7-A2
<400> 26
   atgaatcata gtgaaagatt cgttttcatt gcagagtggt atgatccaaa tgcttcactt        60
   cttcgacgtt atgagctttt attttaccca ggggatggat ctgttgaaat gcatgatgta       120
   aagaatcatc gcaccttttt aaagcggacc aaatatgata acctgcactt ggaagattta       180
   tttataggca acaaagtgaa tgtcttttct cgacaactgg tattaattga ctatggggat       240
   caatatacag ctcgccagct gggcagtagg aaagaaaaaa cgctagccct aattaaacca       300
   gatgcaatat caaaggctgg agaaataatt gaaataataa acaaagctgg atttactata       360
   accaaactca aaatgatgat gctttcaagg aaagaagcat tggattttca tgtagatcac       420
   cagtcaagac cctttttcaa tgagctgatc cagtttatta caactggtcc tattattgcc       480
   atggagattt taagagatga tgctatatgt gaatggaaaa gactgctggg acctgcaaac       540
   tctggagtgg cacgcacaga tgcttctgaa agcattagag ccctctttgg aacagatggc       600
   ataagaaatg cagcgcatgg ccctgattct tttgcttctg cggccagaga aatggagttg       660
   tttttttga                                                            669
<210> 27
   <211> 222
   <212> PRT
   <213> Artificial Sequence
<220>
   <223> Human NME7-A2
<400> 27
   Met Asn His Ser Glu Arg Phe Val Phe Ile Ala Glu Trp Tyr Asp Pro
   1               5                   10                  15
   Asn Ala Ser Leu Leu Arg Arg Tyr Glu Leu Leu Phe Tyr Pro Gly Asp
               20                  25                  30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe
       210                 215                 220
<210> 28
   <211> 714
   <212> DNA
   <213> Artificial Sequence
<220>
   <223> Human NME7-A3
<400> 28
   atgaatcata gtgaaagatt cgttttcatt gcagagtggt atgatccaaa tgcttcactt       60
   cttcgacgtt atgagctttt attttaccca ggggatggat ctgttgaaat gcatgatgta      120
   aagaatcatc gcaccttttt aaagcggacc aaatatgata acctgcactt ggaagattta      180
   tttataggca acaaagtgaa tgtcttttct cgacaactgg tattaattga ctatggggat      240
   caatatacag ctcgccagct gggcagtagg aaagaaaaaa cgctagccct aattaaacca      300
   gatgcaatat caaaggctgg agaaataatt gaaataataa acaaagctgg atttactata      360
   accaaactca aaatgatgat gctttcaagg aaagaagcat tggattttca tgtagatcac      420
   cagtcaagac cctttttcaa tgagctgatc cagtttatta caactggtcc tattattgcc      480
   atggagattt taagagatga tgctatatgt gaatggaaaa gactgctggg acctgcaaac      540
   tctggagtgg cacgcacaga tgcttctgaa agcattagag ccctctttgg aacagatggc      600
   ataagaaatg cagcgcatgg ccctgattct tttgcttctg cggccagaga aatggagttg      660
   ttttttcctt caagtggagg ttgtgggccg gcaaacactg ctaaatttac ttga            714
<210> 29
   <211> 237
   <212> PRT
   <213> Artificial Sequence
<220>
   <223> Human NME7-A3
<400> 29
   Met Asn His Ser Glu Arg Phe Val Phe Ile Ala Glu Trp Tyr Asp Pro
   1               5                   10                  15
   Asn Ala Ser Leu Leu Arg Arg Tyr Glu Leu Leu Phe Tyr Pro Gly Asp
               20                  25                  30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe Pro Ser
       210                 215                 220
   Ser Gly Gly Cys Gly Pro Ala Asn Thr Ala Lys Phe Thr
   225                 230                 235
<210> 30
   <211> 408
   <212> DNA
   <213> Artificial Sequence
<220>
   <223> Human NME7-B
<400> 30
   atgaattgta cctgttgcat tgttaaaccc catgctgtca gtgaaggact gttgggaaag      60
   atcctgatgg ctatccgaga tgcaggtttt gaaatctcag ctatgcagat gttcaatatg     120
   gatcgggtta atgttgagga attctatgaa gtttataaag gagtagtgac cgaatatcat     180
   gacatggtga cagaaatgta ttctggccct tgtgtagcaa tggagattca acagaataat     240
   gctacaaaga catttcgaga attttgtgga cctgctgatc ctgaaattgc ccggcattta     300
   cgccctggaa ctctcagagc aatctttggt aaaactaaga tccagaatgc tgttcactgt     360
   actgatctgc cagaggatgg cctattagag gttcaatact tcttctga                  408
<210> 31
   <211> 135
   <212> PRT
   <213> Artificial Sequence
<220>
   <223> Human NME7-B
<400> 31
   Met Asn Cys Thr Cys Cys Ile Val Lys Pro His Ala Val Ser Glu Gly
   1               5                   10                  15
   Leu Leu Gly Lys Ile Leu Met Ala Ile Arg Asp Ala Gly Phe Glu Ile
               20                  25                  30
   Ser Ala Met Gln Met Phe Asn Met Asp Arg Val Asn Val Glu Glu Phe
           35                  40                  45
   Tyr Glu Val Tyr Lys Gly Val Val Thr Glu Tyr His Asp Met Val Thr
       50                  55                  60
   Glu Met Tyr Ser Gly Pro Cys Val Ala Met Glu Ile Gln Gln Asn Asn
   65 70 75 80
   Ala Thr Lys Thr Phe Arg Glu Phe Cys Gly Pro Ala Asp Pro Glu Ile
   85 90 95
   Ala Arg His Leu Arg Pro Gly Thr Leu Arg Ala Ile Phe Gly Lys Thr
   100 105 110
   Lys Ile Gln Asn Ala Val His Cys Thr Asp Leu Pro Glu Asp Gly Leu
   115 120 125
   Leu Glu Val Gln Tyr Phe Phe
   130 135
<210> 32
   <211> 426
   <212> DNA
   <213> Artificial Sequence
<220>
   <223> Human NME7-B1
<400> 32
   atgaattgta cctgttgcat tgttaaaccc catgctgtca gtgaaggact gttgggaaag 60
   atcctgatgg ctatccgaga tgcaggtttt gaaatctcag ctatgcagat gttcaatatg 120
   gatcgggtta atgttgagga attctatgaa gtttataaag gagtagtgac cgaatatcat 180
   gacatggtga cagaaatgta ttctggccct tgtgtagcaa tggagattca acagaataat 240
   gctacaaaga catttcgaga attttgtgga cctgctgatc ctgaaattgc ccggcattta 300
   cgccctggaa ctctcagagc aatctttggt aaaactaaga tccagaatgc tgttcactgt 360
   actgatctgc cagaggatgg cctattagag gttcaatact tcttcaagat cttggataat 420
   tagtga 426
<210> 33
   <211> 140
   <212> PRT
   <213> Artificial Sequence
<220>
   <223> Human NME7-B1
<400> 33
   Met Asn Cys Thr Cys Cys Ile Val Lys Pro His Ala Val Ser Glu Gly
   1               5                   10                  15
   Leu Leu Gly Lys Ile Leu Met Ala Ile Arg Asp Ala Gly Phe Glu Ile
               20                  25                  30
   Ser Ala Met Gln Met Phe Asn Met Asp Arg Val Asn Val Glu Glu Phe
           35                  40                  45
   Tyr Glu Val Tyr Lys Gly Val Val Thr Glu Tyr His Asp Met Val Thr
       50                  55                  60
   Glu Met Tyr Ser Gly Pro Cys Val Ala Met Glu Ile Gln Gln Asn Asn
   65                  70                  75                  80
   Ala Thr Lys Thr Phe Arg Glu Phe Cys Gly Pro Ala Asp Pro Glu Ile
                   85                  90                  95
   Ala Arg His Leu Arg Pro Gly Thr Leu Arg Ala Ile Phe Gly Lys Thr
               100                 105                 110
   Lys Ile Gln Asn Ala Val His Cys Thr Asp Leu Pro Glu Asp Gly Leu
           115                 120                 125
   Leu Glu Val Gln Tyr Phe Phe Lys Ile Leu Asp Asn
       130                 135                 140
<210> 34
   <211> 453
   <212> DNA
   <213> Artificial Sequence
<220>
   <223> Human NME7-B2
<400> 34
   atgccttcaa gtggaggttg tgggccggca aacactgcta aatttactaa ttgtacctgt      60
   tgcattgtta aaccccatgc tgtcagtgaa ggactgttgg gaaagatcct gatggctatc     120
   cgagatgcag gttttgaaat ctcagctatg cagatgttca atatggatcg ggttaatgtt     180
   gaggaattct atgaagttta taaaggagta gtgaccgaat atcatgacat ggtgacagaa     240
   atgtattctg gcccttgtgt agcaatggag attcaacaga ataatgctac aaagacattt     300
   cgagaatttt gtggacctgc tgatcctgaa attgcccggc atttacgccc tggaactctc     360
   agagcaatct ttggtaaaac taagatccag aatgctgttc actgtactga tctgccagag     420
   gatggcctat tagaggttca atacttcttc tga                                  453
<210> 35
   <211> 150
   <212> PRT
   <213> Artificial Sequence
<220>
   <223> Human NME7-B2
<400> 35
   Met Pro Ser Ser Gly Gly Cys Gly Pro Ala Asn Thr Ala Lys Phe Thr
   1               5                   10                  15
   Asn Cys Thr Cys Cys Ile Val Lys Pro His Ala Val Ser Glu Gly Leu
               20                  25                  30
   Leu Gly Lys Ile Leu Met Ala Ile Arg Asp Ala Gly Phe Glu Ile Ser
           35                  40                  45
   Ala Met Gln Met Phe Asn Met Asp Arg Val Asn Val Glu Glu Phe Tyr
       50                  55                  60
   Glu Val Tyr Lys Gly Val Val Thr Glu Tyr His Asp Met Val Thr Glu
   65                  70                  75                  80
   Met Tyr Ser Gly Pro Cys Val Ala Met Glu Ile Gln Gln Asn Asn Ala
                   85                  90                  95
   Thr Lys Thr Phe Arg Glu Phe Cys Gly Pro Ala Asp Pro Glu Ile Ala
               100                 105                 110
   Arg His Leu Arg Pro Gly Thr Leu Arg Ala Ile Phe Gly Lys Thr Lys
           115                 120                 125
   Ile Gln Asn Ala Val His Cys Thr Asp Leu Pro Glu Asp Gly Leu Leu
       130                 135                 140
   Glu Val Gln Tyr Phe Phe
   145                 150
<210> 36
   <211> 471
   <212> DNA
   <213> Artificial Sequence
<220>
   <223> Human NME7-B3
<400> 36
   atgccttcaa gtggaggttg tgggccggca aacactgcta aatttactaa ttgtacctgt        60
   tgcattgtta aaccccatgc tgtcagtgaa ggactgttgg gaaagatcct gatggctatc       120
   cgagatgcag gttttgaaat ctcagctatg cagatgttca atatggatcg ggttaatgtt       180
   gaggaattct atgaagttta taaaggagta gtgaccgaat atcatgacat ggtgacagaa       240
   atgtattctg gcccttgtgt agcaatggag attcaacaga ataatgctac aaagacattt       300
   cgagaatttt gtggacctgc tgatcctgaa attgcccggc atttacgccc tggaactctc       360
   agagcaatct ttggtaaaac taagatccag aatgctgttc actgtactga tctgccagag       420
   gatggcctat tagaggttca atacttcttc aagatcttgg ataattagtg a                471
<210> 37
   <211> 155
   <212> PRT
   <213> Artificial Sequence
<220>
   <223> Human NME7-B3
<400> 37
   Met Pro Ser Ser Gly Gly Cys Gly Pro Ala Asn Thr Ala Lys Phe Thr
   1               5                   10                  15
   Asn Cys Thr Cys Cys Ile Val Lys Pro His Ala Val Ser Glu Gly Leu
               20                  25                  30
   Leu Gly Lys Ile Leu Met Ala Ile Arg Asp Ala Gly Phe Glu Ile Ser
           35                  40                  45
   Ala Met Gln Met Phe Asn Met Asp Arg Val Asn Val Glu Glu Phe Tyr
       50                  55                  60
   Glu Val Tyr Lys Gly Val Val Thr Glu Tyr His Asp Met Val Thr Glu
   65                  70                  75                  80
   Met Tyr Ser Gly Pro Cys Val Ala Met Glu Ile Gln Gln Asn Asn Ala
                   85                  90                  95
   Thr Lys Thr Phe Arg Glu Phe Cys Gly Pro Ala Asp Pro Glu Ile Ala
               100                 105                 110
   Arg His Leu Arg Pro Gly Thr Leu Arg Ala Ile Phe Gly Lys Thr Lys
           115                 120                 125
   Ile Gln Asn Ala Val His Cys Thr Asp Leu Pro Glu Asp Gly Leu Leu
       130                 135                 140
   Glu Val Gln Tyr Phe Phe Lys Ile Leu Asp Asn
   145                 150                 155
<210> 38
   <211> 864
   <212> DNA
   <213> Artificial Sequence
<220>
   <223> Human NME7-AB
<400> 38
   atggaaaaaa cgctagccct aattaaacca gatgcaatat caaaggctgg agaaataatt      60
   gaaataataa acaaagctgg atttactata accaaactca aaatgatgat gctttcaagg     120
   aaagaagcat tggattttca tgtagatcac cagtcaagac cctttttcaa tgagctgatc     180
   cagtttatta caactggtcc tattattgcc atggagattt taagagatga tgctatatgt     240
   gaatggaaaa gactgctggg acctgcaaac tctggagtgg cacgcacaga tgcttctgaa     300
   agcattagag ccctctttgg aacagatggc ataagaaatg cagcgcatgg ccctgattct     360
   tttgcttctg cggccagaga aatggagttg ttttttcctt caagtggagg ttgtgggccg     420
   gcaaacactg ctaaatttac taattgtacc tgttgcattg ttaaacccca tgctgtcagt     480
   gaaggactgt tgggaaagat cctgatggct atccgagatg caggttttga aatctcagct     540
   atgcagatgt tcaatatgga tcgggttaat gttgaggaat tctatgaagt ttataaagga     600
   gtagtgaccg aatatcatga catggtgaca gaaatgtatt ctggcccttg tgtagcaatg     660
   gagattcaac agaataatgc tacaaagaca tttcgagaat tttgtggacc tgctgatcct     720
   gaaattgccc ggcatttacg ccctggaact ctcagagcaa tctttggtaa aactaagatc     780
   cagaatgctg ttcactgtac tgatctgcca gaggatggcc tattagaggt tcaatacttc     840
   ttcaagatct tggataatta gtga                                            864
<210> 39
   <211> 286
   <212> PRT
   <213> Artificial Sequence
<220>
   <223> Human NME7-AB
<400> 39
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe Pro Ser Ser Gly Gly Cys Gly Pro Ala Asn Thr Ala
       130                 135                 140
   Lys Phe Thr Asn Cys Thr Cys Cys Ile Val Lys Pro His Ala Val Ser
   145                 150                 155                 160
   Glu Gly Leu Leu Gly Lys Ile Leu Met Ala Ile Arg Asp Ala Gly Phe
                   165                 170                 175
   Glu Ile Ser Ala Met Gln Met Phe Asn Met Asp Arg Val Asn Val Glu
               180                 185                 190
   Glu Phe Tyr Glu Val Tyr Lys Gly Val Val Thr Glu Tyr His Asp Met
           195                 200                 205
   Val Thr Glu Met Tyr Ser Gly Pro Cys Val Ala Met Glu Ile Gln Gln
       210                 215                 220
   Asn Asn Ala Thr Lys Thr Phe Arg Glu Phe Cys Gly Pro Ala Asp Pro
   225                 230                 235                 240
   Glu Ile Ala Arg His Leu Arg Pro Gly Thr Leu Arg Ala Ile Phe Gly
                   245                 250                 255
   Lys Thr Lys Ile Gln Asn Ala Val His Cys Thr Asp Leu Pro Glu Asp
               260                 265                 270
   Gly Leu Leu Glu Val Gln Tyr Phe Phe Lys Ile Leu Asp Asn
           275                 280                 285
<210> 40
   <211> 846
   <212> DNA
   <213> Artificial Sequence
<220>
   <223> Human NME7-AB1
<400> 40
   atggaaaaaa cgctagccct aattaaacca gatgcaatat caaaggctgg agaaataatt      60
   gaaataataa acaaagctgg atttactata accaaactca aaatgatgat gctttcaagg     120
   aaagaagcat tggattttca tgtagatcac cagtcaagac cctttttcaa tgagctgatc    180
   cagtttatta caactggtcc tattattgcc atggagattt taagagatga tgctatatgt     240
   gaatggaaaa gactgctggg acctgcaaac tctggagtgg cacgcacaga tgcttctgaa     300
   agcattagag ccctctttgg aacagatggc ataagaaatg cagcgcatgg ccctgattct     360
   tttgcttctg cggccagaga aatggagttg ttttttcctt caagtggagg ttgtgggccg     420
   gcaaacactg ctaaatttac taattgtacc tgttgcattg ttaaacccca tgctgtcagt     480
   gaaggactgt tgggaaagat cctgatggct atccgagatg caggttttga aatctcagct     540
   atgcagatgt tcaatatgga tcgggttaat gttgaggaat tctatgaagt ttataaagga     600
   gtagtgaccg aatatcatga catggtgaca gaaatgtatt ctggcccttg tgtagcaatg     660
   gagattcaac agaataatgc tacaaagaca tttcgagaat tttgtggacc tgctgatcct     720
   gaaattgccc ggcatttacg ccctggaact ctcagagcaa tctttggtaa aactaagatc     780
   cagaatgctg ttcactgtac tgatctgcca gaggatggcc tattagaggt tcaatacttc     840
   ttctga                                                                846
<210> 41
   <211> 281
   <212> PRT
   <213> Artificial Sequence
<220>
   <223> Human NME7-AB1
<400> 41
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe Pro Ser Ser Gly Gly Cys Gly Pro Ala Asn Thr Ala
       130                 135                 140
   Lys Phe Thr Asn Cys Thr Cys Cys Ile Val Lys Pro His Ala Val Ser
   145                 150                 155                 160
   Glu Gly Leu Leu Gly Lys Ile Leu Met Ala Ile Arg Asp Ala Gly Phe
                   165                 170                 175
   Glu Ile Ser Ala Met Gln Met Phe Asn Met Asp Arg Val Asn Val Glu
               180                 185                 190
   Glu Phe Tyr Glu Val Tyr Lys Gly Val Val Thr Glu Tyr His Asp Met
           195                 200                 205
   Val Thr Glu Met Tyr Ser Gly Pro Cys Val Ala Met Glu Ile Gln Gln
       210                 215                 220
   Asn Asn Ala Thr Lys Thr Phe Arg Glu Phe Cys Gly Pro Ala Asp Pro
   225                 230                 235                 240
   Glu Ile Ala Arg His Leu Arg Pro Gly Thr Leu Arg Ala Ile Phe Gly
                   245                 250                 255
   Lys Thr Lys Ile Gln Asn Ala Val His Cys Thr Asp Leu Pro Glu Asp
               260                 265                 270
   Gly Leu Leu Glu Val Gln Tyr Phe Phe
           275                 280
<210> 42
   <211> 399
   <212> DNA
   <213> Artificial Sequence
<220>
   <223> Human NME7-A sequence optimized for E. coli expression
<400> 42
   atggaaaaaa cgctggccct gattaaaccg gatgcaatct ccaaagctgg cgaaattatc       60
   gaaattatca acaaagcggg tttcaccatc acgaaactga aaatgatgat gctgagccgt      120
   aaagaagccc tggattttca tgtcgaccac cagtctcgcc cgtttttcaa tgaactgatt      180
   caattcatca ccacgggtcc gattatcgca atggaaattc tgcgtgatga cgctatctgc      240
   gaatggaaac gcctgctggg cccggcaaac tcaggtgttg cgcgtaccga tgccagtgaa      300
   tccattcgcg ctctgtttgg caccgatggt atccgtaatg cagcacatgg tccggactca      360
   ttcgcatcgg cagctcgtga aatggaactg tttttctga                              399
<210> 43
   <211> 132
   <212> PRT
   <213> Artificial Sequence
<220>
   <223> Human NME7-A sequence optimized for E. coli expression
<400> 43
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe
       130
<210> 44
   <211> 444
   <212> DNA
   <213> Artificial Sequence
<220>
   <223> Human NME7-A1 sequence optimized for E. coli expression
<400> 44
   atggaaaaaa cgctggccct gattaaaccg gatgcaatct ccaaagctgg cgaaattatc      60
   gaaattatca acaaagcggg tttcaccatc acgaaactga aaatgatgat gctgagccgt     120
   aaagaagccc tggattttca tgtcgaccac cagtctcgcc cgtttttcaa tgaactgatt     180
   caattcatca ccacgggtcc gattatcgca atggaaattc tgcgtgatga cgctatctgc     240
   gaatggaaac gcctgctggg cccggcaaac tcaggtgttg cgcgtaccga tgccagtgaa     300
   tccattcgcg ctctgtttgg caccgatggt atccgtaatg cagcacatgg tccggactca     360
   ttcgcatcgg cagctcgtga aatggaactg tttttcccga gctctggcgg ttgcggtccg     420
   gcaaacaccg ccaaatttac ctga                                             444
<210> 45
   <211> 147
   <212> PRT
   <213> Artificial Sequence
<220>
   <223> Human NME7-A1 sequence optimized for E. coli expression
<400> 45
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe Pro Ser Ser Gly Gly Cys Gly Pro Ala Asn Thr Ala
       130                 135                 140
   Lys Phe Thr
   145
<210> 46
   <211> 669
   <212> DNA
   <213> Artificial Sequence
<220>
   <223> Human NME7-A2 sequence optimized for E. coli expression
<400> 46
   atgaatcact ccgaacgctt tgtttttatc gccgaatggt atgacccgaa tgcttccctg        60
   ctgcgccgct acgaactgct gttttatccg ggcgatggta gcgtggaaat gcatgacgtt       120
   aaaaatcacc gtacctttct gaaacgcacg aaatatgata atctgcatct ggaagacctg       180
   tttattggca acaaagtcaa tgtgttctct cgtcagctgg tgctgatcga ttatggcgac       240
   cagtacaccg cgcgtcaact gggtagtcgc aaagaaaaaa cgctggccct gattaaaccg       300
   gatgcaatct ccaaagctgg cgaaattatc gaaattatca acaaagcggg tttcaccatc       360
   acgaaactga aaatgatgat gctgagccgt aaagaagccc tggattttca tgtcgaccac       420
   cagtctcgcc cgtttttcaa tgaactgatt caattcatca ccacgggtcc gattatcgca       480
   atggaaattc tgcgtgatga cgctatctgc gaatggaaac gcctgctggg cccggcaaac       540
   tcaggtgttg cgcgtaccga tgccagtgaa tccattcgcg ctctgtttgg caccgatggt       600
   atccgtaatg cagcacatgg tccggactca ttcgcatcgg cagctcgtga aatggaactg       660
   tttttctga                                                               669
<210> 47
   <211> 222
   <212> PRT
   <213> Artificial Sequence
<220>
   <223> Human NME7-A2 sequence optimized for E. coli expression
<400> 47
   Met Asn His Ser Glu Arg Phe Val Phe Ile Ala Glu Trp Tyr Asp Pro
   1               5                   10                  15
   Asn Ala Ser Leu Leu Arg Arg Tyr Glu Leu Leu Phe Tyr Pro Gly Asp
               20                  25                  30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe
       210                 215                 220
<210> 48
   <211> 714
   <212> DNA
   <213> Artificial Sequence
<220>
   <223> Human NME7-A3 sequence optimized for E. coli expression
<400> 48
   atgaatcact ccgaacgctt tgtttttatc gccgaatggt atgacccgaa tgcttccctg       60
   ctgcgccgct acgaactgct gttttatccg ggcgatggta gcgtggaaat gcatgacgtt      120
   aaaaatcacc gtacctttct gaaacgcacg aaatatgata atctgcatct ggaagacctg      180
   tttattggca acaaagtcaa tgtgttctct cgtcagctgg tgctgatcga ttatggcgac      240
   cagtacaccg cgcgtcaact gggtagtcgc aaagaaaaaa cgctggccct gattaaaccg      300
   gatgcaatct ccaaagctgg cgaaattatc gaaattatca acaaagcggg tttcaccatc      360
   acgaaactga aaatgatgat gctgagccgt aaagaagccc tggattttca tgtcgaccac      420
   cagtctcgcc cgtttttcaa tgaactgatt caattcatca ccacgggtcc gattatcgca      480
   atggaaattc tgcgtgatga cgctatctgc gaatggaaac gcctgctggg cccggcaaac      540
   tcaggtgttg cgcgtaccga tgccagtgaa tccattcgcg ctctgtttgg caccgatggt      600
   atccgtaatg cagcacatgg tccggactca ttcgcatcgg cagctcgtga aatggaactg      660
   tttttcccga gctctggcgg ttgcggtccg gcaaacaccg ccaaatttac ctga            714
<210> 49
   <211> 237
   <212> PRT
   <213> Artificial Sequence
<220>
   <223> Human NME7-A3 sequence optimized for E. coli expression
<400> 49
   Met Asn His Ser Glu Arg Phe Val Phe Ile Ala Glu Trp Tyr Asp Pro
   1               5                   10                  15
   Asn Ala Ser Leu Leu Arg Arg Tyr Glu Leu Leu Phe Tyr Pro Gly Asp
               20                  25                  30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe Pro Ser
       210                 215                 220
   Ser Gly Gly Cys Gly Pro Ala Asn Thr Ala Lys Phe Thr
   225                 230                 235
<210> 50
   <211> 408
   <212> DNA
   <213> Artificial Sequence
<220>
   <223> Human NME7-B sequence optimized for E. coli expression
<400> 50
   atgaattgta cgtgctgtat tgtcaaaccg cacgcagtgt cagaaggcct gctgggtaaa      60
   attctgatgg caatccgtga tgctggcttt gaaatctcgg ccatgcagat gttcaacatg     120
   gaccgcgtta acgtcgaaga attctacgaa gtttacaaag gcgtggttac cgaatatcac     180
   gatatggtta cggaaatgta ctccggtccg tgcgtcgcga tggaaattca gcaaaacaat     240
   gccaccaaaa cgtttcgtga attctgtggt ccggcagatc cggaaatcgc acgtcatctg     300
   cgtccgggta ccctgcgcgc aatttttggt aaaacgaaaa tccagaacgc tgtgcactgt     360
   accgatctgc cggaagacgg tctgctggaa gttcaatact ttttctga                 408
<210> 51
   <211> 135
   <212> PRT
   <213> Artificial Sequence
<220>
   <223> Human NME7-B sequence optimized for E. coli expression
<400> 51
   Met Asn Cys Thr Cys Cys Ile Val Lys Pro His Ala Val Ser Glu Gly
   1               5                   10                  15
   Leu Leu Gly Lys Ile Leu Met Ala Ile Arg Asp Ala Gly Phe Glu Ile
               20                  25                  30
   Ser Ala Met Gln Met Phe Asn Met Asp Arg Val Asn Val Glu Glu Phe
           35                  40                  45
   Tyr Glu Val Tyr Lys Gly Val Val Thr Glu Tyr His Asp Met Val Thr
       50                  55                  60
   Glu Met Tyr Ser Gly Pro Cys Val Ala Met Glu Ile Gln Gln Asn Asn
   65                  70                  75                  80
   Ala Thr Lys Thr Phe Arg Glu Phe Cys Gly Pro Ala Asp Pro Glu Ile
                   85                  90                  95
   Ala Arg His Leu Arg Pro Gly Thr Leu Arg Ala Ile Phe Gly Lys Thr
               100                 105                 110
   Lys Ile Gln Asn Ala Val His Cys Thr Asp Leu Pro Glu Asp Gly Leu
           115                 120                 125
   Leu Glu Val Gln Tyr Phe Phe
       130                 135
<210> 52
   <211> 423
   <212> DNA
   <213> Artificial Sequence
<220>
   <223> Human NME7-B1 sequence optimized for E. coli expression
<400> 52
   atgaattgta cgtgctgtat tgtcaaaccg cacgcagtgt cagaaggcct gctgggtaaa       60
   attctgatgg caatccgtga tgctggcttt gaaatctcgg ccatgcagat gttcaacatg      120
   gaccgcgtta acgtcgaaga attctacgaa gtttacaaag gcgtggttac cgaatatcac      180
   gatatggtta cggaaatgta ctccggtccg tgcgtcgcga tggaaattca gcaaaacaat      240
   gccaccaaaa cgtttcgtga attctgtggt ccggcagatc cggaaatcgc acgtcatctg      300
   cgtccgggta ccctgcgcgc aatttttggt aaaacgaaaa tccagaacgc tgtgcactgt      360
   accgatctgc cggaagacgg tctgctggaa gttcaatact ttttcaaaat tctggataat      420
   tga                                                                    423
<210> 53
   <211> 140
   <212> PRT
   <213> Artificial Sequence
<220>
   <223> Human NME7-B1 sequence optimized for E. coli expression
<400> 53
   Met Asn Cys Thr Cys Cys Ile Val Lys Pro His Ala Val Ser Glu Gly
   1               5                   10                  15
   Leu Leu Gly Lys Ile Leu Met Ala Ile Arg Asp Ala Gly Phe Glu Ile
               20                  25                  30
   Ser Ala Met Gln Met Phe Asn Met Asp Arg Val Asn Val Glu Glu Phe
           35                  40                  45
   Tyr Glu Val Tyr Lys Gly Val Val Thr Glu Tyr His Asp Met Val Thr
       50                  55                  60
   Glu Met Tyr Ser Gly Pro Cys Val Ala Met Glu Ile Gln Gln Asn Asn
   65                  70                  75                  80
   Ala Thr Lys Thr Phe Arg Glu Phe Cys Gly Pro Ala Asp Pro Glu Ile
                   85                  90                  95
   Ala Arg His Leu Arg Pro Gly Thr Leu Arg Ala Ile Phe Gly Lys Thr
               100                 105                 110
   Lys Ile Gln Asn Ala Val His Cys Thr Asp Leu Pro Glu Asp Gly Leu
           115                 120                 125
   Leu Glu Val Gln Tyr Phe Phe Lys Ile Leu Asp Asn
       130                 135                 140
<210> 54
   <211> 453
   <212> DNA
   <213> Artificial Sequence
<220>
   <223> Human NME7-B2 sequence optimized for E. coli expression
<400> 54
   atgccgagct ctggcggttg cggtccggca aacaccgcca aatttaccaa ttgtacgtgc      60
   tgtattgtca aaccgcacgc agtgtcagaa ggcctgctgg gtaaaattct gatggcaatc     120
   cgtgatgctg gctttgaaat ctcggccatg cagatgttca acatggaccg cgttaacgtc     180
   gaagaattct acgaagttta caaaggcgtg gttaccgaat atcacgatat ggttacggaa     240
   atgtactccg gtccgtgcgt cgcgatggaa attcagcaaa acaatgccac caaaacgttt     300
   cgtgaattct gtggtccggc agatccggaa atcgcacgtc atctgcgtcc gggtaccctg     360
   cgcgcaattt ttggtaaaac gaaaatccag aacgctgtgc actgtaccga tctgccggaa     420
   gacggtctgc tggaagttca atactttttc tga                                  453
<210> 55
   <211> 150
   <212> PRT
   <213> Artificial Sequence
<220>
   <223> Human NME7-B2 sequence optimized for E. coli expression
<400> 55
   Met Pro Ser Ser Gly Gly Cys Gly Pro Ala Asn Thr Ala Lys Phe Thr
   1               5                   10                  15
   Asn Cys Thr Cys Cys Ile Val Lys Pro His Ala Val Ser Glu Gly Leu
               20                  25                  30
   Leu Gly Lys Ile Leu Met Ala Ile Arg Asp Ala Gly Phe Glu Ile Ser
           35                  40                  45
   Ala Met Gln Met Phe Asn Met Asp Arg Val Asn Val Glu Glu Phe Tyr
       50                  55                  60
   Glu Val Tyr Lys Gly Val Val Thr Glu Tyr His Asp Met Val Thr Glu
   65                  70                  75                  80
   Met Tyr Ser Gly Pro Cys Val Ala Met Glu Ile Gln Gln Asn Asn Ala
                   85                  90                  95
   Thr Lys Thr Phe Arg Glu Phe Cys Gly Pro Ala Asp Pro Glu Ile Ala
               100                 105                 110
   Arg His Leu Arg Pro Gly Thr Leu Arg Ala Ile Phe Gly Lys Thr Lys
           115                 120                 125
   Ile Gln Asn Ala Val His Cys Thr Asp Leu Pro Glu Asp Gly Leu Leu
       130                 135                 140
   Glu Val Gln Tyr Phe Phe
   145                 150
<210> 56
   <211> 468
   <212> DNA
   <213> Artificial Sequence
<220>
   <223> Human NME7-B3 sequence optimized for E. coli expression
<400> 56
   atgccgagct ctggcggttg cggtccggca aacaccgcca aatttaccaa ttgtacgtgc      60
   tgtattgtca aaccgcacgc agtgtcagaa ggcctgctgg gtaaaattct gatggcaatc     120
   cgtgatgctg gctttgaaat ctcggccatg cagatgttca acatggaccg cgttaacgtc     180
   gaagaattct acgaagttta caaaggcgtg gttaccgaat atcacgatat ggttacggaa     240
   atgtactccg gtccgtgcgt cgcgatggaa attcagcaaa acaatgccac caaaacgttt     300
   cgtgaattct gtggtccggc agatccggaa atcgcacgtc atctgcgtcc gggtaccctg     360
   cgcgcaattt ttggtaaaac gaaaatccag aacgctgtgc actgtaccga tctgccggaa     420
   gacggtctgc tggaagttca atactttttc aaaattctgg ataattga                  468
<210> 57
   <211> 155
   <212> PRT
   <213> Artificial Sequence
<220>
   <223> Human NME7-B3 sequence optimized for E. coli expression
<400> 57
   Met Pro Ser Ser Gly Gly Cys Gly Pro Ala Asn Thr Ala Lys Phe Thr
   1               5                   10                  15
   Asn Cys Thr Cys Cys Ile Val Lys Pro His Ala Val Ser Glu Gly Leu
               20                  25                  30
   Leu Gly Lys Ile Leu Met Ala Ile Arg Asp Ala Gly Phe Glu Ile Ser
           35                  40                  45
   Ala Met Gln Met Phe Asn Met Asp Arg Val Asn Val Glu Glu Phe Tyr
       50                  55                  60
   Glu Val Tyr Lys Gly Val Val Thr Glu Tyr His Asp Met Val Thr Glu
   65                  70                  75                  80
   Met Tyr Ser Gly Pro Cys Val Ala Met Glu Ile Gln Gln Asn Asn Ala
                   85                  90                  95
   Thr Lys Thr Phe Arg Glu Phe Cys Gly Pro Ala Asp Pro Glu Ile Ala
               100                 105                 110
   Arg His Leu Arg Pro Gly Thr Leu Arg Ala Ile Phe Gly Lys Thr Lys
           115                 120                 125
   Ile Gln Asn Ala Val His Cys Thr Asp Leu Pro Glu Asp Gly Leu Leu
                   130                 135                 140
   Glu Val Gln Tyr Phe Phe Lys Ile Leu Asp Asn
   145                 150                 155
<210> 58
   <211> 861
   <212> DNA
   <213> Artificial Sequence
<220>
   <223> Human NME7-AB sequence optimized for E. coli expression
<400> 58
   atggaaaaaa cgctggccct gattaaaccg gatgcaatct ccaaagctgg cgaaattatc        60
   gaaattatca acaaagcggg tttcaccatc acgaaactga aaatgatgat gctgagccgt       120
   aaagaagccc tggattttca tgtcgaccac cagtctcgcc cgtttttcaa tgaactgatt       180
   caattcatca ccacgggtcc gattatcgca atggaaattc tgcgtgatga cgctatctgc       240
   gaatggaaac gcctgctggg cccggcaaac tcaggtgttg cgcgtaccga tgccagtgaa       300
   tccattcgcg ctctgtttgg caccgatggt atccgtaatg cagcacatgg tccggactca       360
   ttcgcatcgg cagctcgtga aatggaactg tttttcccga gctctggcgg ttgcggtccg       420
   gcaaacaccg ccaaatttac caattgtacg tgctgtattg tcaaaccgca cgcagtgtca       480
   gaaggcctgc tgggtaaaat tctgatggca atccgtgatg ctggctttga aatctcggcc       540
   atgcagatgt tcaacatgga ccgcgttaac gtcgaagaat tctacgaagt ttacaaaggc       600
   gtggttaccg aatatcacga tatggttacg gaaatgtact ccggtccgtg cgtcgcgatg       660
   gaaattcagc aaaacaatgc caccaaaacg tttcgtgaat tctgtggtcc ggcagatccg       720
   gaaatcgcac gtcatctgcg tccgggtacc ctgcgcgcaa tttttggtaa aacgaaaatc       780
   cagaacgctg tgcactgtac cgatctgccg gaagacggtc tgctggaagt tcaatacttt       840
   ttcaaaattc tggataattg a                                                 861
<210> 59
   <211> 286
   <212> PRT
   <213> Artificial Sequence
<220>
   <223> Human NME7-AB sequence optimized for E. coli expression
<400> 59
   Met Glu Lys Thr Leu Ala Leu Ile Lys Pro Asp Ala Ile Ser Lys Ala
   1               5                   10                  15
   Gly Glu Ile Ile Glu Ile Ile Asn Lys Ala Gly Phe Thr Ile Thr Lys
               20                  25                  30
   Leu Lys Met Met Met Leu Ser Arg Lys Glu Ala Leu Asp Phe His Val
           35                  40                  45
   Asp His Gln Ser Arg Pro Phe Phe Asn Glu Leu Ile Gln Phe Ile Thr
       50                  55                  60
   Thr Gly Pro Ile Ile Ala Met Glu Ile Leu Arg Asp Asp Ala Ile Cys
   65                  70                  75                  80
   Glu Trp Lys Arg Leu Leu Gly Pro Ala Asn Ser Gly Val Ala Arg Thr
                   85                  90                  95
   Asp Ala Ser Glu Ser Ile Arg Ala Leu Phe Gly Thr Asp Gly Ile Arg
               100                 105                 110
   Asn Ala Ala His Gly Pro Asp Ser Phe Ala Ser Ala Ala Arg Glu Met
           115                 120                 125
   Glu Leu Phe Phe Pro Ser Ser Gly Gly Cys Gly Pro Ala Asn Thr Ala
       130                 135                 140
   Lys Phe Thr Asn Cys Thr Cys Cys Ile Val Lys Pro His Ala Val Ser
   145                 150                 155                 160
   Glu Gly Leu Leu Gly Lys Ile Leu Met Ala Ile Arg Asp Ala Gly Phe
                   165                 170                 175
   Glu Ile Ser Ala Met Gln Met Phe Asn Met Asp Arg Val Asn Val Glu
               180                 185                 190
   Glu Phe Tyr Glu Val Tyr Lys Gly Val Val Thr Glu Tyr His Asp Met
           195                 200                 205
   Val Thr Glu Met Tyr Ser Gly Pro Cys Val Ala Met Glu Ile Gln Gln
       210                 215                 220
   Asn Asn Ala Thr Lys Thr Phe Arg Glu Phe Cys Gly Pro Ala Asp Pro
   225                 230                 235                 240
   Glu Ile Ala Arg His Leu Arg Pro Gly Thr Leu Arg Ala Ile Phe Gly
                   245                 250                 255
   Lys Thr Lys Ile Gln Asn Ala Val His Cys Thr Asp Leu Pro Glu Asp
               260                 265                 270
   Gly Leu Leu Glu Val Gln Tyr Phe Phe Lys Ile Leu Asp Asn
   275 280 285
<210> 60
   <211> 846
   <212> DNA
   <213> Artificial Sequence
<220>
   <223> Human NME7-AB1 sequence optimized for E. coli expression
<400> 60
   atggaaaaaa cgctggccct gattaaaccg gatgcaatct ccaaagctgg cgaaattatc 60
   gaaattatca acaaagcggg tttcaccatc acgaaactga aaatgatgat gctgagccgt 120
   aaagaagccc tggattttca tgtcgaccac cagtctcgcc cgtttttcaa tgaactgatt 180
   caattcatca ccacgggtcc gattatcgca atggaaattc tgcgtgatga cgctatctgc 240
   gaatggaaac gcctgctggg cccggcaaac tcaggtgttg cgcgtaccga tgccagtgaa 300
   tccattcgcg ctctgtttgg caccgatggt atccgtaatg cagcacatgg tccggactca 360
   ttcgcatcgg cagctcgtga aatggaactg tttttcccga gctctggcgg ttgcggtccg 420
   gcaaacaccg ccaaatttac caattgtacg tgctgtattg tcaaaccgca cgcagtgtca 480
   gaaggcctgc tgggtaaaat tctgatggca atccgtgatg ctggctttga aatctcggcc 540
   atgcagatgt tcaacatgga ccgcgttaac gtcgaagaat tctacgaagt ttacaaaggc 600
   gtggttaccg aatatcacga tatggttacg gaaatgtact ccggtccgtg cgtcgcgatg 660
   gaaattcagc aaaacaatgc caccaaaacg tttcgtgaat tctgtggtcc ggcagatccg 720
   gaaatcgcac gtcatctgcg tccgggtacc ctgcgcgcaa tttttggtaa aacgaaaatc 780
   cagaacgctg tgcactgtac cgatctgccg gaagacggtc tgctggaagt tcaatacttt 840
   ttctga 846
<210> 61
   <211> 281
   <212> PRT
   <213> Artificial Sequence
<220>
   <223> Human NME7-AB1 sequence optimized for E. coli expression
<400> 61
   Met Glu Lys Thr Leu Ala Leu Ile Lys Pro Asp Ala Ile Ser Lys Ala
   1 5 10 15
   Gly Glu Ile Ile Glu Ile Ile Asn Lys Ala Gly Phe Thr Ile Thr Lys
   20 25 30
   Leu Lys Met Met Met Leu Ser Arg Lys Glu Ala Leu Asp Phe His Val
   35 40 45
   Asp His Gln Ser Arg Pro Phe Phe Asn Glu Leu Ile Gln Phe Ile Thr
   50 55 60
   Thr Gly Pro Ile Ile Ala Met Glu Ile Leu Arg Asp Asp Ala Ile Cys
   65 70 75 80
   Glu Trp Lys Arg Leu Leu Gly Pro Ala Asn Ser Gly Val Ala Arg Thr
   85 90 95
   Asp Ala Ser Glu Ser Ile Arg Ala Leu Phe Gly Thr Asp Gly Ile Arg
   100 105 110
   Asn Ala Ala His Gly Pro Asp Ser Phe Ala Ser Ala Ala Arg Glu Met
   115 120 125
   Glu Leu Phe Phe Pro Ser Ser Gly Gly Cys Gly Pro Ala Asn Thr Ala
   130 135 140
   Lys Phe Thr Asn Cys Thr Cys Cys Ile Val Lys Pro His Ala Val Ser
   145 150 155 160
   Glu Gly Leu Leu Gly Lys Ile Leu Met Ala Ile Arg Asp Ala Gly Phe
   165 170 175
   Glu Ile Ser Ala Met Gln Met Phe Asn Met Asp Arg Val Asn Val Glu
   180 185 190
   Glu Phe Tyr Glu Val Tyr Lys Gly Val Val Thr Glu Tyr His Asp Met
   195 200 205
   Val Thr Glu Met Tyr Ser Gly Pro Cys Val Ala Met Glu Ile Gln Gln
   210 215 220
   Asn Asn Ala Thr Lys Thr Phe Arg Glu Phe Cys Gly Pro Ala Asp Pro
   225 230 235 240
   Glu Ile Ala Arg His Leu Arg Pro Gly Thr Leu Arg Ala Ile Phe Gly
   245 250 255
   Lys Thr Lys Ile Gln Asn Ala Val His Cys Thr Asp Leu Pro Glu Asp
   260 265 270
   Gly Leu Leu Glu Val Gln Tyr Phe Phe
   275 280
<210> 62
   <211> 570
   <212> DNA
   <213> Artificial Sequence
<220>
   <223> DNA encoding Mouse NME6
<400> 62
   atgacctcca tcttgcgaag tccccaagct cttcagctca cactagccct gatcaagcct      60
   gatgcagttg cccacccact gatcctggag gctgttcatc agcagattct gagcaacaag     120
   ttcctcattg tacgaacgag ggaactgcag tggaagctgg aggactgccg gaggttttac     180
   cgagagcatg aagggcgttt tttctatcag cggctggtgg agttcatgac aagtgggcca     240
   atccgagcct atatccttgc ccacaaagat gccatccaac tttggaggac actgatggga     300
   cccaccagag tatttcgagc acgctatata gccccagatt caattcgtgg aagtttgggc     360
   ctcactgaca cccgaaatac tacccatggc tcagactccg tggtttccgc cagcagagag     420
   attgcagcct tcttccctga cttcagtgaa cagcgctggt atgaggagga ggaaccccag     480
   ctgcggtgtg gtcctgtgca ctacagtcca gaggaaggta tccactgtgc agctgaaaca     540
   ggaggccaca aacaacctaa caaaacctag                                      570
<210> 63
   <211> 189
   <212> PRT
   <213> Artificial Sequence
<220>
   <223> Mouse NME6
<400> 63
   Met Thr Ser Ile Leu Arg Ser Pro Gln Ala Leu Gln Leu Thr Leu Ala
   1               5                   10                  15
   Leu Ile Lys Pro Asp Ala Val Ala His Pro Leu Ile Leu Glu Ala Val
               20                  25                  30
   His Gln Gln Ile Leu Ser Asn Lys Phe Leu Ile Val Arg Thr Arg Glu
           35                  40                  45
   Leu Gln Trp Lys Leu Glu Asp Cys Arg Arg Phe Tyr Arg Glu His Glu
       50                  55                  60
   Gly Arg Phe Phe Tyr Gln Arg Leu Val Glu Phe Met Thr Ser Gly Pro
   65                  70                  75                  80
   Ile Arg Ala Tyr Ile Leu Ala His Lys Asp Ala Ile Gln Leu Trp Arg
                   85                  90                  95
   Thr Leu Met Gly Pro Thr Arg Val Phe Arg Ala Arg Tyr Ile Ala Pro
               100                 105                 110
   Asp Ser Ile Arg Gly Ser Leu Gly Leu Thr Asp Thr Arg Asn Thr Thr
           115                 120                 125
   His Gly Ser Asp Ser Val Val Ser Ala Ser Arg Glu Ile Ala Ala Phe
       130                 135                 140
   Phe Pro Asp Phe Ser Glu Gln Arg Trp Tyr Glu Glu Glu Glu Pro Gln
   145                 150                 155                 160
   Leu Arg Cys Gly Pro Val His Tyr Ser Pro Glu Glu Gly Ile His Cys
                   165                 170                 175
   Ala Ala Glu Thr Gly Gly His Lys Gln Pro Asn Lys Thr
               180                 185
<210> 64
   <211> 585
   <212> DNA
   <213> Artificial Sequence
<220>
   <223> DNA encoding Human NME6
<400> 64
   atgacccaga atctggggag tgagatggcc tcaatcttgc gaagccctca ggctctccag      60
   ctcactctag ccctgatcaa gcctgacgca gtcgcccatc cactgattct ggaggctgtt     120
   catcagcaga ttctaagcaa caagttcctg attgtacgaa tgagagaact actgtggaga     180
   aaggaagatt gccagaggtt ttaccgagag catgaagggc gttttttcta tcagaggctg     240
   gtggagttca tggccagcgg gccaatccga gcctacatcc ttgcccacaa ggatgccatc     300
   cagctctgga ggacgctcat gggacccacc agagtgttcc gagcacgcca tgtggcccca     360
   gattctatcc gtgggagttt cggcctcact gacacccgca acaccaccca tggttcggac     420
   tctgtggttt cagccagcag agagattgca gccttcttcc ctgacttcag tgaacagcgc     480
   tggtatgagg aggaagagcc ccagttgcgc tgtggccctg tgtgctatag cccagaggga     540
   ggtgtccact atgtagctgg aacaggaggc ctaggaccag cctga                    585
<210> 65
   <211> 194
   <212> PRT
   <213> Artificial Sequence
<220>
   <223> Human NME6
<400> 65
   Met Thr Gln Asn Leu Gly Ser Glu Met Ala Ser Ile Leu Arg Ser Pro
   1               5                   10                  15
   Gln Ala Leu Gln Leu Thr Leu Ala Leu Ile Lys Pro Asp Ala Val Ala
               20                  25                  30
   His Pro Leu Ile Leu Glu Ala Val His Gln Gln Ile Leu Ser Asn Lys
           35                  40                  45
   Phe Leu Ile Val Arg Met Arg Glu Leu Leu Trp Arg Lys Glu Asp Cys
       50                  55                  60
   Gln Arg Phe Tyr Arg Glu His Glu Gly Arg Phe Phe Tyr Gln Arg Leu
   65                  70                  75                  80
   Val Glu Phe Met Ala Ser Gly Pro Ile Arg Ala Tyr Ile Leu Ala His
                   85                  90                  95
   Lys Asp Ala Ile Gln Leu Trp Arg Thr Leu Met Gly Pro Thr Arg Val
               100                 105                 110
   Phe Arg Ala Arg His Val Ala Pro Asp Ser Ile Arg Gly Ser Phe Gly
           115                 120                 125
   Leu Thr Asp Thr Arg Asn Thr Thr His Gly Ser Asp Ser Val Val Ser
       130                 135                 140
   Ala Ser Arg Glu Ile Ala Ala Phe Phe Pro Asp Phe Ser Glu Gln Arg
   145                 150                 155                 160
   Trp Tyr Glu Glu Glu Glu Pro Gln Leu Arg Cys Gly Pro Val Cys Tyr
                   165                 170                 175
   Ser Pro Glu Gly Gly Val His Tyr Val Ala Gly Thr Gly Gly Leu Gly
               180                 185                 190
   Pro Ala
<210> 66
   <211> 525
   <212> DNA
   <213> Artificial Sequence
<220>
   <223> DNA encoding Human NME6 1
<400> 66
   atgacccaga atctggggag tgagatggcc tcaatcttgc gaagccctca ggctctccag 60
   ctcactctag ccctgatcaa gcctgacgca gtcgcccatc cactgattct ggaggctgtt 120
   catcagcaga ttctaagcaa caagttcctg attgtacgaa tgagagaact actgtggaga 180
   aaggaagatt gccagaggtt ttaccgagag catgaagggc gttttttcta tcagaggctg 240
   gtggagttca tggccagcgg gccaatccga gcctacatcc ttgcccacaa ggatgccatc 300
   cagctctgga ggacgctcat gggacccacc agagtgttcc gagcacgcca tgtggcccca 360
   gattctatcc gtgggagttt cggcctcact gacacccgca acaccaccca tggttcggac 420
   tctgtggttt cagccagcag agagattgca gccttcttcc ctgacttcag tgaacagcgc 480
   tggtatgagg aggaagagcc ccagttgcgc tgtggccctg tgtga 525
<210> 67
   <211> 174
   <212> PRT
   <213> Artificial Sequence
<220>
   <223> Human NME6 1
<400> 67
   Met Thr Gln Asn Leu Gly Ser Glu Met Ala Ser Ile Leu Arg Ser Pro
   1               5                   10                  15
   Gln Ala Leu Gln Leu Thr Leu Ala Leu Ile Lys Pro Asp Ala Val Ala
               20                  25                  30
   His Pro Leu Ile Leu Glu Ala Val His Gln Gln Ile Leu Ser Asn Lys
           35                  40                  45
   Phe Leu Ile Val Arg Met Arg Glu Leu Leu Trp Arg Lys Glu Asp Cys
       50                  55                  60
   Gln Arg Phe Tyr Arg Glu His Glu Gly Arg Phe Phe Tyr Gln Arg Leu
   65                  70                  75                  80
   Val Glu Phe Met Ala Ser Gly Pro Ile Arg Ala Tyr Ile Leu Ala His
                   85                  90                  95
   Lys Asp Ala Ile Gln Leu Trp Arg Thr Leu Met Gly Pro Thr Arg Val
               100                 105                 110
   Phe Arg Ala Arg His Val Ala Pro Asp Ser Ile Arg Gly Ser Phe Gly
           115                 120                 125
   Leu Thr Asp Thr Arg Asn Thr Thr His Gly Ser Asp Ser Val Val Ser
       130                 135                 140
   Ala Ser Arg Glu Ile Ala Ala Phe Phe Pro Asp Phe Ser Glu Gln Arg
   145                 150                 155                 160
   Trp Tyr Glu Glu Glu Glu Pro Gln Leu Arg Cys Gly Pro Val
                   165                 170
<210> 68
   <211> 468
   <212> DNA
   <213> Artificial Sequence
<220>
   <223> DNA encoding Human NME6 2
<400> 68
   atgctcactc tagccctgat caagcctgac gcagtcgccc atccactgat tctggaggct      60
   gttcatcagc agattctaag caacaagttc ctgattgtac gaatgagaga actactgtgg     120
   agaaaggaag attgccagag gttttaccga gagcatgaag ggcgtttttt ctatcagagg     180
   ctggtggagt tcatggccag cgggccaatc cgagcctaca tccttgccca caaggatgcc     240
   atccagctct ggaggacgct catgggaccc accagagtgt tccgagcacg ccatgtggcc     300
   ccagattcta tccgtgggag tttcggcctc actgacaccc gcaacaccac ccatggttcg     360
   gactctgtgg tttcagccag cagagagatt gcagccttct tccctgactt cagtgaacag     420
   cgctggtatg aggaggaaga gccccagttg cgctgtggcc ctgtgtga                 468
<210> 69
   <211> 155
   <212> PRT
   <213> Artificial Sequence
<220>
   <223> Human NME6 2
<400> 69
   Met Leu Thr Leu Ala Leu Ile Lys Pro Asp Ala Val Ala His Pro Leu
   1               5                   10                  15
   Ile Leu Glu Ala Val His Gln Gln Ile Leu Ser Asn Lys Phe Leu Ile
               20                  25                  30
   Val Arg Met Arg Glu Leu Leu Trp Arg Lys Glu Asp Cys Gln Arg Phe
           35                  40                  45
   Tyr Arg Glu His Glu Gly Arg Phe Phe Tyr Gln Arg Leu Val Glu Phe
       50                  55                  60
   Met Ala Ser Gly Pro Ile Arg Ala Tyr Ile Leu Ala His Lys Asp Ala
   65                  70                  75                  80
   Ile Gln Leu Trp Arg Thr Leu Met Gly Pro Thr Arg Val Phe Arg Ala
                   85                  90                  95
   Arg His Val Ala Pro Asp Ser Ile Arg Gly Ser Phe Gly Leu Thr Asp
               100                 105                 110
   Thr Arg Asn Thr Thr His Gly Ser Asp Ser Val Val Ser Ala Ser Arg
           115                 120                 125
   Glu Ile Ala Ala Phe Phe Pro Asp Phe Ser Glu Gln Arg Trp Tyr Glu
       130                 135                 140
   Glu Glu Glu Pro Gln Leu Arg Cys Gly Pro Val
   145                 150                 155
<210> 70
   <211> 528
   <212> DNA
   <213> Artificial Sequence
<220>
   <223> DNA encoding Human NME6 3
<400> 70
   atgctcactc tagccctgat caagcctgac gcagtcgccc atccactgat tctggaggct      60
   gttcatcagc agattctaag caacaagttc ctgattgtac gaatgagaga actactgtgg     120
   agaaaggaag attgccagag gttttaccga gagcatgaag ggcgtttttt ctatcagagg     180
   ctggtggagt tcatggccag cgggccaatc cgagcctaca tccttgccca caaggatgcc     240
   atccagctct ggaggacgct catgggaccc accagagtgt tccgagcacg ccatgtggcc     300
   ccagattcta tccgtgggag tttcggcctc actgacaccc gcaacaccac ccatggttcg     360
   gactctgtgg tttcagccag cagagagatt gcagccttct tccctgactt cagtgaacag     420
   cgctggtatg aggaggaaga gccccagttg cgctgtggcc ctgtgtgcta tagcccagag     480
   ggaggtgtcc actatgtagc tggaacagga ggcctaggac cagcctga                  528
<210> 71
   <211> 175
   <212> PRT
   <213> Artificial Sequence
<220>
   <223> Human NME6 3
<400> 71
   Met Leu Thr Leu Ala Leu Ile Lys Pro Asp Ala Val Ala His Pro Leu
   1               5                   10                  15
   Ile Leu Glu Ala Val His Gln Gln Ile Leu Ser Asn Lys Phe Leu Ile
               20                  25                  30
   Val Arg Met Arg Glu Leu Leu Trp Arg Lys Glu Asp Cys Gln Arg Phe
           35                  40                  45
   Tyr Arg Glu His Glu Gly Arg Phe Phe Tyr Gln Arg Leu Val Glu Phe
       50                  55                  60
   Met Ala Ser Gly Pro Ile Arg Ala Tyr Ile Leu Ala His Lys Asp Ala
   65                  70                  75                  80
   Ile Gln Leu Trp Arg Thr Leu Met Gly Pro Thr Arg Val Phe Arg Ala
                   85                  90                  95
   Arg His Val Ala Pro Asp Ser Ile Arg Gly Ser Phe Gly Leu Thr Asp
               100                 105                 110
   Thr Arg Asn Thr Thr His Gly Ser Asp Ser Val Val Ser Ala Ser Arg
           115                 120                 125
   Glu Ile Ala Ala Phe Phe Pro Asp Phe Ser Glu Gln Arg Trp Tyr Glu
       130                 135                 140
   Glu Glu Glu Pro Gln Leu Arg Cys Gly Pro Val Cys Tyr Ser Pro Glu
   145                 150                 155                 160
   Gly Gly Val His Tyr Val Ala Gly Thr Gly Gly Leu Gly Pro Ala
                   165                 170                 175
<210> 72
   <211> 585
   <212> DNA
   <213> Artificial Sequence
<220>
   <223> Human NME6 sequence optimized for E. coli expression
<400> 72
   atgacgcaaa atctgggctc ggaaatggca agtatcctgc gctccccgca agcactgcaa 60
   ctgaccctgg ctctgatcaa accggacgct gttgctcatc cgctgattct ggaagcggtc 120
   caccagcaaa ttctgagcaa caaatttctg atcgtgcgta tgcgcgaact gctgtggcgt 180
   aaagaagatt gccagcgttt ttatcgcgaa catgaaggcc gtttctttta tcaacgcctg 240
   gttgaattca tggcctctgg tccgattcgc gcatatatcc tggctcacaa agatgcgatt 300
   cagctgtggc gtaccctgat gggtccgacg cgcgtctttc gtgcacgtca tgtggcaccg 360
   gactcaatcc gtggctcgtt cggtctgacc gatacgcgca ataccacgca cggtagcgac 420
   tctgttgtta gtgcgtcccg tgaaatcgcg gcctttttcc cggacttctc cgaacagcgt 480
   tggtacgaag aagaagaacc gcaactgcgc tgtggcccgg tctgttattc tccggaaggt 540
   ggtgtccatt atgtggcggg cacgggtggt ctgggtccgg catga 585
<210> 73
   <211> 194
   <212> PRT
   <213> Artificial Sequence
<220>
   <223> Human NME6 sequence optimized for E. coli expression
<400> 73
   Met Thr Gln Asn Leu Gly Ser Glu Met Ala Ser Ile Leu Arg Ser Pro
   1               5                   10                  15
   Gln Ala Leu Gln Leu Thr Leu Ala Leu Ile Lys Pro Asp Ala Val Ala
               20                  25                  30
   His Pro Leu Ile Leu Glu Ala Val His Gln Gln Ile Leu Ser Asn Lys
           35                  40                  45
   Phe Leu Ile Val Arg Met Arg Glu Leu Leu Trp Arg Lys Glu Asp Cys
       50                  55                  60
   Gln Arg Phe Tyr Arg Glu His Glu Gly Arg Phe Phe Tyr Gln Arg Leu
   65                  70                  75                  80
   Val Glu Phe Met Ala Ser Gly Pro Ile Arg Ala Tyr Ile Leu Ala His
                   85                  90                  95
   Lys Asp Ala Ile Gln Leu Trp Arg Thr Leu Met Gly Pro Thr Arg Val
               100                 105                 110
   Phe Arg Ala Arg His Val Ala Pro Asp Ser Ile Arg Gly Ser Phe Gly
           115                 120                 125
   Leu Thr Asp Thr Arg Asn Thr Thr His Gly Ser Asp Ser Val Val Ser
       130                 135                 140
   Ala Ser Arg Glu Ile Ala Ala Phe Phe Pro Asp Phe Ser Glu Gln Arg
   145                 150                 155                 160
   Trp Tyr Glu Glu Glu Glu Pro Gln Leu Arg Cys Gly Pro Val Cys Tyr
                   165                 170                 175
   Ser Pro Glu Gly Gly Val His Tyr Val Ala Gly Thr Gly Gly Leu Gly
               180                 185                 190
   Pro Ala
<210> 74
   <211> 525
   <212> DNA
   <213> Artificial Sequence
<220>
   <223> Human NME6 1 sequence optimized for E. coli expression
<400> 74
   atgacgcaaa atctgggctc ggaaatggca agtatcctgc gctccccgca agcactgcaa       60
   ctgaccctgg ctctgatcaa accggacgct gttgctcatc cgctgattct ggaagcggtc      120
   caccagcaaa ttctgagcaa caaatttctg atcgtgcgta tgcgcgaact gctgtggcgt      180
   aaagaagatt gccagcgttt ttatcgcgaa catgaaggcc gtttctttta tcaacgcctg      240
   gttgaattca tggcctctgg tccgattcgc gcatatatcc tggctcacaa agatgcgatt      300
   cagctgtggc gtaccctgat gggtccgacg cgcgtctttc gtgcacgtca tgtggcaccg      360
   gactcaatcc gtggctcgtt cggtctgacc gatacgcgca ataccacgca cggtagcgac      420
   tctgttgtta gtgcgtcccg tgaaatcgcg gcctttttcc cggacttctc cgaacagcgt      480
   tggtacgaag aagaagaacc gcaactgcgc tgtggcccgg tctga                      525
<210> 75
   <211> 174
   <212> PRT
   <213> Artificial Sequence
<220>
   <223> Human NME6 1 sequence optimized for E. coli expression
<400> 75
   Met Thr Gln Asn Leu Gly Ser Glu Met Ala Ser Ile Leu Arg Ser Pro
   1               5                   10                  15
   Gln Ala Leu Gln Leu Thr Leu Ala Leu Ile Lys Pro Asp Ala Val Ala
               20                  25                  30
   His Pro Leu Ile Leu Glu Ala Val His Gln Gln Ile Leu Ser Asn Lys
           35                  40                  45
   Phe Leu Ile Val Arg Met Arg Glu Leu Leu Trp Arg Lys Glu Asp Cys
   50 55 60
   Gln Arg Phe Tyr Arg Glu His Glu Gly Arg Phe Phe Tyr Gln Arg Leu
   65 70 75 80
   Val Glu Phe Met Ala Ser Gly Pro Ile Arg Ala Tyr Ile Leu Ala His
   85 90 95
   Lys Asp Ala Ile Gln Leu Trp Arg Thr Leu Met Gly Pro Thr Arg Val
   100 105 110
   Phe Arg Ala Arg His Val Ala Pro Asp Ser Ile Arg Gly Ser Phe Gly
   115 120 125
   Leu Thr Asp Thr Arg Asn Thr Thr His Gly Ser Asp Ser Val Val Ser
   130 135 140
   Ala Ser Arg Glu Ile Ala Ala Phe Phe Pro Asp Phe Ser Glu Gln Arg
   145 150 155 160
   Trp Tyr Glu Glu Glu Glu Pro Gln Leu Arg Cys Gly Pro Val
   165 170
<210> 76
   <211> 468
   <212> DNA
   <213> Artificial Sequence
<220>
   <223> Human NME6 2 sequence optimized for E. coli expression
<400> 76
   atgctgaccc tggctctgat caaaccggac gctgttgctc atccgctgat tctggaagcg 60
   gtccaccagc aaattctgag caacaaattt ctgatcgtgc gtatgcgcga actgctgtgg 120
   cgtaaagaag attgccagcg tttttatcgc gaacatgaag gccgtttctt ttatcaacgc 180
   ctggttgaat tcatggcctc tggtccgatt cgcgcatata tcctggctca caaagatgcg 240
   attcagctgt ggcgtaccct gatgggtccg acgcgcgtct ttcgtgcacg tcatgtggca 300
   ccggactcaa tccgtggctc gttcggtctg accgatacgc gcaataccac gcacggtagc 360
   gactctgttg ttagtgcgtc ccgtgaaatc gcggcctttt tcccggactt ctccgaacag 420
   cgttggtacg aagaagaaga accgcaactg cgctgtggcc cggtctga 468
<210> 77
   <211> 155
   <212> PRT
   <213> Artificial Sequence
<220>
   <223> Human NME6 2 sequence optimized for E. coli expression
<400> 77
   Met Leu Thr Leu Ala Leu Ile Lys Pro Asp Ala Val Ala His Pro Leu
   1               5                   10                  15
   Ile Leu Glu Ala Val His Gln Gln Ile Leu Ser Asn Lys Phe Leu Ile
               20                  25                  30
   Val Arg Met Arg Glu Leu Leu Trp Arg Lys Glu Asp Cys Gln Arg Phe
           35                  40                  45
   Tyr Arg Glu His Glu Gly Arg Phe Phe Tyr Gln Arg Leu Val Glu Phe
       50                  55                  60
   Met Ala Ser Gly Pro Ile Arg Ala Tyr Ile Leu Ala His Lys Asp Ala
   65                  70                  75                  80
   Ile Gln Leu Trp Arg Thr Leu Met Gly Pro Thr Arg Val Phe Arg Ala
                   85                  90                  95
   Arg His Val Ala Pro Asp Ser Ile Arg Gly Ser Phe Gly Leu Thr Asp
               100                 105                 110
   Thr Arg Asn Thr Thr His Gly Ser Asp Ser Val Val Ser Ala Ser Arg
           115                 120                 125
   Glu Ile Ala Ala Phe Phe Pro Asp Phe Ser Glu Gln Arg Trp Tyr Glu
       130                 135                 140
   Glu Glu Glu Pro Gln Leu Arg Cys Gly Pro Val
   145                 150                 155
<210> 78
   <211> 528
   <212> DNA
   <213> Artificial Sequence
<220>
   <223> Human NME6 3 sequence optimized for E. coli expression
<400> 78
   atgctgaccc tggctctgat caaaccggac gctgttgctc atccgctgat tctggaagcg      60
   gtccaccagc aaattctgag caacaaattt ctgatcgtgc gtatgcgcga actgctgtgg     120
   cgtaaagaag attgccagcg tttttatcgc gaacatgaag gccgtttctt ttatcaacgc     180
   ctggttgaat tcatggcctc tggtccgatt cgcgcatata tcctggctca caaagatgcg     240
   attcagctgt ggcgtaccct gatgggtccg acgcgcgtct ttcgtgcacg tcatgtggca     300
   ccggactcaa tccgtggctc gttcggtctg accgatacgc gcaataccac gcacggtagc     360
   gactctgttg ttagtgcgtc ccgtgaaatc gcggcctttt tcccggactt ctccgaacag     420
   cgttggtacg aagaagaaga accgcaactg cgctgtggcc cggtctgtta ttctccggaa     480
   ggtggtgtcc attatgtggc gggcacgggt ggtctgggtc cggcatga                  528
<210> 79
   <211> 175
   <212> PRT
   <213> Artificial Sequence
<220>
   <223> Human NME6 3 sequence optimized for E. coli expression
<400> 79
   Met Leu Thr Leu Ala Leu Ile Lys Pro Asp Ala Val Ala His Pro Leu
   1               5                   10                  15
   Ile Leu Glu Ala Val His Gln Gln Ile Leu Ser Asn Lys Phe Leu Ile
               20                  25                  30
   Val Arg Met Arg Glu Leu Leu Trp Arg Lys Glu Asp Cys Gln Arg Phe
           35                  40                  45
   Tyr Arg Glu His Glu Gly Arg Phe Phe Tyr Gln Arg Leu Val Glu Phe
       50                  55                  60
   Met Ala Ser Gly Pro Ile Arg Ala Tyr Ile Leu Ala His Lys Asp Ala
   65                  70                  75                  80
   Ile Gln Leu Trp Arg Thr Leu Met Gly Pro Thr Arg Val Phe Arg Ala
                   85                  90                  95
   Arg His Val Ala Pro Asp Ser Ile Arg Gly Ser Phe Gly Leu Thr Asp
               100                 105                 110
   Thr Arg Asn Thr Thr His Gly Ser Asp Ser Val Val Ser Ala Ser Arg
           115                 120                 125
   Glu Ile Ala Ala Phe Phe Pro Asp Phe Ser Glu Gln Arg Trp Tyr Glu
       130                 135                 140
   Glu Glu Glu Pro Gln Leu Arg Cys Gly Pro Val Cys Tyr Ser Pro Glu
   145                 150                 155                 160
   Gly Gly Val His Tyr Val Ala Gly Thr Gly Gly Leu Gly Pro Ala
                   165                 170                 175
<210> 80
   <211> 1306
   <212> DNA
   <213> Artificial Sequence
<220>
   <223> OriGene-NME7-1 full length
<400> 80
   gacgttgtat acgactccta tagggcggcc gggaattcgt cgactggatc cggtaccgag     60
   gagatctgcc gccgcgatcg ccatgaatca tagtgaaaga ttcgttttca ttgcagagtg    120
   gtatgatcca aatgcttcac ttcttcgacg ttatgagctt ttattttacc caggggatgg    180
   atctgttgaa atgcatgatg taaagaatca tcgcaccttt ttaaagcgga ccaaatatga    240
   taacctgcac ttggaagatt tatttatagg caacaaagtg aatgtcttct ctcgacaact    300
   ggtattaatt gactatgggg atcaatatac agctcgccag ctgggcagta ggaaagaaaa    360
   aacgctagcc ctaattaaac cagatgcaat atcaaaggct ggagaaataa ttgaaataat    420
   aaacaaagct ggatttacta taaccaaact caaaatgatg atgctttcaa ggaaagaagc    480
   attggatttt catgtagatc accagtcaag accctttttc aatgagctga tccagtttat     540
   tacaactggt cctattattg ccatggagat tttaagagat gatgctatat gtgaatggaa    600
   aagactgctg ggacctgcaa actctggagt ggcacgcaca gatgcttctg aaagcattag    660
   agccctcttt ggaacagatg gcataagaaa tgcagcgcat ggccctgatt cttttgcttc    720
   tgcggccaga gaaatggagt tgttttttcc ttcaagtgga ggttgtgggc cggcaaacac     780
   tgctaaattt actaattgta cctgttgcat tgttaaaccc catgctgtca gtgaaggact    840
   gttgggaaag atcctgatgg ctatccgaga tgcaggtttt gaaatctcag ctatgcagat    900
   gttcaatatg gatcgggtta atgttgagga attctatgaa gtttataaag gagtagtgac    960
   cgaatatcat gacatggtga cagaaatgta ttctggccct tgtgtagcaa tggagattca   1020
   acagaataat gctacaaaga catttcgaga attttgtgga cctgctgatc ctgaaattgc   1080
   ccggcattta cgccctggaa ctctcagagc aatctttggt aaaactaaga tccagaatgc   1140
   tgttcactgt actgatctgc cagaggatgg cctattagag gttcaatact tcttcaagat   1200
   cttggataat acgcgtacgc ggccgctcga gcagaaactc atctcagaag aggatctggc   1260
   agcaaatgat atcctggatt acaaggatga cgacgataag gtttaa                  1306
<210> 81
   <211> 407
   <212> PRT
   <213> Artificial Sequence
<220>
   <223> OriGene-NME7-1 full length
<400> 81
   Met Asn His Ser Glu Arg Phe Val Phe Ile Ala Glu Trp Tyr Asp Pro
   1               5                   10                  15
   Asn Ala Ser Leu Leu Arg Arg Tyr Glu Leu Leu Phe Tyr Pro Gly Asp
               20                  25                  30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe Pro Ser
       210                 215                 220
   Ser Gly Gly Cys Gly Pro Ala Asn Thr Ala Lys Phe Thr Asn Cys Thr
   225                 230                 235                 240
   Cys Cys Ile Val Lys Pro His Ala Val Ser Glu Gly Leu Leu Gly Lys
   245 250 255
   Ile Leu Met Ala Ile Arg Asp Ala Gly Phe Glu Ile Ser Ala Met Gln
   260 265 270
   Met Phe Asn Met Asp Arg Val Asn Val Glu Glu Phe Tyr Glu Val Tyr
   275 280 285
   Lys Gly Val Val Thr Glu Tyr His Asp Met Val Thr Glu Met Tyr Ser
   290 295 300
   Gly Pro Cys Val Ala Met Glu Ile Gln Gln Asn Asn Ala Thr Lys Thr
   305 310 315 320
   Phe Arg Glu Phe Cys Gly Pro Ala Asp Pro Glu Ile Ala Arg His Leu
   325 330 335
   Arg Pro Gly Thr Leu Arg Ala Ile Phe Gly Lys Thr Lys Ile Gln Asn
   340 345 350
   Ala Val His Cys Thr Asp Leu Pro Glu Asp Gly Leu Leu Glu Val Gln
   355 360 365
   Tyr Phe Phe Lys Ile Leu Asp Asn Thr Arg Thr Arg Arg Leu Glu Gln
   370 375 380
   Lys Leu Ile Ser Glu Glu Asp Leu Ala Ala Asn Asp Ile Leu Asp Tyr
   385 390 395 400
   Lys Asp Asp Asp Asp Lys Val
   405
<210> 82
   <211> 376
   <212> PRT
   <213> Artificial Sequence
<220>
   <223> Abnova NME7-1 Full length
<400> 82
   Met Asn His Ser Glu Arg Phe Val Phe Ile Ala Glu Trp Tyr Asp Pro
   1 5 10 15
   Asn Ala Ser Leu Leu Arg Arg Tyr Glu Leu Leu Phe Tyr Pro Gly Asp
   20 25 30
   Gly Ser Val Glu Met His Asp Val Lys Asn His Arg Thr Phe Leu Lys
           35                  40                  45
   Arg Thr Lys Tyr Asp Asn Leu His Leu Glu Asp Leu Phe Ile Gly Asn
       50                  55                  60
   Lys Val Asn Val Phe Ser Arg Gln Leu Val Leu Ile Asp Tyr Gly Asp
   65                  70                  75                  80
   Gln Tyr Thr Ala Arg Gln Leu Gly Ser Arg Lys Glu Lys Thr Leu Ala
                   85                  90                  95
   Leu Ile Lys Pro Asp Ala Ile Ser Lys Ala Gly Glu Ile Ile Glu Ile
               100                 105                 110
   Ile Asn Lys Ala Gly Phe Thr Ile Thr Lys Leu Lys Met Met Met Leu
           115                 120                 125
   Ser Arg Lys Glu Ala Leu Asp Phe His Val Asp His Gln Ser Arg Pro
       130                 135                 140
   Phe Phe Asn Glu Leu Ile Gln Phe Ile Thr Thr Gly Pro Ile Ile Ala
   145                 150                 155                 160
   Met Glu Ile Leu Arg Asp Asp Ala Ile Cys Glu Trp Lys Arg Leu Leu
                   165                 170                 175
   Gly Pro Ala Asn Ser Gly Val Ala Arg Thr Asp Ala Ser Glu Ser Ile
               180                 185                 190
   Arg Ala Leu Phe Gly Thr Asp Gly Ile Arg Asn Ala Ala His Gly Pro
           195                 200                 205
   Asp Ser Phe Ala Ser Ala Ala Arg Glu Met Glu Leu Phe Phe Pro Ser
       210                 215                 220
   Ser Gly Gly Cys Gly Pro Ala Asn Thr Ala Lys Phe Thr Asn Cys Thr
   225                 230                 235                 240
   Cys Cys Ile Val Lys Pro His Ala Val Ser Glu Gly Leu Leu Gly Lys
                   245                 250                 255
   Ile Leu Met Ala Ile Arg Asp Ala Gly Phe Glu Ile Ser Ala Met Gln
               260                 265                 270
   Met Phe Asn Met Asp Arg Val Asn Val Glu Glu Phe Tyr Glu Val Tyr
   275 280 285
   Lys Gly Val Val Thr Glu Tyr His Asp Met Val Thr Glu Met Tyr Ser
   290 295 300
   Gly Pro Cys Val Ala Met Glu Ile Gln Gln Asn Asn Ala Thr Lys Thr
   305 310 315 320
   Phe Arg Glu Phe Cys Gly Pro Ala Asp Pro Glu Ile Ala Arg His Leu
   325 330 335
   Arg Pro Gly Thr Leu Arg Ala Ile Phe Gly Lys Thr Lys Ile Gln Asn
   340 345 350
   Ala Val His Cys Thr Asp Leu Pro Glu Asp Gly Leu Leu Glu Val Gln
   355 360 365
   Tyr Phe Phe Lys Ile Leu Asp Asn
   370 375
<210> 83
   <211> 98
   <212> PRT
   <213> Artificial Sequence
<220>
   <223> Abnova Partial NME7-B
<400> 83
   Asp Arg Val Asn Val Glu Glu Phe Tyr Glu Val Tyr Lys Gly Val Val
   1 5 10 15
   Thr Glu Tyr His Asp Met Val Thr Glu Met Tyr Ser Gly Pro Cys Val
   20 25 30
   Ala Met Glu Ile Gln Gln Asn Asn Ala Thr Lys Thr Phe Arg Glu Phe
   35 40 45
   Cys Gly Pro Ala Asp Pro Glu Ile Ala Arg His Leu Arg Pro Gly Thr
   50 55 60
   Leu Arg Ala Ile Phe Gly Lys Thr Lys Ile Gln Asn Ala Val His Cys
   65 70 75 80
   Thr Asp Leu Pro Glu Asp Gly Leu Leu Glu Val Gln Tyr Phe Phe Lys
   85 90 95
   Ile Leu
<210> 84
   <211> 27
   <212> DNA
   <213> Artificial Sequence
<220>
   <223> DNA encoding Histidine Tag
<400> 84
   ctcgagcacc accaccacca ccactga 27
<210> 85
   <211> 36
   <212> DNA
   <213> Artificial Sequence
<220>
   <223> DNA encoding Strept II Tag
<400> 85
   accggttgga gccatcctca gttcgaaaag taatga 36
<210> 86
   <211> 35
   <212> PRT
   <213> Artificial Sequence
<220>
   <223> N-10 peptide
<400> 86
   Gln Phe Asn Gln Tyr Lys Thr Glu Ala Ala Ser Arg Tyr Asn Leu Thr
   1               5                   10                  15
   Ile Ser Asp Val Ser Val Ser Asp Val Pro Phe Pro Phe Ser Ala Gln
               20                  25                  30
   Ser Gly Ala
           35
<210> 87
   <211> 35
   <212> PRT
   <213> Artificial Sequence
<220>
   <223> C-10 peptide
<400> 87
   Gly Thr Ile Asn Val His Asp Val Glu Thr Gln Phe Asn Gln Tyr Lys
   1               5                   10                  15
   Thr Glu Ala Ala Ser Arg Tyr Asn Leu Thr Ile Ser Asp Val Ser Val
               20                  25                  30
   Ser Asp Val
           35
<210> 88
   <211> 8
   <212> PRT
   <213> Artificial Sequence
<220>
   <223> Immunizing peptides derived from human NME7
<400> 88
   Leu Ala Leu Ile Lys Pro Asp Ala
   1               5
<210> 89
   <211> 18
   <212> PRT
   <213> Artificial Sequence
<220>
   <223> Immunizing peptides derived from human NME7
<400> 89
   Met Met Met Leu Ser Arg Lys Glu Ala Leu Asp Phe His Val Asp His
   1               5                   10                  15
   Gln Ser
<210> 90
   <211> 10
   <212> PRT
   <213> Artificial Sequence
<220>
   <223> Immunizing peptides derived from human NME7
<400> 90
   Ala Leu Asp Phe His Val Asp His Gln Ser
   1               5                   10
<210> 91
   <211> 14
   <212> PRT
   <213> Artificial Sequence
<220>
   <223> Immunizing peptides derived from human NME7
<400> 91
   Glu Ile Leu Arg Asp Asp Ala Ile Cys Glu Trp Lys Arg Leu
   1 5 10
<210> 92
   <211> 12
   <212> PRT
   <213> Artificial Sequence
<220>
   <223> Immunizing peptides derived from human NME7
<400> 92
   Phe Asn Glu Leu Ile Gln Phe Ile Thr Thr Gly Pro
   1 5 10
<210> 93
   <211> 8
   <212> PRT
   <213> Artificial Sequence
<220>
   <223> Immunizing peptides derived from human NME7
<400> 93
   Arg Asp Asp Ala Ile Cys Glu Trp
   1 5
<210> 94
   <211> 25
   <212> PRT
   <213> Artificial Sequence
<220>
   <223> Immunizing peptides derived from human NME7
<400> 94
   Ser Gly Val Ala Arg Thr Asp Ala Ser Glu Ser Ile Arg Ala Leu Phe
   1 5 10 15
   Gly Thr Asp Gly Ile Arg Asn Ala Ala
   20 25
<210> 95
   <211> 9
   <212> PRT
   <213> Artificial Sequence
<220>
   <223> Immunizing peptides derived from human NME7
<400> 95
   Glu Leu Phe Phe Pro Ser Ser Gly Gly
   1               5
<210> 96
   <211> 26
   <212> PRT
   <213> Artificial Sequence
<220>
   <223> Immunizing peptides derived from human NME7
<400> 96
   Lys Phe Thr Asn Cys Thr Cys Cys Ile Val Lys Pro His Ala Val Ser
   1               5                   10                  15
   Glu Gly Leu Leu Gly Lys Ile Leu Met Ala
               20                  25
<210> 97
   <211> 36
   <212> PRT
   <213> Artificial Sequence
<220>
   <223> Immunizing peptides derived from human NME7
<400> 97
   Leu Met Ala Ile Arg Asp Ala Gly Phe Glu Ile Ser Ala Met Gln Met
   1               5                   10                  15
   Phe Asn Met Asp Arg Val Asn Val Glu Glu Phe Tyr Glu Val Tyr Lys
               20                  25                  30
   Gly Val Val Thr
           35
<210> 98
   <211> 15
   <212> PRT
   <213> Artificial Sequence
<220>
   <223> Immunizing peptides derived from human NME7
<400> 98
   Glu Phe Tyr Glu Val Tyr Lys Gly Val Val Thr Glu Tyr His Asp
   1               5                   10                  15
<210> 99
   <211> 43
   <212> PRT
   <213> Artificial Sequence
<220>
   <223> Immunizing peptides derived from human NME7
<400> 99
   Glu Ile Gln Gln Asn Asn Ala Thr Lys Thr Phe Arg Glu Phe Cys Gly
   1               5                   10                  15
   Pro Ala Asp Pro Glu Ile Ala Arg His Leu Arg Pro Gly Thr Leu Arg
               20                  25                  30
   Ala Ile Phe Gly Lys Thr Lys Ile Gln Asn Ala
           35                  40
<210> 100
   <211> 8
   <212> PRT
   <213> Artificial Sequence
<220>
   <223> Immunizing peptides derived from human NME7
<400> 100
   Tyr Ser Gly Pro Cys Val Ala Met
   1               5
<210> 101
   <211> 7
   <212> PRT
   <213> Artificial Sequence
<220>
   <223> Immunizing peptides derived from human NME7
<400> 101
   Phe Arg Glu Phe Cys Gly Pro
   1               5
<210> 102
   <211> 23
   <212> PRT
   <213> Artificial Sequence
<220>
   <223> Immunizing peptides derived from human NME7
<400> 102
   Val His Cys Thr Asp Leu Pro Glu Asp Gly Leu Leu Glu Val Gln Tyr
   1 5 10 15
   Phe Phe Lys Ile Leu Asp Asn
   20
<210> 103
   <211> 9
   <212> PRT
   <213> Artificial Sequence
<220>
   <223> Immunizing peptides derived from human NME7
<400> 103
   Ile Gln Asn Ala Val His Cys Thr Asp
   1 5
<210> 104
   <211> 20
   <212> PRT
   <213> Artificial Sequence
<220>
   <223> Immunizing peptides derived from human NME7
<400> 104
   Thr Asp Leu Pro Glu Asp Gly Leu Leu Glu Val Gln Tyr Phe Phe Lys
   1 5 10 15
   Ile Leu Asp Asn
   20
<210> 105
   <211> 13
   <212> PRT
   <213> Artificial Sequence
<220>
   <223> Immunizing peptides derived from human NME7
<400> 105
   Pro Glu Asp Gly Leu Leu Glu Val Gln Tyr Phe Phe Lys
   1 5 10
<210> 106
   <211> 12
   <212> PRT
   <213> Artificial Sequence
<220>
   <223> Immunizing peptides derived from human NME7
<400> 106
   Glu Ile Ile Asn Lys Ala Gly Phe Thr Ile Thr Lys
   1 5 10
<210> 107
   <211> 16
   <212> PRT
   <213> Artificial Sequence
<220>
   <223> Immunizing peptides derived from human NME7
<400> 107
   Met Leu Ser Arg Lys Glu Ala Leu Asp Phe His Val Asp His Gln Ser
   1 5 10 15
<210> 108
   <211> 9
   <212> PRT
   <213> Artificial Sequence
<220>
   <223> Immunizing peptides derived from human NME7
<400> 108
   Asn Glu Leu Ile Gln Phe Ile Thr Thr
   1 5
<210> 109
   <211> 14
   <212> PRT
   <213> Artificial Sequence
<220>
   <223> Immunizing peptides derived from human NME7
<400> 109
   Glu Ile Leu Arg Asp Asp Ala Ile Cys Glu Trp Lys Arg Leu
   1 5 10
<210> 110
   <211> 21
   <212> PRT
   <213> Artificial Sequence
<220>
   <223> Immunizing peptides derived from human NME7
<400> 110
   Ser Gly Val Ala Arg Thr Asp Ala Ser Glu Ser Ile Arg Ala Leu Phe
   1               5                   10                  15
   Gly Thr Asp Gly Ile
               20
<210> 111
   <211> 11
   <212> PRT
   <213> Artificial Sequence
<220>
   <223> Immunizing peptides derived from human NME7
<400> 111
   Ser Gly Val Ala Arg Thr Asp Ala Ser Glu Ser
   1               5                   10
<210> 112
   <211> 8
   <212> PRT
   <213> Artificial Sequence
<220>
   <223> Immunizing peptides derived from human NME7
<400> 112
   Ala Leu Phe Gly Thr Asp Gly Ile
   1               5
<210> 113
   <211> 14
   <212> PRT
   <213> Artificial Sequence
<220>
   <223> Immunizing peptides derived from human NME7
<400> 113
   Asn Cys Thr Cys Cys Ile Val Lys Pro His Ala Val Ser Glu
   1               5                   10
<210> 114
   <211> 11
   <212> PRT
   <213> Artificial Sequence
<220>
   <223> Immunizing peptides derived from human NME7
<400> 114
   Leu Gly Lys Ile Leu Met Ala Ile Arg Asp Ala
   1 5 10
<210> 115
   <211> 16
   <212> PRT
   <213> Artificial Sequence
<220>
   <223> Immunizing peptides derived from human NME7
<400> 115
   Glu Ile Ser Ala Met Gln Met Phe Asn Met Asp Arg Val Asn Val Glu
   1 5 10 15
<210> 116
   <211> 8
   <212> PRT
   <213> Artificial Sequence
<220>
   <223> Immunizing peptides derived from human NME7
<400> 116
   Glu Val Tyr Lys Gly Val Val Thr
   1 5
<210> 117
   <211> 8
   <212> PRT
   <213> Artificial Sequence
<220>
   <223> Immunizing peptides derived from human NME7
<400> 117
   Glu Tyr His Asp Met Val Thr Glu
   1 5
<210> 118
   <211> 15
   <212> PRT
   <213> Artificial Sequence
<220>
   <223> Immunizing peptides derived from human NME7
<400> 118
   Glu Phe Cys Gly Pro Ala Asp Pro Glu Ile Ala Arg His Leu Arg
   1 5 10 15
<210> 119
   <211> 12
   <212> PRT
   <213> Artificial Sequence
<220>
   <223> Immunizing peptides derived from human NME7
<400> 119
   Ala Ile Phe Gly Lys Thr Lys Ile Gln Asn Ala Val
   1               5                   10
<210> 120
   <211> 18
   <212> PRT
   <213> Artificial Sequence
<220>
   <223> Immunizing peptides derived from human NME7
<400> 120
   Leu Pro Glu Asp Gly Leu Leu Glu Val Gln Tyr Phe Phe Lys Ile Leu
   1               5                   10                  15
   Asp Asn
<210> 121
   <211> 17
   <212> PRT
   <213> Artificial Sequence
<220>
   <223> Immunizing peptides derived from human NME7
<400> 121
   Gly Pro Asp Ser Phe Ala Ser Ala Ala Arg Glu Met Glu Leu Phe Phe
   1               5                   10                  15
   Pro
<210> 122
   <211> 7
   <212> PRT
   <213> Artificial Sequence
<220>
   <223> Immunizing peptides derived from human NME7
<400> 122
   Ile Cys Glu Trp Lys Arg Leu
   1               5
<210> 123
   <211> 11
   <212> PRT
   <213> Artificial Sequence
<220>
   <223> Immunizing peptides derived from human NME7
<400> 123
   Leu Gly Lys Ile Leu Met Ala Ile Arg Asp Ala
   1 5 10
<210> 124
   <211> 10
   <212> PRT
   <213> Artificial Sequence
<220>
   <223> Immunizing peptides derived from human NME7
<400> 124
   His Ala Val Ser Glu Gly Leu Leu Gly Lys
   1 5 10
<210> 125
   <211> 8
   <212> PRT
   <213> Artificial Sequence
<220>
   <223> Immunizing peptides derived from human NME7
<400> 125
   Val Thr Glu Met Tyr Ser Gly Pro
   1 5
<210> 126
   <211> 9
   <212> PRT
   <213> Artificial Sequence
<220>
   <223> Immunizing peptides derived from human NME7
<400> 126
   Asn Ala Thr Lys Thr Phe Arg Glu Phe
   1 5
<210> 127
   <211> 9
   <212> PRT
   <213> Artificial Sequence
<220>
   <223> Immunizing peptides derived from human NME7
<400> 127
   Ala Ile Arg Asp Ala Gly Phe Glu Ile
   1 5
<210> 128
   <211> 13
   <212> PRT
   <213> Artificial Sequence
<220>
   <223> Immunizing peptides derived from human NME7
<400> 128
   Ala Ile Cys Glu Trp Lys Arg Leu Leu Gly Pro Ala Asn
   1 5 10
<210> 129
   <211> 8
   <212> PRT
   <213> Artificial Sequence
<220>
   <223> Immunizing peptides derived from human NME7
<400> 129
   Asp His Gln Ser Arg Pro Phe Phe
   1 5
<210> 130
   <211> 13
   <212> PRT
   <213> Artificial Sequence
<220>
   <223> Immunizing peptides derived from human NME7
<400> 130
   Ala Ile Cys Glu Trp Lys Arg Leu Leu Gly Pro Ala Asn
   1 5 10
<210> 131
   <211> 8
   <212> PRT
   <213> Artificial Sequence
<220>
   <223> Immunizing peptides derived from human NME7
<400> 131
   Val Asp His Gln Ser Arg Pro Phe
   1 5
<210> 132
   <211> 6
   <212> PRT
   <213> Artificial Sequence
<220>
   <223> Immunizing peptides derived from human NME7
<400> 132
   Pro Asp Ser Phe Ala Ser
   1               5
<210> 133
   <211> 18
   <212> PRT
   <213> Artificial Sequence
<220>
   <223> Immunizing peptides derived from human NME7
<400> 133
   Lys Ala Gly Glu Ile Ile Glu Ile Ile Asn Lys Ala Gly Phe Thr Ile
   1               5                   10                  15
   Thr Lys
<210> 134
   <211> 29
   <212> PRT
   <213> Artificial Sequence
<220>
   <223> Immunizing peptides derived from human NME1
<400> 134
   Met Ala Asn Cys Glu Arg Thr Phe Ile Ala Ile Lys Pro Asp Gly Val
   1               5                   10                  15
   Gln Arg Gly Leu Val Gly Glu Ile Ile Lys Arg Phe Glu
               20                  25
<210> 135
   <211> 8
   <212> PRT
   <213> Artificial Sequence
<220>
   <223> Immunizing peptides derived from human NME1
<400> 135
   Val Asp Leu Lys Asp Arg Pro Phe
   1 5
<210> 136
   <211> 17
   <212> PRT
   <213> Artificial Sequence
<220>
   <223> Immunizing peptides derived from human NME1
<400> 136
   His Gly Ser Asp Ser Val Glu Ser Ala Glu Lys Glu Ile Gly Leu Trp
   1 5 10 15
   Phe
<210> 137
   <211> 25
   <212> PRT
   <213> Artificial Sequence
<220>
   <223> Immunizing peptides derived from human NME1
<400> 137
   Glu Arg Thr Phe Ile Ala Ile Lys Pro Asp Gly Val Gln Arg Gly Leu
   1 5 10 15
   Val Gly Glu Ile Ile Lys Arg Phe Glu
   20 25
<210> 138
   <211> 30
   <212> PRT
   <213> Artificial Sequence
<220>
   <223> Immunizing peptides derived from human NME1
<400> 138
   Val Asp Leu Lys Asp Arg Pro Phe Phe Ala Gly Leu Val Lys Tyr Met
   1 5 10 15
   His Ser Gly Pro Val Val Ala Met Val Trp Glu Gly Leu Asn
   20 25 30
<210> 139
   <211> 26
   <212> PRT
   <213> Artificial Sequence
<220>
   <223> Immunizing peptides derived from human NME1
<400> 139
   Asn Ile Ile His Gly Ser Asp Ser Val Glu Ser Ala Glu Lys Glu Ile
   1               5                   10                  15
   Gly Leu Trp Phe His Pro Glu Glu Leu Val
               20                  25
<210> 140
   <211> 14
   <212> PRT
   <213> Artificial Sequence
<220>
   <223> Immunizing peptides derived from human NME1
<400> 140
   Lys Pro Asp Gly Val Gln Arg Gly Leu Val Gly Glu Ile Ile
   1               5                   10